# EUROPÄISCHE PATENTSCHRIFT

(11) **EP 1 200 429 B1**
(45) Veröffentlichungstag und Bekanntmachung des Hinweises auf die Patenterteilung: **16.06.2004**
(21) Anmeldenummer: 99958149.9
(22) Anmeldetag: 01.12.1999
(51) Int. Cl.: C07D 413/10, A01N 43/74

(54) **1-CYCLOALKYLPYRAZOLYL-BENZOYL-DERIVATE**
1-CYCLOALKYLPYRAZOYL-BENZOYL DERIVATIVES
DERIVES DE 1-CYCLOALKYLPYRAZOLYL-BENZOYLE

(30) Priorität: 06.08.1999 DE 19936518
(43) Veröffentlichungstag der Anmeldung: 02.05.2002
(73) Patentinhaber: BASF AKTIENGESELLSCHAFT, 67056 Ludwigshafen (DE)
(72) Erfinder: NEIDLEIN, Ulf, D-68165 Mannheim (DE); GÖTZ, Norbert, D-67547 Worms (DE); MISSLITZ, Ulf, D-67433 Neustadt (DE); GÖTZ, Roland, D-68809 Neulu heim (DE); BAUMANN, Ernst, D-67373 Dudenhofen (DE); VON DEYN, Wolfgang, D-67435 Neustadt (DE); KUDIS, Steffen, D-68167 Mannheim (DE); LANGEMANN, Klaus, D-67551 Worms (DE); MAYER, Guido, D-67433 Neustadt (DE); WITSCHEL, Matthias, D-67061 Ludwigshafen (DE); OTTEN, Martina, D-67069 Ludwigshafen (DE); WESTPHALEN, Karl-Otto, D-67346 Speyer (DE); WALTER, Helmut, D-67283 Obrigheim (DE)
(86) Internationale Anmeldenummer: PCT/EP1999/009342
(87) Internationale Veröffentlichungsnummer: WO 2001/010864

(56) Entgegenhaltungen:
- WO-A-96/26206
- WO-A-98/42678
- WO-A-98/52926

## Beschreibung

Die vorliegende Erfindung betrifft 1-Cycloalkylpyrazolyl-benzoyl-Derivate der Formel I 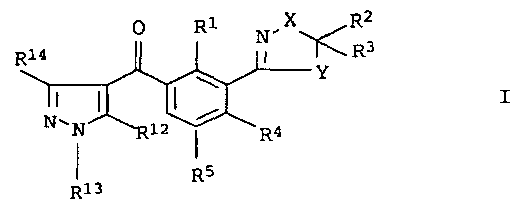 in der die Variablen folgende Bedeutung haben:
- X: O, NR⁶ oder CR⁷R⁸;
- Y: O, S, NR⁹ oder CR¹⁰R¹¹;
- R¹: Nitro, Halogen, C₁-C₄-Alkyl, oder C₁-C₄-Alkoxy;
- R²,R³,R⁷,R⁸,R¹⁰,R¹¹: Wasserstoff, C₁-C₄-Alkyl oder C₁-C₄-Halogenalkyl;
oder
R³ und R⁶ oder R³ und R⁸ oder R³ und R⁹ oder R³ und R¹¹ bilden gemeinsam eine Bindung aus;
- R⁴: Halogen, Nitro, C₁-C₄-Halogenalkyl, C₁-C₄-Alkoxy, C₁-C₄-Halogenalkoxy, C₁-C₄-Alkylthio oder C₁-C₄-Alkylsulfonyl;
- R⁵: Wasserstoff;
- R⁶,R⁹: Wasserstoff oder C₁-C₆-Alkyl;
- R¹²: Hydroxy, C₁-C₆-Alkoxy, C₃-C₆-Alkenyloxy, C₁-C₆-Alkylsulfonyloxy, C₁-C₆-Alkylcarbonyloxy, Phenylsulfonyloxy oder Phenylcarbonyloxy, wobei der Phenylrest der beiden letztgenannten Substituenten partiell oder vollständig halogeniert sein kann und/oder eine bis drei der folgenden Gruppen tragen kann:
Nitro, Cyano, C₁-C₄-Alkyl, C₁-C₄-Halogenalkyl, C₁-C₄-Alkoxy oder C₁-C₄-Halogenalkoxy;
- R¹³: C₃-C₆-Cycloalkyl;
- R¹⁴: Wasserstoff oder C₁-C₄-Alkyl;
sowie deren landwirtschaftlich brauchbaren Salze.

Außerdem betrifft die Erfindung Verfahren zur Herstellung von Verbindungen der Formel I, Mittel welche diese enthalten sowie die Verwendung dieser Derivate oder diese enthaltende Mittel zur Schadpflanzenbekämpfung.

Aus der Literatur, beispielsweise aus WO 98/42678 und WO 98/52926 sind Cycloalkyl-substituierte Pyrazolyl-benzoyl-Derivate bekannt, die in 3-Stellung des Benzoylrestes durch einen N-gebundenen Heterocyclus oder durch Phenyl substituiert sind.

Die herbiziden Eigenschaften der bisher bekannten Verbindungen sowie die Verträglichkeiten gegenüber Kulturpflanzen können jedoch nur bedingt befriedigen. Es lag daher dieser Erfindung die Aufgabe zugrunde, neue, insbesondere herbizid wirksame, Verbindungen mit verbesserten Eigenschaften zu finden.

Demgemäß wurden die 1-Cycloalkylpyrazolyl-benzoyl-Derivate der Formel I sowie deren herbizide Wirkung gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen, insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di(2-hydroxyeth-1-yl)ammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Die für die Substituenten R¹-R¹² oder als Reste an Phenylringen genannten organischen Molekülteile stellen Sammelbegriffe für individuelle Aufzählungen der einzelnen Gruppenmitglieder dar. Sämtliche Kohlenwasserstoffketten, also alle Alkyl-, Alkylcarbonyl-, Halogenalkyl-, Alkoxy-, Halogenalkoxy-, Alkylcarbonyloxy-, Alkylsulfonyloxy-, Alkylthio-, Halogenalkylthio-, Alkylsulfonyl-, Halogenalkylsulfonyl-, Alkenyl- und Alkenyloxy-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl, sowie die Alkylteile von C₁-C₄-Alkylcarbonyl und C₁-C₄-Alkylcarbonyloxy: z.B. Methyl, Ethyl, Propyl, 1-Methylethyl, Butyl, 1-Methylpropyl, 2-Methylpropyl und 1,1-Dimethylethyl;
- C₁-C₆-Alkyl, sowie die Alkylteile von C₁-C₆-Alkylcarbonyl und C₁-C₆-Alkylcarbonyloxy: C₁-C₄-Alkyl, wie voranstehend genannt, sowie z.B. Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- C₁-C₄-Halogenalkyl: einen C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, Brommethyl, Iodmethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₄-Alkoxy: z.B. Methoxy, Ethoxy, Propoxy, 1-Methylethoxy, Butoxy, 1-Methylpropoxy, 2-Methylpropoxy und 1,1-Dimethylethoxy;
- C₁-C₆-Alkoxy: C₁-C₄-Alkoxy wie voranstehend genannt, sowie z.B. Pentoxy, 1-Methylbutoxy, 2-Methylbutoxy, 3-Methoxylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
- C₁-C₄-Halogenalkoxy: einen C₁-C₄-Alkoxyrest wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy und Nonafluorbutoxy;
- C₁-C₄-Alkylthio: z.B. Methylthio, Ethylthio, Propylthio, 1-Methylethylthio, Butylthio, 1-Methylpropylthio, 2-Methylpropylthio und 1,1-Dimethylethylthio;
- C₁-C₄-Halogenalkylthio: einen C₁-C₄-Alkylthiorest wie voranstehend genannt, der partiell oder vollständig durch Fluor, Chlor, Brom und/oder Iod substituiert ist, also z.B. Fluormethylthio, Difluormethylthio, Trifluormethylthio, Chlordifluormethylthio, Bromdifluormethylthio, 2-Fluorethylthio, 2-Chlorethylthio, 2-Bromethylthio, 2-Iodethylthio, 2,2-Difluorethylthio, 2,2,2-Trifluorethylthio, 2,2,2-Trichlorethylthio, 2-Chlor-2-fluorethylthio, 2-Chlor-2,2-difluorethylthio, 2,2-Dichlor-2-fluorethylthio, Pentafluorethylthio, 2-Fluorpropylthio, 3-Fluorpropylthio, 2-Chlorpropylthio, 3-Chlorpropylthio, 2-Brompropylthio, 3-Brompropylthio, 2,2-Difluorpropylthio, 2,3-Difluorpropylthio, 2,3-Dichlorpropylthio, 3,3,3-Trifluorpropylthio, 3,3,3-Trichlorpropylthio, 2,2,3,3,3-Pentafluorpropylthio, Heptafluorpropylthio, 1-(Fluormethyl)-2-fluorethylthio, 1-(Chlormethyl)-2-chlorethylthio, 1-(Brommethyl)-2-bromethylthio, 4-Fluorbutylthio, 4-Chlorbutylthio, 4-Brombutylthio und Nonafluorbutylthio;
- C₁-C₄-Alkylsulfonyl (C₁-C₄-Alkyl-S(=O)₂-), sowie die Alkylsulfonylteile von C₁-C₄-Alkylsulfonyloxy: z.B. Methylsulfonyl, Ethylsulfonyl, Propylsulfonyl, 1-Methylethylsulfonyl, Butylsulfonyl, 1-Methylpropylsulfonyl, 2-Methylpropylsulfonyl und 1,1-Dimethylethylsulfonyl;
- C₁-C₆-Alkylsulfonyl, sowie die Alkylsulfonylteile von C₁-C₆-Alkylsulfonyloxy: einen C₁-C₄-Alkylsulfonylrest wie voranstehend genannt, sowie z.B. Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl;
- C₁-C₄-Halogenalkylsulfonyl: einen C₁-C₄-Alkylsulfonylrest wie voranstehend genannt, der partiell oder vollständig durch Fluor, Chlor, Brom und/oder Iod substituiert ist, also z.B. Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2-Chlor-2-fluorethylsulfonyl, 2-Chlor-2,2-difluorethylsulfonyl, 2,2-Dichlor-2-fluorethylsulfonyl, 2,2,2-Trichlorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl und Nonafluorbutylsulfonyl;
- C₃-C₆-Alkenyloxy: z.B. Prop-1-en-1-yloxy, Prop-2-en-1-yloxy, 1-Methylethenyloxy, Buten-1-yloxy, Buten-2-yloxy, Buten-3-yloxy, 1-Methyl-prop-1-en-1-yloxy, 2-Methyl-prop-1-en-1-yloxy, 1-Methyl-prop-2-en-1-yloxy, 2-Methyl-prop-2-en-1-yloxy, Penten-1-yloxy, Penten-2-yloxy, Penten-3-yloxy, Penten-4-yloxy, 1-Methyl-but-1-en-1-yloxy, 2-Methyl-but-1-en-1-yloxy, 3-Methyl-but-1-en-1-yloxy, 1-Methyl-but-2-en-1-yloxy, 2-Methyl-but-2-en-1-yloxy, 3-Methyl-but-2-en-1-yloxy, 1-Methyl-but-3-en-1-yloxy, 2-Methyl-but-3-en-1-yloxy, 3-Methyl-but-3-en-1-yloxy, 1,1-Dimethyl-prop-2-en-1-yloxy, 1,2-Dimethyl-prop-1-en-1-yloxy, 1,2-Dimethyl-prop-2-en-1-yloxy, 1-Ethyl-prop-1-en-2-yloxy, 1-Ethyl-prop-2-en-1-yloxy, Hex-1-en-1-yloxy, Hex-2-en-1-yloxy, Hex-3-en-1-yloxy, Hex-4-en-1-yloxy, Hex-5-en-1-yloxy, 1-Methyl-pent-1-en-1-yloxy, 2-Methyl-pent-1-en-1-yloxy, 3-Methyl-pent-1-en-1-yloxy, 4-Methyl-pent-1-en-1-yloxy, 1-Methyl-pent-2-en-1-yloxy, 2-Methyl-pent-2-en-1-yloxy, 3-Methyl-pent-2-en-1-yloxy, 4-Methylpent-2-en-1-yloxy, 1-Methyl-pent-3-en-1-yloxy, 2-Methylpent-3-en-1-yloxy, 3-Methyl-pent-3-en-1-yloxy, 4-Methylpent-3-en-1-yloxy, 1-Methyl-pent-4-en-1-yloxy, 2-Methylpent-4-en-1-yloxy, 3-Methyl-pent-4-en-1-yloxy, 4-Methylpent-4-en-1-yloxy, 1,1-Dimethyl-but-2-en-1-yloxy, 1,1-Dimethyl-but-3-en-1-yloxy, 1,2-Dimethyl-but-1-en-1-yloxy, 1,2-Dimethyl-but-2-en-1-yloxy, 1,2-Dimethyl-but-3-en-1-yloxy, 1,3-Dimethyl-but-1-en-1-yloxy, 1,3-Dimethyl-but-2-en-1-yloxy, 1,3-Dimethyl-but-3-en-1-yloxy, 2,2-Dimethyl-but-3-en-1-yloxy, 2,3-Dimethyl-but-1-en-1-yloxy, 2,3-Dimethyl-but-2-en-1-yloxy, 2,3-Dimethyl-but-3-en-1-yloxy, 3,3-Dimethyl-but-1-en-1-yloxy, 3,3-Dimethyl-but-2-en-1-yloxy, 1-Ethyl-but-1-en-1-yloxy, 1-Ethyl-but-2-en-1-yloxy, 1-Ethyl-but-3-en-1-yloxy, 2-Ethylbut-1-en-1-yloxy, 2-Ethyl-but-2-en-1-yloxy, 2-Ethylbut-3-en-1-yloxy, 1,1,2-Trimethyl-prop-2-en-1-yloxy, 1-Ethyl-1-methyl-prop-2-en-1-yloxy, 1-Ethyl-2-methylprop-1-en-1-yloxy und 1-Ethyl-2-methyl-prop-2-en-1-yloxy;
- C₃-C₆-Alkenyl: Prop-1-en-1-yl, Prop-2-en-1-yl, 1-Methylethenyl, Buten-1-yl, Buten-2-yl, Buten-3-yl, 1-Methylprop-1-en-1-yl, 2-Methyl-prop-1-en-1-yl, 1-Methyl-prop-2-en-1-yl, 2-Methyl-prop-2-en-1-yl, Penten-1-yl, Penten-2-yl, Penten-3-yl, Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-D,-methyl-prop-2-en-1-yl, 1-Ethyl-prop-1-en-2-yl, 1-Ethyl-prop-2-en-1-yl, Hex-1-en-1-yl, Hex-2-en-1-yl, Hex-3-en-1-yl, Hex-4-en-1-yl, Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethylbut-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methylprop-1-en-1-yl und 1-Ethyl-2-methyl-prop-2-en-1-yl;
- C₃-C₆-Cycloalkyl: Cyclopropyl, Cyclobutyl, Cyclopentyl oder Cyclohexyl;

Die Phenylringe sind vorzugsweise unsubstituiert oder tragen ein bis drei Halogenatome und/oder eine Nitrogruppe, eine Cyanogruppe, eine oder zwei Methyl-, Trifluormethyl-, Methoxy- oder Trifluormethoxygruppen.

Zu betonen sind die 1-Cycloalkylpyrazolyl-benzoyl-Derivate der Formel I, in der die Variablen, sowohl für sich allein wie auch in Kombination miteinander, folgende Bedeutung haben:
- X: O, NR⁶ oder CR⁷R⁸;
- Y: O, S, NR⁹ oder CR¹⁰R¹¹;
- R¹: Nitro, Halogen, C₁-C₄-Alkyl oder C₁-C₄-Alkoxy;
insbesonders bevorzugt Halogen, wie Fluor, Chlor oder Brom, C₁-C₄-Alkyl, wie Methyl oder Ethyl, oder C₁-C₄-Alkoxy, wie Methoxy oder Ethoxy;
insbesonderst bevorzugt Chlor, Methyl oder Methoxy;
- R²,R³,R⁷,R⁸,R¹⁰,R¹¹: Wasserstoff, C₁-C₄-Alkyl oder C₁-C₄-Halogenalkyl;
insbesonders bevorzugt Wasserstoff, Methyl, Ethyl, Propyl, 1-Methylethyl, Fluormethyl oder Chlormethyl;
insbesonderst bevorzugt Wasserstoff, Methyl, Ethyl oder Chlormethyl;
oder
R³ und R⁶ oder R³ und R⁸ oder R³ und R⁹ oder R³ und R¹¹ bilden gemeinsam eine Bindung aus;
- R⁴: Halogen, Nitro, C₁-C₄-Halogenalkyl, C₁-C₄-Alkoxy, C₁-C₄-Halogenalkoxy, C₁-C₄-Alkylthio oder C₁-C₄-Alkylsulfonyl;
insbesonders bevorzugt Halogen, wie Chlor oder Brom, Nitro, C₁-C₂-Halogenalkyl, wie Difluormethyl oder Trifluormethyl, C₁-C₂-Alkoxy, wie Methoxy oder Ethoxy, C₁-C₂-Halogenalkoxy, wie Difluormethoxy, Chlordifluormethoxy oder Trifluormethoxy, C₁-C₃-Alkylthio, wie Methylthio, Ethylthio oder 1-Methyl-1-ethylthio oder C₁-C₃-Alkylsulfonyl, wie Methylsulfonyl, Ethylsulfonyl, 1-Methylethylsulfonyl oder Propylsulfonyl;
- R⁵: Wasserstoff;
- R⁶, R⁹: Wasserstoff oder C₁-C₄-Alkyl;
insbesondere bevorzugt Wasserstoff, Methyl oder Ethyl;
insbesonderst bevorzugt Methyl;
- R¹²: Hydroxy, C₁-C₆-Alkoxy, C₃-C₆-Alkenyloxy, C₁-C₆-Alkylsulfonyloxy, C₁-C₆-Alkylcarbonyloxy, Phenylsulfonyloxy oder Phenylcarbonyloxy, wobei der Phenylrest der beiden letztgenannten Substituenten partiell oder vollständig halogeniert sein kann und/oder eine bis drei der folgenden Gruppen tragen kann:
Nitro, Cyano, C₁-C₄-Alkyl, C₁-C₄-Halogenalkyl, C₁-C₄-Alkoxy oder C₁-C₄-Halogenalkoxy;
insbesonders bevorzugt Hydroxy, C₁-C₄-Alkoxy, C₃-C₆-Alkenyloxy, C₁-C₄-Alkylsulfonyloxy, C₁-C₄-Alkylcarbonyloxy, Phenylsulfonyloxy oder Phenylcarbonyloxy, wobei der Phenylrest der beiden letztgenannte Substituenten partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy;
- R¹³: C₃-C₆-Cycloalkyl;
- R¹⁴: Wasserstoff oder C₁-C₄-Alkyl;
besonders bevorzugt Wasserstoff oder Methyl;

- Bevorzugt sind die 1-Cycloalkylpyrazolyl-benzoyl-Derivate der Formel I, in der die Variablen folgende Bedeutung haben:
   - X: O
   - R¹: Nitro, Halogen, C₁-C₄-Alkyl oder C₁-C₄-Alkoxy;
   besonders bevorzugt Halogen, C₁-C₄-Alkyl oder C₁-C₄-Alkoxy;
   insbesonders bevorzugt Halogen, wie Fluor, Chlor oder Brom, Methyl oder Ethyl oder Methoxy oder Ethoxy;
   insbesonderst bevorzugt Chlor, Methyl oder Methoxy;
   - R⁴: Halogen, Nitro, C₁-C₄-Halogenalkyl, C₁-C₄-Alkoxy, C₁-C₄-Halogenalkoxy, C₁-C₄-Alkylthio oder C₁-C₄-Alkylsulfonyl;
   besonders bevorzugt Halogen, wie Chlor oder Brom, Nitro, C₁-C₂-Halogenalkyl, wie Difluormethyl oder Trifluormethyl, C₁-C₂-Alkoxy, C₁-C₂-Halogenalkoxy, wie Difluormethoxy, Chlordifluormethoxy oder Trifluormethoxy, C₁-C₂-Alkylthio wie Methylthio oder Ethylthio oder C₁-C₂-Alkylsulfonyl wie Methylsulfonyl oder Ethylsulfonyl.

   Insbesondere bevorzugt sind die 1-Cycloalkylpyrazolyl-benzoyl-Derivate der Formel I, wobei
   - X: O;
   - Y: CR¹⁰R¹¹;
   - R¹: Halogen, C₁-C₄-Alkyl oder C₁-C₄-Alkoxy;
   besonders bevorzugt Halogen wie Fluor, Chlor oder Brom, Methyl oder Ethyl oder Methoxy oder Ethoxy;
   insbesondere bevorzugt Chlor, Methyl oder Methoxy;
   - R²,R³,R¹⁰,R¹¹: Wasserstoff, C₁-C₄-Alkyl;
   oder R³ und R¹¹ bilden gemeinsam eine Bindung aus;
   besonders bevorzugt Wasserstoff, Methyl oder Ethyl;
   oder R³ und R¹¹ bilden gemeinsam eine Bindung aus;
   - R⁴: Nitro, C₁-C₄-Halogenalkyl, C₁-C₄-Alkoxy oder C₁-C₄-Halogenalkoxy;
   besonders bevorzugt Nitro, C₁-C₂-Halogenalkyl, wie Difluormethyl oder Trifluormethyl, C₁-C₂-Alkoxy oder C₁-C₂-Halogenalkoxy, wie Difluormethoxy;
   - R⁵: Wasserstoff;
   - R¹²: Hydroxy, C₁-C₆-Alkylsulfonyloxy, C₁-C₆-Alkylcarbonyloxy, Phenylsulfonyloxy oder Phenylcarbonyloxy, wobei der Phenylrest der beiden letztgenannten Substituenten partiell oder vollständig halogeniert sein kann und/oder eine bis drei der folgenden Gruppen tragen kann:
   Nitro, Cyano, C₁-C₄-Alkyl, C₁-C₄-Halogenalkyl, C₁-C₄-Alkoxy oder C₁-C₄-Halogenalkoxy;
   besonders bevorzugt Hydroxy;
   - R¹³: C₃-C₆-Cycloalkyl;
   bedeuten.
   Außerordentlich bevorzugt sind die 1-Cycloalkylpyrazolylbenzoyl-Derivate der Formel I, wobei
   - X: 0;
   - Y: CR¹⁰R¹¹;
   - R²,R³,R¹⁰,R¹¹: Wasserstoff oder Methyl oder Ethyl; bevorzugt Wasserstoff oder Methyl;
   bedeuten.
   Ebenso außerordentlich bevorzugt sind die 1-Cycloalkylpyrazolylbenzoyl-Derivate der Formel I, wobei
   - X: 0;
   - Y: CR¹⁰R¹¹;
   - R²,R¹⁰: Wasserstoff oder Methyl oder Ethyl;
   bevorzugt Wasserstoff oder Methyl;
   - R³ und R¹¹: bilden gemeinsam eine Bindung aus;
   bedeuten.
   Ebenso insbesondere bevorzugt sind die 1-Cycloalkylpyrazolyl-benzoyl-Derivate der Formel I, wobei
   - X: O;
   - Y: CR¹⁰R¹¹;
   - R¹: Halogen, C₁-C₄-Alkyl oder C₁-C₄-Alkoxy;
   besonders bevorzugt Halogen wie Fluor, Chlor oder Brom, Methyl oder Ethyl oder Methoxy oder Ethoxy;
   insbesondere bevorzugt Chlor, Methyl oder Methoxy;
   - R²,R³,R¹⁰,R¹¹: Wasserstoff oder C₁-C₄-Alkyl;
   oder R³ und R¹¹ bilden gemeinsam eine Bindung aus;
   besonders bevorzugt Wasserstoff, Methyl oder Ethyl;
   oder R³ und R¹¹ bilden gemeinsam eine Bindung aus;
   - R⁴: Halogen, C₁-C₄-Alkylthio oder C₁-C₄-Alkylsulfonyl;
   insbesondere bevorzugt Chlor oder Brom, Methylthio, Ethylthio oder 1-Methyl-1-ethylthio oder Methylsulfonyl, Ethylsulfonyl, 1-Methylethylsulfonyl oder Propylsulfonyl;
   - R⁵: Wasserstoff;
   - R¹²: Hydroxy, C₁-C₆-Alkylsulfonyloxy, C₁-C₆-Alkylcarbonyloxy, Phenylsulfonyloxy oder Phenylcarbonyloxy, wobei der Phenylrest der beiden letztgenannten Substituenten partiell oder vollständig halogeniert sein kann und/oder eine bis drei der folgenden Gruppen tragen kann:
   Nitro, Cyano, C₁-C₄-Alkyl, C₁-C₄-Halogenalkyl, C₁-C₄-Alkoxy oder C₁-C₄-Halogenalkoxy;
   besonders bevorzugt Hydroxy;
   - R¹³: C₃-C₆-Cycloalkyl;
   bedeuten.
   Außerordentlich bevorzugt sind die 1-Cycloalkylpyrazolylbenzoyl-Derivate der Formel I, wobei
   - X: 0;
   - Y: CR¹⁰R¹¹;
   - R²,R³,R¹⁰,R¹¹: Wasserstoff oder Methyl oder Ethyl; bevorzugt Wasserstoff oder Methyl;
   bedeuten.
   Ebenso außerordentlich bevorzugt sind die 1-Cycloalkylpyrazolylbenzoyl-Derivate der Formel I, wobei
   - X: 0;
   - Y: CR¹⁰R¹¹;
   - R²,R¹⁰: Wasserstoff oder Methyl oder Ethyl;
   bevorzugt Wasserstoff oder Methyl;
   - R³ und R¹¹: bilden gemeinsam eine Bindung aus;
   bedeuten.
- Ebenso bevorzugt sind die 1-Cycloalkylpyrazolyl-benzoyl-Derivate der Formel I, in der die Variablen folgende Bedeutung haben:
   - X: NR⁶;
   - R¹: Nitro, Halogen, C₁-C₄-Alkyl oder C₁-C₄-Alkoxy;
   besonders bevorzugt Halogen, C₁-C₄-Alkyl oder C₁-C₄-Alkoxy;
   insbesonders bevorzugt Halogen, wie Fluor, Chlor oder Brom, Methyl oder Ethyl oder Methoxy oder Ethoxy;
   insbesonderst bevorzugt Chlor, Methyl oder Methoxy;
   - R⁴: Halogen, Nitro, C₁-C₄-Halogenalkyl, C₁-C₄-Alkoxy, C₁-C₄-Halogenalkoxy, C₁-C₄-Alkylthio oder C₁-C₄-Alkylsulfonyl;
   besonders bevorzugt Halogen, wie Chlor oder Brom, Nitro, C₁-C₂-Halogenalkyl, wie Difluormethyl oder Trifluormethyl, C₁-C₂-Alkoxy, wie Methoxy oder Ethoxy, C₁-C₂-Halogenalkoxy wie Difluormethoxy, Chlordifluormethoxy oder Trifluormethoxy, C₁-C₂-Alkylthio wie Methylthio oder Ethylthio oder C₁-C₂-Alkylsulfonyl, wie Methylsulfonyl oder Ethylsulfonyl;

   Insbesondere bevorzugt sind die 1-Cycloalkylpyrazolyl-benzoyl-Derivate der Formel I, wobei
   - X: N(C₁-C₆-Alkyl);
   besonders bevorzugt N-Methyl, N-Ethyl, N-(1-Methyl-1-ethyl) oder N-Propyl;
   - Y: NR⁹ oder CR¹⁰R¹¹;
   - R¹: Halogen, C₁-C₄-Alkyl oder C₁-C₄-Alkoxy;
   besonders bevorzugt Halogen wie Fluor, Chlor oder Brom, Methyl oder Ethyl oder Methoxy oder Ethoxy;
   insbesondere bevorzugt Chlor, Methyl oder Methoxy;
   - R²,R³,R¹⁰,R¹¹: Wasserstoff oder C₁-C₄-Alkyl;
   oder R³ und R⁹ oder R³ und R¹¹ bilden gemeinsam eine Bindung aus;
   besonders bevorzugt Wasserstoff, Methyl oder Ethyl;
   oder R³ und R⁹ oder R³ und R¹¹ bilden gemeinsam eine Bindung aus;
   - R⁴: Nitro, C₁-C₄-Halogenalkyl, C₁-C₄-Alkoxy oder C₁-C₄-Halogenalkoxy;
   besonders bevorzugt Nitro, C₁-C₂-Halogenalkyl, wie Difluormethyl oder Trifluormethyl, C₁-C₂-Alkoxy oder C₁-C₂-Halogenalkoxy, wie Difluormethoxy;
   - R⁵: Wasserstoff;
   - R⁹: Wasserstoff oder C₁-C₄-Alkyl;
   besonders bevorzugt C₁-C₄-Alkyl;
   insbesondere bevorzugt Methyl oder Ethyl;
   - R¹²: Hydroxy, C₁-C₆-Alkylsulfonyloxy, C₁-C₆-Alkylcarbonyloxy, Phenylsulfonyloxy oder Phenylcarbonyloxy, wobei der Phenylrest der beiden letztgenannten Substituenten partiell oder vollständig halogeniert sein kann und/oder eine bis drei der folgenden Gruppen tragen kann:
   Nitro, Cyano, C₁-C₄-Alkyl, C₁-C₄-Halogenalkyl, C₁-C₄-Alkoxy oder C₁-C₄-Halogenalkoxy;
   besonders bevorzugt Hydroxy;
   - R¹³: C₃-C₆-Cycloalkyl;
   bedeuten.
   Außerordentlich bevorzugt sind die 1-Cycloalkylpyrazolyl-benzoyl-Derivate der Formel I, wobei
   - X: N-(C₁-C₆-Alkyl);
   besonders bevorzugt N-Methyl, N-Ethyl, N-(1-Methyl-1-ethyl) oder N-Propyl;
   - Y: CR¹⁰R¹¹;
   - R²,R³,R¹⁰,R¹¹: Wasserstoff oder C₁-C₄-Alkyl;
   bevorzugt Wasserstoff, Methyl oder Ethyl;
   insbesondere bevorzugt Wasserstoff oder Methyl;
   bedeuten.
   Ebenso außerordentlich bevorzugt sind die 1-Cycloalkylpyrazolyl-benzoyl-Derivate der Formel I, wobei
   - X: N-(C₁-C₆-Alkyl);
   besonders bevorzugt N-Methyl, N-Ethyl, N-(1-Methyl-1-ethyl) oder N-Propyl;
   - Y: NR⁹ oder CR¹⁰R¹¹;
   - R², R¹⁰: Wasserstoff oder C₁-C₄-Alkyl;
   bevorzugt Wasserstoff, Methyl oder Ethyl;
   insbesondere bevorzugt Wasserstoff oder Methyl;
   R³ und R⁹ oder R³ und R¹¹ bilden gemeinsam eine Bindung aus;
   bedeuten.
   Ebenso insbesondere bevorzugt sind die 1-Cycloalkylpyrazolyl-benzoyl-Derivate der Formel I, wobei
   - X: N(C₁-C₆-Alkyl);
   besonders bevorzugt N-Methyl, N-Ethyl, N-(1-Methyl-1-ethyl) oder N-Propyl;
   - Y: NR⁹ oder CR¹⁰R¹¹;
   - R¹: Halogen, C₁-C₄-Alkyl oder C₁-C₄-Alkoxy;
   besonders bevorzugt Halogen wie Fluor, Chlor oder Brom, Methyl oder Ethyl oder Methoxy oder Ethoxy;
   insbesondere bevorzugt Chlor, Methyl oder Methoxy;
   - R²,R³,R¹⁰,R¹¹: Wasserstoff oder C₁-C₄-Alkyl;
   oder R³ und R⁹ oder R³ und R¹¹ bilden gemeinsam eine Bindung aus;
   besonders bevorzugt Wasserstoff, Methyl oder Ethyl;
   oder R³ und R⁹ oder R³ und R¹¹ bilden gemeinsam eine Bindung aus;
   - R⁴: Halogen, C₁-C₄-Alkylthio oder C₁-C₄-Alkylsulfonyl; insbesondere bevorzugt Chlor oder Brom, Methylthio, Ethylthio oder 1-Methyl-1-ethylthio oder Methylsulfonyl, Ethylsulfonyl, 1-Methylethylsulfonyl oder Propylsulfonyl;
   - R⁵: Wasserstoff;
   - R⁹: Wasserstoff oder C₁-C₄-Alkyl;
   besonders bevorzugt C₁-C₄-Alkyl;
   insbesondere bevorzugt Methyl oder Ethyl;
   - R¹²: Hydroxy, C₁-C₆-Alkylsulfonyloxy, C₁-C₆-Alkylcarbonyloxy, Phenylsulfonyloxy oder Phenylcarbonyloxy, wobei der Phenylrest der beiden letztgenannten Substituenten partiell oder vollständig halogeniert sein kann und/oder eine bis drei der folgenden Gruppen tragen kann:
   Nitro, Cyano, C₁-C₄-Alkyl, C₁-C₄-Halogenalkyl, C₁-C₄-Alkoxy oder C₁-C₄-Halogenalkoxy;
   besonders bevorzugt Hydroxy;
   - R¹³: C₃-C₆-Cycloalkyl;
   bedeuten.
   Außerordentlich bevorzugt sind die 1-Cycloalkylpyrazolyl-benzoyl-Derivate der Formel I, wobei
   - X: N-(C₁-C₆-Alkyl);
   besonders bevorzugt N-Methyl, N-Ethyl, N-(1-Methyl-1-ethyl) oder N-Propyl;
   - Y: CR¹⁰R¹¹;
   - R²,R³,R¹⁰,R¹¹: Wasserstoff oder C₁-C₄-Alkyl;
   bevorzugt Wasserstoff, Methyl oder Ethyl;
   insbesondere bevorzugt Wasserstoff oder Methyl;
   bedeuten.
   Ebenso außerordentlich bevorzugt sind die 1-Cycloalkylpyrazolyl-benzoyl-Derivate der Formel I, wobei
   - X: N-(C₁-C₆-Alkyl);
   besonders bevorzugt N-Methyl, N-Ethyl, N-(1-Methyl-1-ethyl) oder N-Propyl;
   - Y: NR⁹ oder CR¹⁰R¹¹;
   - R²,R¹⁰: Wasserstoff oder C₁-C₄-Alkyl;
   bevorzugt Wasserstoff, Methyl oder Ethyl;
   insbesondere bevorzugt Wasserstoff oder Methyl;
   R³ und R⁹ oder R³ und R¹¹ bilden gemeinsam eine Bindung aus;
   bedeuten.
   Ebenso insbesondere bevorzugt sind die 1-Cycloalkylpyrazolyl-benzoyl-Derivate der Formel I, wobei
   - X: NR⁶;
   - Y: O, S oder N(C₁-C₆-Alkyl);
   besonders bevorzugt O, S oder N-Methyl, N-Ethyl, N-(1-Methyl-1-ethyl) oder N-Propyl;
   - R¹: Halogen, C₁-C₄-Alkyl oder C₁-C₄-Alkoxy;
   besonders bevorzugt Halogen, wie Fluor, Chlor oder Brom, Methyl oder Ethyl oder Methoxy oder Ethoxy;
   insbesondere bevorzugt Chlor, Methyl oder Methoxy;
   - R²: Wasserstoff oder C₁-C₄-Alkyl;
   besonders bevorzugt Wasserstoff, Methyl oder Ethyl;
   - R³ und R⁶: bilden gemeinsam eine Bindung aus;
   - R⁵: Wasserstoff;
   - R¹²: Hydroxy, C₁-C₆-Alkylsulfonyloxy, C₁-C₆-Alkylcarbonyloxy, Phenylsulfonyloxy oder Phenylcarbonyloxy, wobei der Phenylrest der beiden letztgenannten Substituenten partiell oder vollständig halogeniert sein kann und/oder eine bis drei der folgenden Gruppen tragen kann:
   Nitro, Cyano, C₁-C₄-Alkyl, C₁-C₄-Halogenalkyl, C₁-C₄-Alkoxy oder C₁-C₄-Halogenalkoxy;
   besonders bevorzugt Hydroxy;
   - R¹³: C₃-C₆-Cycloalkyl;
   bedeuten.
   Außerordentlich bevorzugt sind die 1-Cycloalkylpyrazolyl-benzoyl-Derivate der Formel I, wobei
   - R⁴: Nitro, C₁-C₄-Halogenalkyl, C₁-C₄-Alkoxy oder C₁-C₄-Halogenalkoxy;
   besonders bevorzugt Nitro, C₁-C₂-Halogenalkyl, wie Difluormethyl oder Trifluormethyl, C₁-C₂-Alkoxy oder C₁-C₂-Halogenalkoxy, wie Difluormethoxy;
   bedeutet.
   Ebenso außerordentlich bevorzugt sind die 1-Cycloalkylpyrazolyl-benzoyl-Derivate der Formel I, wobei
   - R⁴: Halogen, C₁-C₄-Alkylthio oder C₁-C₄-Alkylsulfonyl, insbesondere bevorzugt Chlor oder Brom, Methylthio, Ethylthio oder 1-Methyl-1-ethylthio oder Methylsulfonyl, Ethylsulfonyl, 1-Methylethylsulfonyl oder Propylsulfonyl;
   bedeuten.
- Ebenso bevorzugt sind die 1-Cycloalkylpyrazolyl-benzoyl-Derivate der Formel I, in der die Variablen folgende Bedeutung haben:
   - X: CR⁷R⁸;
   - R¹: Nitro, Halogen, C₁-C₄-Alkyl oder C₁-C₄-Alkoxy;
   besonders bevorzugt Halogen, C₁-C₄-Alkyl oder C₁-C₄-Alkoxy;
   insbesonders bevorzugt Halogen, wie Fluor, Chlor oder Brom, Methyl oder Ethyl oder Methoxy oder Ethoxy;
   insbesonderst bevorzugt Chlor, Methyl oder Methoxy;
   - R⁴: Halogen, Nitro, C₁-C₄-Halogenalkyl, C₁-C₄-Alkoxy, C₁-C₄-Halogenalkoxy, C₁-C₄-Alkylthio oder C₁-C₄-Alkylsulfonyl;
   besonders bevorzugt Halogen, wie Chlor oder Brom, Nitro, C₁-C₂-Halogenalkyl, wie Difluormethyl oder Trifluormethyl, C₁-C₂-Alkoxy, wie Methoxy oder Ethoxy, C₁-C₂-Halogenalkoxy wie Difluormethoxy, Chlordifluormethoxy oder Trifluormethoxy, C₁-C₂-Alkylthio, wie Methylthio oder Ethylthio oder C₁-C₂-Alkylsulfonyl, wie Methylsulfonyl oder Ethylsulfonyl;

   Insbesondere bevorzugt sind die 1-Cycloalkylpyrazolyl-benzoyl-Derivate der Formel I, wobei
   - X: CR⁷R⁸;
   - Y: O, S oder NR⁹;
   - R¹: Halogen, C₁-C₄-Alkyl oder C₁-C₄-Alkoxy;
   besonders bevorzugt Halogen wie Fluor, Chlor oder Brom, Methyl oder Ethyl, Methoxy oder Ethoxy;
   insbesondere bevorzugt Chlor, Methyl oder Methoxy;
   - R²,R³,R⁷,R⁸: Wasserstoff oder C₁-C₄-Alkyl;
   oder R³ und R⁸ bilden gemeinsam eine Bindung aus;
   besonders bevorzugt Wasserstoff, Methyl oder Ethyl;
   oder R³ und R⁸ bilden gemeinsam eine Bindung aus;
   - R⁴: Nitro, C₁-C₄-Halogenalkyl, C₁-C₄-Alkoxy oder C₁-C₄-Halogenalkoxy;
   besonders bevorzugt Nitro, C₁-C₂-Halogenalkyl, wie Difluormethyl oder Trifluormethyl, C₁-C₂-Alkoxy oder C₁-C₂-Halogenalkoxy wie Difluormethyloxy;
   - R⁵: Wasserstoff;
   - R⁹: Wasserstoff oder C₁-C₄-Alkyl;
   besonders bevorzugt C₁-C₄-Alkyl;
   - R¹²: Hydroxy, C₁-C₆-Alkylsulfonyloxy, C₁-C₆-Alkylcarbonyloxy, Phenylsulfonyloxy oder Phenylcarbonyloxy, wobei der Phenylrest der beiden letztgenannten Substituenten partiell oder vollständig halogeniert sein kann und/oder eine bis drei der folgenden Gruppen tragen kann:
   Nitro, Cyano, C₁-C₄-Alkyl, C₁-C₄-Halogenalkyl, C₁-C₄-Alkoxy oder C₁-C₄-Halogenalkoxy;
   besonders bevorzugt Hydroxy;
   - R¹³: C₃-C₆-Cycloalkyl;
   bedeuten.
   Außerordentlich bevorzugt sind die 1-Cycloalkylpyrazolyl-benzoyl-Derivate der Formel I, wobei
   - X: CR⁷R⁸;
   - Y: O, S oder NR⁹;
   - R²,R³,R⁷,R⁸: Wasserstoff oder C₁-C₄-Alkyl;
   bevorzugt Wasserstoff, Methyl oder Ethyl;
   insbesondere bevorzugt Wasserstoff oder Methyl;
   - R⁹: Wasserstoff oder C₁-C₄-Alkyl; bevorzugt C₁-C₄-Alkyl;
   bedeuten.
   Ebenso außerordentlich bevorzugt sind die 1-Cycloalkylpyrazolyl-benzoyl-Derivate der Formel I, wobei
   - X: CR⁷R⁸;
   - Y: O, S oder NR⁹;
   - R²,R⁷: Wasserstoff oder C₁-C₄-Alkyl;
   bevorzugt Wasserstoff, Methyl oder Ethyl;
   insbesondere bevorzugt Wasserstoff oder Methyl;
   - R³ und R⁸: bilden gemeinsam eine Bindung aus;
   - R⁹: Wasserstoff oder C₁-C₄-Alkyl;
   bevorzugt C₁-C₄-Alkyl;
   bedeuten;
   Besonders außerordentlich bevorzugt sind hierbei die 1-Cycloalkylpyrazolyl-benzoyl-Derivate der Formel I, wobei Y für S steht.
   Ebenso insbesondere bevorzugt sind die 1-Cycloalkylpyrazolyl-benzoyl-Derivate der Formel I, wobei
   - X: CR⁷R⁸;
   - Y: O, S oder NR⁹;
   - R¹: Halogen, C₁-C₄-Alkyl oder C₁-C₄-Alkoxy;
   besonders bevorzugt Halogen wie Fluor, Chlor oder Brom, Methyl oder Ethyl oder Methoxy oder Ethoxy;
   insbesondere bevorzugt Chlor, Methyl oder Methoxy;
   - R²,R³,R⁷,R⁸: Wasserstoff oder C₁-C₄-Alkyl;
   oder R³ und R⁸ bilden gemeinsam eine Bindung aus;
   besonders bevorzugt Wasserstoff, Methyl oder Ethyl; oder R³ und R⁸ bilden gemeinsam eine Bindung aus;
   - R⁴: Halogen, C₁-C₄-Alkylthio oder C₁-C₄-Alkylsulfonyl;
   insbesondere bevorzugt Chlor oder Brom, Methylthio, Ethylthio oder 1-Methyl-1-ethylthio oder Methylsulfonyl, Ethylsulfonyl, 1-Methylethylsulfonyl oder Propylsulfonyl;
   - R⁵: Wasserstoff;
   - R⁹: Wasserstoff oder C₁-C₄-Alkyl;
   besonders bevorzugt C₁-C₄-Alkyl;
   insbesondere bevorzugt Methyl oder Ethyl;
   - R¹²: Hydroxy, C₁-C₆-Alkylsulfonyloxy, C₁-C₆-Alkylcarbonyloxy, Phenylsulfonyloxy oder Phenylcarbonyloxy, wobei der Phenylrest der beiden letztgenannten Substituenten partiell oder vollständig halogeniert sein kann und/oder eine bis drei der folgenden Gruppen tragen kann:
   Nitro, Cyano, C₁-C₄-Alkyl, C₁-C₄-Halogenalkyl, C₁-C₄-Alkoxy oder C₁-C₄-Halogenalkoxy;
   besonders bevorzugt Hydroxy;
   - R¹³: C₃-C₆-Cycloalkyl;
   bedeuten.
   Außerdem bevorzugt sind die 1-Cycloalkylpyrazolyl-benzoyl-Derivate der Formel I, wobei
   - X: CR⁷R⁸;
   - Y: O, S oder NR⁹;
   - R²,R³,R⁷,R⁸: Wasserstoff oder C₁-C₄-Alkyl;
   bevorzugt Wasserstoff, Methyl oder Ethyl;
   insbesondere bevorzugt Wasserstoff oder Methyl;
   - R⁹: Wasserstoff oder C₁-C₄-Alkyl;
   bevorzugt C₁-C₄-Alkyl;
   bedeuten.
   Ebenso außerordentlich bevorzugt sind die 1-Cycloalkylpyrazolyl-benzoyl-Derivate der Formel I, wobei
   - X: CR⁷R⁸;
   - Y: O, S oder NR⁹;
   - R²,R⁷: Wasserstoff oder C₁-C₄-Alkyl;
   bevorzugt Wasserstoff, Methyl oder Ethyl;
   insbesondere bevorzugt Wasserstoff oder Methyl;
   - R³ und R⁸: bilden gemeinsam eine Bindung aus;
   - R⁹: Wasserstoff oder C₁-C₄-Alkyl;
   bevorzugt C₁-C₄-Alkyl;
   bedeuten;
   Besonders außerordentlich bevorzugt sind hierbei die 1-Cycloalkylpyrazolyl-benzoyl-Derivate der Formel I, wobei Y für S steht.
- Ebenso bevorzugt sind die 1-Cycloalkylpyrazolyl-benzoyl-Derivate der Formel I, in der die Variablen folgende Bedeutung haben:
   - X: O;
   - Y: CR¹⁰R¹¹;
   - R¹: Halogen, C₁-C₄-Alkyl oder C₁-C₄-Alkoxy;
   besonders bevorzugt Chlor, Methyl oder Methoxy;
   ebenso besonders bevorzugt Halogen oder C₁-C₄-Alkyl;
   insbesondere bevorzugt Chlor oder Methyl;
   - R²,R³,R¹⁰,R¹¹: Wasserstoff;
   - R⁴: C₁-C₄-Alkylsulfonyl;
   besonders bevorzugt Methylsulfonyl;
   - R⁵: Wasserstoff;
   - R¹²: Hydroxy, C₁-C₆-Alkoxy, Phenylsulfonyloxy oder Phenylcarbonyloxy, wobei der Phenylrest der beiden letztgenannten Substituenten partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy;
   besonders bevorzugt Hydroxy, C₁-C₆-Alkoxy oder Phenylcarbonyloxy, wobei der Phenylrest partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy;
   - R¹³: C₃-C₆-Cycloalkyl;
   - R¹⁴: Wasserstoff;
- Ebenso bevorzugt sind die 1-Cycloalkylpyrazolyl-benzoyl-Derivate der Formel I, in der die Variablen folgende Bedeutung haben:
   - X: O;
   - Y: CR¹⁰R¹¹;
   - R¹: Halogen, C₁-C₄-Alkyl oder C₁-C₄-Alkoxy;
   - R⁴: C₁-C₄-Alkylsulfonyl;
   - R¹³: C₃-C₆-Cycloalkyl.

Außerordentlichst bevorzugt sind die Verbindungen der Formel Ia1 (≡I mit R¹ = Cl; R¹³ = cyclo-C₃H₅ und R⁵ und R¹⁴ = H), insbesondere die Verbindungen Ia1.1 bis Ia1.765 der Tabelle 1, wobei die Restedefinitionen X, Y und R¹ bis R¹⁴ nicht nur in Kombination miteinander, sondern auch jeweils für sich allein betrachtet für die erfindungsgemäßen verbindungen eine besondere Bedeutung haben.

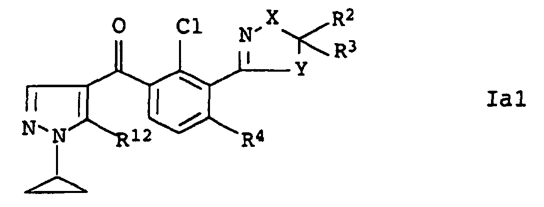

**Tabelle 1:**

| Nr. | X | R² | R³ | Y | R⁴ | R¹² |
|---|---|---|---|---|---|---|
| Ia1.1 | O | H | H | CH₂ | SCH₃ | OH |
| Ia1.2 | O | H | H | CH₂ | SCH₂CH₃ | OH |
| Ia1.3 | O | H | H | CH₂ | SO₂CH₃ | OH |
| Ia1.4 | O | H | H | CH₂ | SO₂CH₂CH₃ | OH |
| Ia1.5 | O | H | H | CH₂ | SO₂CH(CH₃)₂ | OH |
| Ia1.6 | O | H | H | CH₂ | SO₂(CH₂)₂CH₃ | OH |
| Ia1.7 | O | H | H | CH₂ | Cl | OH |
| Ia1.8 | O | H | H | CH₂ | Br | OH |
| Ia1.9 | O | H | H | CH₂ | NO₂ | OH |
| Ia1.10 | O | H | H | CH₂ | CHF₂ | OH |
| Ia1.11 | O | H | H | CH₂ | CF₃ | OH |
| Ia1.12 | O | H | H | CH₂ | OCH₃ | OH |
| Ia1.13 | O | H | H | CH₂ | OCH₂CH₃ | OH |
| Ia1.14 | O | H | H | CH₂ | OCHF₂ | OH |
| Ia1.15 | O | H | H | CH₂ | OCF₃ | OH |
| Ia1.16 | O | CH₃ | H | CH₂ | SCH₃ | OH |
| Ia1.17 | O | CH₃ | H | CH₂ | SCH₂CH₃ | OH |
| Ia1.18 | O | CH₃ | H | CH₂ | SO₂CH₃ | OH |
| Ia1.19 | O | CH₃ | H | CH₂ | SO₂CH₂CH₃ | OH |
| Ia1.20 | O | CH₃ | H | CH₂ | SO₂CH(CH₃)₂ | OH |
| Ia1.21 | O | CH₃ | H | CH₂ | SO₂(CH₂)₂CH₃ | OH |
| Ia1.22 | O | CH₃ | H | CH₂ | Cl | OH |
| Ia1.23 | O | CH₃ | H | CH₂ | Br | OH |
| Ia1.24 | O | CH₃ | H | CH₂ | NO₂ | OH |
| Ia1.25 | O | CH₃ | H | CH₂ | CHF₂ | OH |
| Ia1.26 | O | CH₃ | H | CH₂ | CF₃ | OH |
| Ia1.27 | O | CH₃ | H | CH₂ | OCH₃ | OH |
| Ia1.28 | O | CH₃ | H | CH₂ | OCH₂CH₃ | OH |
| Ia1.29 | O | CH₃ | H | CH₂ | OCHF₂ | OH |
| Ia1.30 | O | CH₃ | H | CH₂ | OCF₃ | OH |
| Ia1.31 | O | CH₃ | CH₃ | CH₂ | SCH₃ | OH |
| Ia1.32 | O | CH₃ | CH₃ | CH₂ | SCH₂CH₃ | OH |
| Ia1.33 | O | CH₃ | CH₃ | CH₂ | SO₂CH₃ | OH |
| Ia1.34 | O | CH₃ | CH₃ | CH₂ | SO₂CH₂CH₃ | OH |
| Ia1.35 | O | CH₃ | CH₃ | CH₂ | SO₂CH(CH₃)₂ | OH |
| Ia1.36 | O | CH₃ | CH₃ | CH₂ | SO₂(CH₂)₂CH₃ | OH |
| Ia1.37 | O | CH₃ | CH₃ | CH₂ | Cl | OH |
| Ia1.38 | O | CH₃ | CH₃ | CH₂ | Br | OH |
| Ia1.39 | O | CH₃ | CH₃ | CH₂ | NO₂ | OH |
| Ia1.40 | O | CH₃ | CH₃ | CH₂ | CHF₂ | OH |
| Ia1.41 | O | CH₃ | CH₃ | CH₂ | CF₃ | OH |
| Ia1.42 | O | CH₃ | CH₃ | CH₂ | OCH₃ | OH |
| Ia1.43 | O | CH₃ | CH₃ | CH₂ | OCH₂CH₃ | OH |
| Ia1.44 | O | CH₃ | CH₃ | CH₂ | OCHF₂ | OH |
| Ia1.45 | O | CH₃ | CH₃ | CH₂ | OCF₃ | OH |
| Ia1.46 | O | CH₂Cl | H | CH₂ | SCH₃ | OH |
| Ia1.47 | O | CH₂Cl | H | CH₂ | SCH₂CH₃ | OH |
| Ia1.48 | O | CH₂Cl | H | CH₂ | SO₂CH₃ | OH |
| Ia1.49 | O | CH₂Cl | H | CH₂ | SO₂CH₂CH₃ | OH |
| Ia1.50 | O | CH₂Cl | H | CH₂ | SO₂CH(CH₃)₂ | OH |
| Ia1.51 | O | CH₂Cl | H | CH₂ | SO₂(CH₂)₂CH₃ | OH |
| Ia1.52 | O | CH₂Cl | H | CH₂ | Cl | OH |
| Ia1.53 | O | CH₂Cl | H | CH₂ | Br | OH |
| Ia1.54 | O | CH₂Cl | H | CH₂ | NO₂ | OH |
| Ia1.55 | O | CH₂Cl | H | CH₂ | CHF₂ | OH |
| Ia1.56 | O | CH₂Cl | H | CH₂ | CF₃ | OH |
| Ia1.57 | O | CH₂Cl | H | CH₂ | OCH₃ | OH |
| Ia1.58 | O | CH₂Cl | H | CH₂ | OCH₂CH₃ | OH |
| Ia1.59 | O | CH₂Cl | H | CH₂ | OCHF₂ | OH |
| Ia1.60 | O | CH₂Cl | H | CH₂ | OCF₃ | OH |
| Ia1.61 | O | CH₂CH₃ | H | CH₂ | SCH₃ | OH |
| Ia1.62 | O | CH₂CH₃ | H | CH₂ | SCH₂CH₃ | OH |
| Ia1.63 | O | CH₂CH₃ | H | CH₂ | SO₂CH₃ | OH |
| Ia1.64 | O | CH₂CH₃ | H | CH₂ | SO₂CH₂CH₃ | OH |
| Ia1.65 | O | CH₂CH₃ | H | CH₂ | SO₂CH(CH₃)₂ | OH |
| Ia1.66 | O | CH₂CH₃ | H | CH₂ | SO₂(CH₂)₂CH₃ | OH |
| Ia1.67 | O | CH₂CH₃ | H | CH₂ | Cl | OH |
| Ia1.68 | O | CH₂CH₃ | H | CH₂ | Br | OH |
| Ia1.69 | O | CH₂CH₃ | H | CH₂ | NO₂ | OH |
| Ia1.70 | O | CH₂CH₃ | H | CH₂ | CHF₂ | OH |
| Ia1.71 | O | CH₂CH₃ | H | CH₂ | CF₃ | OH |
| Ia1.72 | O | CH₂CH₃ | H | CH₂ | OCH₃ | OH |
| Ia1.73 | O | CH₂CH₃ | H | CH₂ | OCH₂CH₃ | OH |
| Ia1.74 | O | CH₂CH₃ | H | CH₂ | OCHF₂ | OH |
| Ia1.75 | O | CH₂CH₃ | H | CH₂ | OCF₃ | OH |
| Ia1.76 | O | CH₂CH₃ | CH₂CH₃ | CH₂ | SCH₃ | OH |
| Ia1.77 | O | CH₂CH₃ | CH₂CH₃ | CH₂ | SCH₂CH₃ | OH |
| Ia1.78 | O | CH₂CH₃ | CH₂CH₃ | CH₂ | SO₂CH₃ | OH |
| Ia1.79 | O | CH₂CH₃ | CH₂CH₃ | CH₂ | SO₂CH₂CH₃ | OH |
| Ia1.80 | O | CH₂CH₃ | CH₂CH₃ | CH₂ | SO₂CH(CH₃)₂ | OH |
| Ia1.81 | O | CH₂CH₃ | CH₂CH₃ | CH₂ | SO₂(CH₂)₂CH₃ | OH |
| Ia1.82 | O | CH₂CH₃ | CH₂CH₃ | CH₂ | Cl | OH |
| Ia1.83 | O | CH₂CH₃ | CH₂CH₃ | CH₂ | Br | OH |
| Ia1.84 | O | CH₂CH₃ | CH₂CH₃ | CH₂ | NO₂ | OH |
| Ia1.85 | O | CH₂CH₃ | CH₂CH₃ | CH₂ | CHF₂ | OH |
| Ia1.86 | O | CH₂CH₃ | CH₂CH₃ | CH₂ | CF₃ | OH |
| Ia1.87 | O | CH₂CH₃ | CH₂CH₃ | CH₂ | OCH₃ | OH |
| Ia1.88 | O | CH₂CH₃ | CH₂CH₃ | CH₂ | OCH₂CH₃ | OH |
| Ia1.89 | O | CH₂CH₃ | CH₂CH₃ | CH₂ | OCHF₂ | OH |
| Ia1.90 | O | CH₂CH₃ | CH₂CH₃ | CH₂ | OCF₃ | OH |
| Ia1.91 | O | H | H | CH(CH₃) | SCH₃ | OH |
| Ia1.92 | O | H | H | CH(CH₃) | SCH₂CH₃ | OH |
| Ia1.93 | O | H | H | CH(CH₃) | SO₂CH₃ | OH |
| Ia1.94 | O | H | H | CH(CH₃) | SO₂CH₂CH₃ | OH |
| Ia1.95 | O | H | H | CH(CH₃) | SO₂CH(CH₃)₂ | OH |
| Ia1.96 | O | H | H | CH(CH₃) | SO₂(CH₂)₂CH₃ | OH |
| Ia1.97 | O | H | H | CH(CH₃) | Cl | OH |
| Ia1.98 | O | H | H | CH(CH₃) | Br | OH |
| Ia1.99 | O | H | H | CH(CH₃) | NO₂ | OH |
| Ia1.100 | O | H | H | CH(CH₃) | CHF₂ | OH |
| Ia1.101 | O | H | H | CH(CH₃) | CF₃ | OH |
| Ia1.102 | O | H | H | CH(CH₃) | OCH₃ | OH |
| Ia1.103 | O | H | H | CH(CH₃) | OCH₂CH₃ | OH |
| Ia1.104 | O | H | H | CH(CH₃) | OCHF₂ | OH |
| Ia1.105 | O | H | H | CH(CH₃) | OCF₃ | OH |
| Ia1.106 | O | CH₃ | H | CH(CH₃) | SCH₃ | OH |
| Ia1.107 | O | CH₃ | H | CH (CH₃) | SCH₂CH₃ | OH |
| Ia1.108 | O | CH₃ | H | CH(CH₃) | SO₂CH₃ | OH |
| Ia1.109 | O | CH₃ | H | CH(CH₃) | SO₂CH₂CH₃ | OH |
| Ia1.110 | O | CH₃ | H | CH(CH₃) | SO₂CH(CH₃)₂ | OH |
| Ia1.111 | O | CH₃ | H | CH(CH₃) | SO₂(CH₂)₂CH₃ | OH |
| Ia1.112 | O | CH₃ | H | CH(CH₃) | Cl | OH |
| Ia1.113 | O | CH₃ | H | CH(CH₃) | Br | OH |
| Ia1.114 | O | CH₃ | H | CH(CH₃) | NO₂ | OH |
| Ia1.115 | O | CH₃ | H | CH(CH₃) | CHF₂ | OH |
| Ia1.116 | O | CH₃ | H | CH(CH₃) | CF₃ | OH |
| Ia1.117 | O | CH₃ | H | CH(CH₃) | OCH₃ | OH |
| Ia1.118 | O | CH₃ | H | CH(CH₃) | OCH₂CH₃ | OH |
| Ia1.119 | O | CH₃ | H | CH(CH₃) | OCHF₂ | OH |
| Ia1.120 | O | CH₃ | H | CH(CH₃) | OCF₃ | OH |
| Ia1.121 | O | CH₃ | CH₃ | CH(CH₃) | SCH₃ | OH |
| Ia1.122 | O | CH₃ | CH₃ | CH(CH₃) | SCH₂CH₃ | OH |
| Ia1.123 | O | CH₃ | CH₃ | CH(CH₃) | SO₂CH₃ | OH |
| Ia1.124 | O | CH₃ | CH₃ | CH(CH₃) | SO₂CH₂CH₃ | OH |
| Ia1.125 | O | CH₃ | CH₃ | CH(CH₃) | SO₂CH(CH₃)₂ | OH |
| Ia1.126 | O | CH₃ | CH₃ | CH(CH₃) | SO₂(CH₂)₂CH₃ | OH |
| Ia1.127 | O | CH₃ | CH₃ | CH(CH₃) | Cl | OH |
| Ia1.128 | O | CH₃ | CH₃ | CH(CH₃) | Br | OH |
| Ia1.129 | O | CH₃ | CH₃ | CH(CH₃) | NO₂ | OH |
| Ia1.130 | O | CH₃ | CH₃ | CH(CH₃) | CHF₂ | OH |
| Ia1.131 | O | CH₃ | CH₃ | CH(CH₃) | CF₃ | OH |
| Ia1.132 | O | CH₃ | CH₃ | CH(CH₃) | OCH₃ | OH |
| Ia1.133 | O | CH₃ | CH₃ | CH(CH₃) | OCH₂CH₃ | OH |
| Ia1.134 | O | CH₃ | CH₃ | CH(CH₃) | OCHF₂ | OH |
| Ia1.135 | O | CH₃ | CH₃ | CH(CH₃) | OCF₃ | OH |
| Ia1.136 | O | H | H | C(CH₃)₂ | SCH₃ | OH |
| Ia1.137 | O | H | H | C(CH₃)₂ | SCH₂CH₃ | OH |
| Ia1.138 | O | H | H | C(CH₃)₂ | SO₂CH₃ | OH |
| Ia1.139 | O | H | H | C(CH₃)₂ | SO₂CH₂CH₃ | OH |
| Ia1.140 | O | H | H | C(CH₃)₂ | SO₂CH(CH₃)₂ | OH |
| Ia1.141 | O | H | H | C(CH₃)₂ | SO₂(CH₂)₂CH₃ | OH |
| Ia1.142 | O | H | H | C(CH₃)₂ | Cl | OH |
| Ia1.143 | O | H | H | C(CH₃)₂ | Br | OH |
| Ia1.144 | O | H | H | C(CH₃)₂ | NO₂ | OH |
| Ia1.145 | O | H | H | C(CH₃)₂ | CHF₂ | OH |
| Ia1.146 | O | H | H | C(CH₃)₂ | CF₃ | OH |
| Ia1.147 | O | H | H | C(CH₃)₂ | OCH₃ | OH |
| Ia1.148 | O | H | H | C(CH₃)₂ | OCH₂CH₃ | OH |
| Ia1.149 | O | H | H | C(CH₃)₂ | OCHF₂ | OH |
| Ia1.150 | O | H | H | C(CH₃)₂ | OCF₃ | OH |
| Ia1.151 | O | CH₃ | H | C(CH₃)₂ | SCH₃ | OH |
| Ia1.152 | O | CH₃ | H | C(CH₃)₂ | SCH₂CH₃ | OH |
| Ia1.153 | O | CH₃ | H | C(CH₃)₂ | SO₂CH₃ | OH |
| Ia1.154 | O | CH₃ | H | C(CH₃)₂ | SO₂CH₂CH₃ | OH |
| Ia1.155 | O | CH₃ | H | C(CH₃)₂ | SO₂CH(CH₃)₂ | OH |
| Ia1.156 | O | CH₃ | H | C(CH₃)₂ | SO₂(CH₂)₂CH₃ | OH |
| Ia1.157 | O | CH₃ | H | C(CH₃)₂ | Cl | OH |
| Ia1.158 | O | CH₃ | H | C(CH₃)₂ | Br | OH |
| Ia1.159 | O | CH₃ | H | C(CH₃)₂ | NO₂ | OH |
| Ia1.160 | O | CH₃ | H | C(CH₃)₂ | CHF₂ | OH |
| Ia1.161 | O | CH₃ | H | C(CH₃)₂ | CF₃ | OH |
| Ia1.162 | O | CH₃ | H | C(CH₃)₂ | OCH₃ | OH |
| Ia1.163 | O | CH₃ | H | C(CH₃)₂ | OCH₂CH₃ | OH |
| Ia1.164 | O | CH₃ | H | C(CH₃)₂ | OCHF₂ | OH |
| Ia1.165 | O | CH₃ | H | C(CH₃)₂ | OCF₃ | OH |
| Ia1.166 | O | CH₃ | CH₃ | C(CH₃)₂ | SCH₃ | OH |
| Ia1.167 | O | CH₃ | CH₃ | C(CH₃)₂ | SCH₂CH₃ | OH |
| Ia1.168 | O | CH₃ | CH₃ | C(CH₃)₂ | SO₂CH₃ | OH |
| Ia1.169 | O | CH₃ | CH₃ | C(CH₃)₂ | SO₂CH₂CH₃ | OH |
| Ia1.170 | O | CH₃ | CH₃ | C(CH₃)₂ | SO₂CH(CH₃)₂ | OH |
| Ia1.171 | O | CH₃ | CH₃ | C(CH₃)₂ | SO₂(CH₂)₂CH₃ | OH |
| Ia1.172 | O | CH₃ | CH₃ | C(CH₃)₂ | Cl | OH |
| Ia1.173 | O | CH₃ | CH₃ | C(CH₃)₂ | Br | OH |
| Ia1.174 | O | CH₃ | CH₃ | C(CH₃)₂ | NO₂ | OH |
| Ia1.175 | O | CH₃ | CH₃ | C(CH₃)₂ | CHF₂ | OH |
| Ia1.176 | O | CH₃ | CH₃ | C(CH₃)₂ | CF₃ | OH |
| Ia1.177 | O | CH₃ | CH₃ | C(CH₃)₂ | OCH₃ | OH |
| Ia1.178 | O | CH₃ | CH₃ | C(CH₃)₂ | OCH₂CH₃ | OH |
| Ia1.179 | O | CH₃ | CH₃ | C(CH₃)₂ | OCHF₂ | OH |
| Ia1.180 | O | CH₃ | CH₃ | C(CH₃)₂ | OCF₃ | OH |
| Ia1.181 | NCH₃ | H | H | CH₂ | SCH₃ | OH |
| Ia1.182 | NCH₃ | H | H | CH₂ | SCH₂CH₃ | OH |
| Ia1.183 | NCH₃ | H | H | CH₂ | SO₂CH₃ | OH |
| Ia1.184 | NCH₃ | H | H | CH₂ | SO₂CH₂CH₃ | OH |
| Ia1.185 | NCH₃ | H | H | CH₂ | SO₂CH(CH₃)₂ | OH |
| Ia1.186 | NCH₃ | H | H | CH₂ | SO₂(CH₂)₂CH₃ | OH |
| Ia1.187 | NCH₃ | H | H | CH₂ | Cl | OH |
| Ia1.188 | NCH₃ | H | H | CH₂ | Br | OH |
| Ia1.189 | NCH₃ | H | H | CH₂ | NO₂ | OH |
| Ia1.190 | NCH₃ | H | H | CH₂ | CHF₂ | OH |
| Ia1.191 | NCH₃ | H | H | CH₂ | CF₃ | OH |
| Ia1.192 | NCH₃ | H | H | CH₂ | OCH₃ | OH |
| Ia1.193 | NCH₃ | H | H | CH₂ | OCH₂CH₃ | OH |
| Ia1.194 | NCH₃ | H | H | CH₂ | OCHF₂ | OH |
| Ia1.195 | NCH₃ | H | H | CH₂ | OCF₃ | OH |
| Ia1.196 | NCH₃ | CH₃ | H | CH₂ | SCH₃ | OH |
| Ia1.197 | NCH₃ | CH₃ | H | CH₂ | SCH₂CH₃ | OH |
| Ia1.198 | NCH₃ | CH₃ | H | CH₂ | SO₂CH₃ | OH |
| Ia1.199 | NCH₃ | CH₃ | H | CH₂ | SO₂CH₂CH₃ | OH |
| Ia1.200 | NCH₃ | CH₃ | H | CH₂ | SO₂CH(CH₃)₂ | OH |
| Ia1.201 | NCH₃ | CH₃ | H | CH₂ | SO₂(CH₂)₂CH₃ | OH |
| Ia1.202 | NCH₃ | CH₃ | H | CH₂ | Cl | OH |
| Ia1.203 | NCH₃ | CH₃ | H | CH₂ | Br | OH |
| Ia1.204 | NCH₃ | CH₃ | H | CH₂ | NO₂ | OH |
| Ia1.205 | NCH₃ | CH₃ | H | CH₂ | CHF₂ | OH |
| Ia1.206 | NCH₃ | CH₃ | H | CH₂ | CF₃ | OH |
| Ia1.207 | NCH₃ | CH₃ | H | CH₂ | OCH₃ | OH |
| Ia1.208 | NCH₃ | CH₃ | H | CH₂ | OCH₂CH₃ | OH |
| Ia1.209 | NCH₃ | CH₃ | H | CH₂ | OCHF₂ | OH |
| Ia1.210 | NCH₃ | CH₃ | H | CH₂ | OCF₃ | OH |
| Ia1.211 | NCH₃ | CH₃ | CH₃ | CH₂ | SCH₃ | OH |
| Ia1.212 | NCH₃ | CH₃ | CH₃ | CH₂ | SCH₂CH₃ | OH |
| Ia1.213 | NCH₃ | CH₃ | CH₃ | CH₂ | SO₂CH₃ | OH |
| Ia1.214 | NCH₃ | CH₃ | CH₃ | CH₂ | SO₂CH₂CH₃ | OH |
| Ia1.215 | NCH₃ | CH₃ | CH₃ | CH₂ | SO₂CH(CH₃)₂ | OH |
| Ia1.216 | NCH₃ | CH₃ | CH₃ | CH₂ | SO₂(CH₂)₂CH₃ | OH |
| Ia1.217 | NCH₃ | CH₃ | CH₃ | CH₂ | Cl | OH |
| Ia1.218 | NCH₃ | CH₃ | CH₃ | CH₂ | Br | OH |
| Ia1.219 | NCH₃ | CH₃ | CH₃ | CH₂ | NO₂ | OH |
| Ia1.220 | NCH₃ | CH₃ | CH₃ | CH₂ | CHF₂ | OH |
| Ia1.221 | NCH₃ | CH₃ | CH₃ | CH₂ | CF₃ | OH |
| Ia1.222 | NCH₃ | CH₃ | CH₃ | CH₂ | OCH₃ | OH |
| Ia1.223 | NCH₃ | CH₃ | CH₃ | CH₂ | OCH₂CH₃ | OH |
| Ia1.224 | NCH₃ | CH₃ | CH₃ | CH₂ | OCHF₂ | OH |
| Ia1.225 | NCH₃ | CH₃ | CH₃ | CH₂ | OCF₃ | OH |
| Ia1.226 | NCH₃ | CH₂Cl | H | CH₂ | SCH₃ | OH |
| Ia1.227 | NCH₃ | CH₂Cl | H | CH₂ | SCH₂CH₃ | OH |
| Ia1.228 | NCH₃ | CH₂Cl | H | CH₂ | SO₂CH₃ | OH |
| Ia1.229 | NCH₃ | CH₂Cl | H | CH₂ | SO₂CH₂CH₃ | OH |
| Ia1.230 | NCH₃ | CH₂Cl | H | CH₂ | SO₂CH(CH₃)₂ | OH |
| Ia1.231 | NCH₃ | CH₂Cl | H | CH₂ | SO₂(CH₂)₂CH₃ | OH |
| Ia1.232 | NCH₃ | CH₂Cl | H | CH₂ | Cl | OH |
| Ia1.233 | NCH₃ | CH₂Cl | H | CH₂ | Br | OH |
| Ia1.234 | NCH₃ | CH₂Cl | H | CH₂ | NO₂ | OH |
| Ia1.235 | NCH₃ | CH₂Cl | H | CH₂ | CHF₂ | OH |
| Ia1.236 | NCH₃ | CH₂Cl | H | CH₂ | CF₃ | OH |
| Ia1.237 | NCH₃ | CH₂Cl | H | CH₂ | OCH₃ | OH |
| Ia1.238 | NCH₃ | CH₂Cl | H | CH₂ | OCH₂CH₃ | OH |
| Ia1.239 | NCH₃ | CH₂Cl | H | CH₂ | OCHF₂ | OH |
| Ia1.240 | NCH₃ | CH₂Cl | H | CH₂ | OCF₃ | OH |
| Ia1.241 | NCH₃ | CH₂CH₃ | H | CH₂ | SCH₃ | OH |
| Ia1.242 | NCH₃ | CH₂CH₃ | H | CH₂ | SCH₂CH₃ | OH |
| Ia1.243 | NCH₃ | CH₂CH₃ | H | CH₂ | SO₂CH₃ | OH |
| Ia1.244 | NCH₃ | CH₂CH₃ | H | CH₂ | SO₂CH₂CH₃ | OH |
| Ia1.245 | NCH₃ | CH₂CH₃ | H | CH₂ | SO₂CH (CH₃)₂ | OH |
| Ia1.246 | NCH₃ | CH₂CH₃ | H | CH₂ | SO₂(CH₂)₂CH₃ | OH |
| Ia1.247 | NCH₃ | CH₂CH₃ | H | CH₂ | Cl | OH |
| Ia1.248 | NCH₃ | CH₂CH₃ | H | CH₂ | Br | OH |
| Ia1.249 | NCH₃ | CH₂CH₃ | H | CH₂ | NO₂ | OH |
| Ia1.250 | NCH₃ | CH₂CH₃ | H | CH₂ | CHF₂ | OH |
| Ia1.251 | NCH₃ | CH₂CH₃ | H | CH₂ | CF₃ | OH |
| Ia1.252 | NCH₃ | CH₂CH₃ | H | CH₂ | OCH₃ | OH |
| Ia1.253 | NCH₃ | CH₂CH₃ | H | CH₂ | OCH₂CH₃ | OH |
| Ia1.254 | NCH₃ | CH₂CH₃ | H | CH₂ | OCHF₂ | OH |
| Ia1.255 | NCH₃ | CH₂CH₃ | H | CH₂ | OCF₃ | OH |
| Ia1.256 | NCH₃ | CH₂CH₃ | CH₂CH₃ | CH₂ | SCH₃ | OH |
| Ia1.257 | NCH₃ | CH₂CH₃ | CH₂CH₃ | CH₂ | SCH₂CH₃ | OH |
| Ia1.258 | NCH₃ | CH₂CH₃ | CH₂CH₃ | CH₂ | SO₂CH₃ | OH |
| Ia1.259 | NCH₃ | CH₂CH₃ | CH₂CH₃ | CH₂ | SO₂CH₂CH₃ | OH |
| Ia1.260 | NCH₃ | CH₂CH₃ | CH₂CH₃ | CH₂ | SO₂CH(CH₃)₂ | OH |
| Ia1.261 | NCH₃ | CH₂CH₃ | CH₂CH₃ | CH₂ | SO₂(CH₂)₂CH₃ | OH |
| Ia1.262 | NCH₃ | CH₂CH₃ | CH₂CH₃ | CH₂ | Cl | OH |
| Ia1.263 | NCH₃ | CH₂CH₃ | CH₂CH₃ | CH₂ | Br | OH |
| Ia1.264 | NCH₃ | CH₂CH₃ | CH₂CH₃ | CH₂ | NO₂ | OH |
| Ia1.265 | NCH₃ | CH₂CH₃ | CH₂CH₃ | CH₂ | CHF₂ | OH |
| Ia1.266 | NCH₃ | CH₂CH₃ | CH₂CH₃ | CH₂ | CF₃ | OH |
| Ia1.267 | NCH₃ | CH₂CH₃ | CH₂CH₃ | CH₂ | OCH₃ | OH |
| Ia1.268 | NCH₃ | CH₂CH₃ | CH₂CH₃ | CH₂ | OCH₂CH₃ | OH |
| Ia1.269 | NCH₃ | CH₂CH₃ | CH₂CH₃ | CH₂ | OCHF₂ | OH |
| Ia1.270 | NCH₃ | CH₂CH₃ | CH₂CH₃ | CH₂ | OCF₃ | OH |
| Ia1.271 | NCH₃ | H | H | CH(CH₃) | SCH₃ | OH |
| Ia1.272 | NCH₃ | H | H | CH(CH₃) | SCH₂CH₃ | OH |
| Ia1.273 | NCH₃ | H | H | CH(CH₃) | SO₂CH₃ | OH |
| Ia1.274 | NCH₃ | H | H | CH(CH₃) | SO₂CH₂CH₃ | OH |
| Ia1.275 | NCH₃ | H | H | CH(CH₃) | SO₂CH(CH₃)₂ | OH |
| Ia1.276 | NCH₃ | H | H | CH(CH₃) | SO₂(CH₂)₂CH₃ | OH |
| Ia1.277 | NCH₃ | H | H | CH(CH₃) | Cl | OH |
| Ia1.278 | NCH₃ | H | H | CH(CH₃) | Br | OH |
| Ia1.279 | NCH₃ | H | H | CH(CH₃) | NO₂ | OH |
| Ia1.280 | NCH₃ | H | H | CH(CH₃) | CHF₂ | OH |
| Ial.281 | NCH₃ | H | H | CH(CH₃) | CF₃ | OH |
| Ia1.282 | NCH₃ | H | H | CH(CH₃) | OCH₃ | OH |
| Ia1.283 | NCH₃ | H | H | CH(CH₃) | OCH₂CH₃ | OH |
| Ia1.284 | NCH₃ | H | H | CH(CH₃) | OCHF₂ | OH |
| Ia1.285 | NCH₃ | H | H | CH(CH₃) | OCF₃ | OH |
| Ia1.286 | NCH₃ | CH₃ | H | CH(CH₃) | SCH₃ | OH |
| Ia1.287 | NCH₃ | CH₃ | H | CH(CH₃) | SCH₂CH₃ | OH |
| Ia1.288 | NCH₃ | CH₃ | H | CH(CH₃) | SO₂CH₃ | OH |
| Ia1.289 | NCH₃ | CH₃ | H | CH(CH₃) | SO₂CH₂CH₃ | OH |
| Ia1.290 | NCH₃ | CH₃ | H | CH(CH₃) | SO₂CH(CH₃)₂ | OH |
| Ia1.291 | NCH₃ | CH₃ | H | CH(CH₃) | SO₂(CH₂)₂CH₃ | OH |
| Ia1.292 | NCH₃ | CH₃ | H | CH(CH₃) | Cl | OH |
| Ia1.293 | NCH₃ | CH₃ | H | CH(CH₃) | Br | OH |
| Ia1.294 | NCH₃ | CH₃ | H | CH(CH₃) | NO₂ | OH |
| Ia1.295 | NCH₃ | CH₃ | H | CH(CH₃) | CHF₂ | OH |
| Ia1.296 | NCH₃ | CH₃ | H | CH(CH₃) | CF₃ | OH |
| Ia1.297 | NCH₃ | CH₃ | H | CH(CH₃) | OCH₃ | OH |
| Ia1.298 | NCH₃ | CH₃ | H | CH(CH₃) | OCH₂CH₃ | OH |
| Ia1.299 | NCH₃ | CH₃ | H | CH(CH₃) | OCHF₂ | OH |
| Ia1.300 | NCH₃ | CH₃ | H | CH(CH₃) | OCF₃ | OH |
| Ia1.301 | NCH₃ | CH₃ | CH₃ | CH(CH₃) | SCH₃ | OH |
| Ia1.302 | NCH₃ | CH₃ | CH₃ | CH(CH₃) | SCH₂CH₃ | OH |
| Ia1.303 | NCH₃ | CH₃ | CH₃ | CH(CH₃) | SO₂CH₃ | OH |
| Ia1.304 | NCH₃ | CH₃ | CH₃ | CH(CH₃) | SO₂CH₂CH₃ | OH |
| Ia1.305 | NCH₃ | CH₃ | CH₃ | CH(CH₃) | SO₂CH(CH₃)₂ | OH |
| Ia1.306 | NCH₃ | CH₃ | CH₃ | CH(CH₃) | SO₂(CH₂)₂CH₃ | OH |
| Ia1.307 | NCH₃ | CH₃ | CH₃ | CH(CH₃) | Cl | OH |
| Ia1.308 | NCH₃ | CH₃ | CH₃ | CH(CH₃) | Br | OH |
| Ia1.309 | NCH₃ | CH₃ | CH₃ | CH(CH₃) | NO₂ | OH |
| Ia1.310 | NCH₃ | CH₃ | CH₃ | CH(CH₃) | CHF₂ | OH |
| Ia1.311 | NCH₃ | CH₃ | CH₃ | CH(CH₃) | CF₃ | OH |
| Ia1.312 | NCH₃ | CH₃ | CH₃ | CH(CH₃) | OCH₃ | OH |
| Ia1.313 | NCH₃ | CH₃ | CH₃ | CH(CH₃) | OCH₂CH₃ | OH |
| Ia1.314 | NCH₃ | CH₃ | CH₃ | CH(CH₃) | OCHF₂ | OH |
| Ia1.315 | NCH₃ | CH₃ | CH₃ | CH(CH₃) | OCF₃ | OH |
| Ia1.316 | NCH₃ | H | H | C(CH₃)₂ | SCH₃ | OH |
| Ia1.317 | NCH₃ | H | H | C(CH₃)₂ | SCH₂CH₃ | OH |
| Ia1.318 | NCH₃ | H | H | C(CH₃)₂ | SO₂CH₃ | OH |
| Ia1.319 | NCH₃ | H | H | C(CH₃)₂ | SO₂CH₂CH₃ | OH |
| Ia1.320 | NCH₃ | H | H | C(CH₃)₂ | SO₂CH(CH₃)₂ | OH |
| Ia1.321 | NCH₃ | H | H | C(CH₃)₂ | SO₂(CH₂)₂CH₃ | OH |
| Ia1.322 | NCH₃ | H | H | C(CH₃)₂ | Cl | OH |
| Ia1.323 | NCH₃ | H | H | C(CH₃)₂ | Br | OH |
| Ia1.324 | NCH₃ | H | H | C(CH₃)₂ | NO₂ | OH |
| Ia1.325 | NCH₃ | H | H | C(CH₃)₂ | CHF₂ | OH |
| Ia1.326 | NCH₃ | H | H | C(CH₃)₂ | CF₃ | OH |
| Ia1.327 | NCH₃ | H | H | C(CH₃)₂ | OCH₃ | OH |
| Ia1.328 | NCH₃ | H | H | C(CH₃)₂ | OCH₂CH₃ | OH |
| Ia1.329 | NCH₃ | H | H | C(CH₃)₂ | OCHF₂ | OH |
| Ia1.330 | NCH₃ | H | H | C(CH₃)₂ | OCF₃ | OH |
| Ia1.331 | NCH₃ | CH₃ | H | C(CH₃)₂ | SCH₃ | OH |
| Ia1.332 | NCH₃ | CH₃ | H | C(CH₃)₂ | SCH₂CH₃ | OH |
| Ia1.333 | NCH₃ | CH₃ | H | C(CH₃)₂ | SO₂CH₃ | OH |
| Ia1.334 | NCH₃ | CH₃ | H | C(CH₃)₂ | SO₂CH₂CH₃ | OH |
| Ia1.335 | NCH₃ | CH₃ | H | C(CH₃)₂ | SO₂CH(CH₃)₂ | OH |
| Ia1.336 | NCH₃ | CH₃ | H | C(CH₃)₂ | SO₂(CH₂)₂CH₃ | OH |
| Ia1.337 | NCH₃ | CH₃ | H | C(CH₃)₂ | Cl | OH |
| Ia1.338 | NCH₃ | CH₃ | H | C(CH₃)₂ | Br | OH |
| Ia1.339 | NCH₃ | CH₃ | H | C(CH₃)₂ | NO₂ | OH |
| Ia1.340 | NCH₃ | CH₃ | H | C(CH₃)₂ | CHF₂ | OH |
| Ia1.341 | NCH₃ | CH₃ | H | C(CH₃)₂ | CF₃ | OH |
| Ia1.342 | NCH₃ | CH₃ | H | C(CH₃)₂ | OCH₃ | OH |
| Ia1.343 | NCH₃ | CH₃ | H | C(CH₃)₂ | OCH₂CH₃ | OH |
| Ia1.344 | NCH₃ | CH₃ | H | C(CH₃)₂ | OCHF₂ | OH |
| Ia1. 345 | NCH₃ | CH₃ | H | C(CH₃)₂ | OCF₃ | OH |
| Ia1.346 | NCH₃ | CH₃ | CH₃ | C(CH₃)₂ | SCH₃ | OH |
| Ia1.347 | NCH₃ | CH₃ | CH₃ | C(CH₃)₂ | SCH₂CH₃ | OH |
| Ia1.348 | NCH₃ | CH₃ | CH₃ | C(CH₃)₂ | SO₂CH₃ | OH |
| Ia1.349 | NCH₃ | CH₃ | CH₃ | C(CH₃)₂ | SO₂CH₂CH₃ | OH |
| Ia1.350 | NCH₃ | CH₃ | CH₃ | C(CH₃)₂ | SO₂CH(CH₃)₂ | OH |
| Ia1.351 | NCH₃ | CH₃ | CH₃ | C(CH₃)₂ | SO₂(CH₂)₂CH₃ | OH |
| Ia1.352 | NCH₃ | CH₃ | CH₃ | C(CH₃)₂ | Cl | OH |
| Ia1.353 | NCH₃ | CH₃ | CH₃ | C(CH₃)₂ | Br | OH |
| Ia1.354 | NCH₃ | CH₃ | CH₃ | C(CH₃)₂ | NO₂ | OH |
| Ia1.355 | NCH₃ | CH₃ | CH₃ | C(CH₃)₂ | CHF₂ | OH |
| Ia1.356 | NCH₃ | CH₃ | CH₃ | C(CH₃)₂ | CF₃ | OH |
| Ia1.357 | NCH₃ | CH₃ | CH₃ | C(CH₃)₂ | OCH₃ | OH |
| Ia1.358 | NCH₃ | CH₃ | CH₃ | C(CH₃)₂ | OCH₂CH₃ | OH |
| Ia1.359 | NCH₃ | CH₃ | CH₃ | C(CH₃)₂ | OCHF₂ | OH |
| Ia1.360 | NCH₃ | CH₃ | CH₃ | C(CH₃)₂ | OCF₃ | OH |
| Ia1.361 | CH₂ | H | H | O | SCH₃ | OH |
| Ia1.362 | CH₂ | H | H | O | SCH₂CH₃ | OH |
| Ia1.363 | CH₂ | H | H | O | SO₂CH₃ | OH |
| Ia1.364 | CH₂ | H | H | O | SO₂CH₂CH₃ | OH |
| Ia1.365 | CH₂ | H | H | O | SO₂CH(CH₃)₂ | OH |
| Ia1.366 | CH₂ | H | H | O | SO₂(CH₂)₂CH₃ | OH |
| Ia1.367 | CH₂ | H | H | O | Cl | OH |
| Ia1.368 | CH₂ | H | H | O | Br | OH |
| Ia1.369 | CH₂ | H | H | O | NO₂ | OH |
| Ia1.370 | CH₂ | H | H | O | CHF₂ | OH |
| Ia1.371 | CH₂ | H | H | O | CF₃ | OH |
| Ia1.372 | CH₂ | H | H | O | OCH₃ | OH |
| Ia1.373 | CH₂ | H | H | O | OCH₂CH₃ | OH |
| Ia1.374 | CH₂ | H | H | O | OCHF₂ | OH |
| Ia1.375 | CH₂ | H | H | O | OCF₃ | OH |
| Ia1.376 | CH₂ | CH₃ | H | O | SCH₃ | OH |
| Ia1.377 | CH₂ | CH₃ | H | O | SCH₂CH₃ | OH |
| Ia1.378 | CH₂ | CH₃ | H | O | SO₂CH₃ | OH |
| Ia1.379 | CH₂ | CH₃ | H | O | SO₂CH₂CH₃ | OH |
| Ia1.380 | CH₂ | CH₃ | H | O | SO₂CH(CH₃)₂ | OH |
| Ia1.381 | CH₂ | CH₃ | H | O | SO₂(CH₂)₂CH₃ | OH |
| Ia1.382 | CH₂ | CH₃ | H | O | Cl | OH |
| Ia1.383 | CH₂ | CH₃ | H | O | Br | OH |
| Ia1.384 | CH₂ | CH₃ | H | O | NO₂ | OH |
| Ia1.385 | CH₂ | CH₃ | H | O | CHF₂ | OH |
| Ia1.386 | CH₂ | CH₃ | H | O | CF₃ | OH |
| Ia1.387 | CH₂ | CH₃ | H | O | OCH₃ | OH |
| Ia1.388 | CH₂ | CH₃ | H | O | OCH₂CH₃ | OH |
| Ia1.389 | CH₂ | CH₃ | H | O | OCHF₂ | OH |
| Ia1.390 | CH₂ | CH₃ | H | O | OCF₃ | OH |
| Ia1.391 | CH₂ | CH₃ | CH₃ | O | SCH₃ | OH |
| Ia1.392 | CH₂ | CH₃ | CH₃ | O | SCH₂CH₃ | OH |
| Ia1.393 | CH₂ | CH₃ | CH₃ | O | SO₂CH₃ | OH |
| Ia1.394 | CH₂ | CH₃ | CH₃ | O | SO₂CH₂CH₃ | OH |
| Ia1.395 | CH₂ | CH₃ | CH₃ | O | SO₂CH(CH₃)₂ | OH |
| Ia1.396 | CH₂ | CH₃ | CH₃ | O | SO₂(CH₂)₂CH₃ | OH |
| Ia1.397 | CH₂ | CH₃ | CH₃ | O | Cl | OH |
| Ia1.398 | CH₂ | CH₃ | CH₃ | O | Br | OH |
| Ia1.399 | CH₂ | CH₃ | CH₃ | O | NO₂ | OH |
| Ia1.400 | CH₂ | CH₃ | CH₃ | O | CHF₂ | OH |
| Ia1.401 | CH₂ | CH₃ | CH₃ | O | CF₃ | OH |
| Ia1.402 | CH₂ | CH₃ | CH₃ | O | OCH₃ | OH |
| Ia1.403 | CH₂ | CH₃ | CH₃ | O | OCH₂CH₃ | OH |
| Ia1.404 | CH₂ | CH₃ | CH₃ | O | OCHF₂ | OH |
| Ia1.405 | CH₂ | CH₃ | CH₃ | O | OCF₃ | OH |
| Ia1.406 | CH₂ | H | H | S | SCH₃ | OH |
| Ia1.407 | CH₂ | H | H | S | SCH₂CH₃ | OH |
| Ia1.408 | CH₂ | H | H | S | SO₂CH₃ | OH |
| Ia1.409 | CH₂ | H | H | S | SO₂CH₂CH₃ | OH |
| Ia1.410 | CH₂ | H | H | S | SO₂CH(CH₃)₂ | OH |
| Ia1.411 | CH₂ | H | H | S | SO₂(CH₂)₂CH₃ | OH |
| Ia1.412 | CH₂ | H | H | S | Cl | OH |
| Ia1.413 | CH₂ | H | H | S | Br | OH |
| Ia1.414 | CH₂ | H | H | S | NO₂ | OH |
| Ia1.415 | CH₂ | H | H | S | CHF₂ | OH |
| Ia1.416 | CH₂ | H | H | S | CF₃ | OH |
| Ia1.417 | CH₂ | H | H | S | OCH₃ | OH |
| Ia1.418 | CH₂ | H | H | S | OCH₂CH₃ | OH |
| Ia1.419 | CH₂ | H | H | S | OCHF₂ | OH |
| Ia1.420 | CH₂ | H | H | S | OCF₃ | OH |
| Ia1.421 | CH₂ | CH₃ | H | S | SCH₃ | OH |
| Ia1.422 | CH₂ | CH₃ | H | S | SCH₂CH₃ | OH |
| Ia1.423 | CH₂ | CH₃ | H | S | SO₂CH₃ | OH |
| Ia1.424 | CH₂ | CH₃ | H | S | SO₂CH₂CH₃ | OH |
| Ia1.425 | CH₂ | CH₃ | H | S | SO₂CH(CH₃)₂ | OH |
| Ia1.426 | CH₂ | CH₃ | H | S | SO₂(CH₂)₂CH₃ | OH |
| Ia1.427 | CH₂ | CH₃ | H | S | Cl | OH |
| Ia1.428 | CH₂ | CH₃ | H | S | Br | OH |
| Ia1.429 | CH₂ | CH₃ | H | S | NO₂ | OH |
| Ia1.430 | CH₂ | CH₃ | H | S | CHF₂ | OH |
| Ia1.431 | CH₂ | CH₃ | H | S | CF₃ | OH |
| Ia1.432 | CH₂ | CH₃ | H | S | OCH₃ | OH |
| Ia1.433 | CH₂ | CH₃ | H | S | OCH₂CH₃ | OH |
| Ia1.434 | CH₂ | CH₃ | H | S | OCHF₂ | OH |
| Ia1.435 | CH₂ | CH₃ | H | S | OCF₃ | OH |
| Ia1.436 | CH₂ | CH₃ | CH₃ | S | SCH₃ | OH |
| Ia1.437 | CH₂ | CH₃ | CH₃ | S | SCH₂CH₃ | OH |
| Ia1.438 | CH₂ | CH₃ | CH₃ | S | SO₂CH₃ | OH |
| Ia1.439 | CH₂ | CH₃ | CH₃ | S | SO₂CH₂CH₃ | OH |
| Ia1.440 | CH₂ | CH₃ | CH₃ | S | SO₂CH(CH₃)₂ | OH |
| Ia1.441 | CH₂ | CH₃ | CH₃ | S | SO₂(CH₂)₂CH₃ | OH |
| Ia1.442 | CH₂ | CH₃ | CH₃ | S | Cl | OH |
| Ia1.443 | CH₂ | CH₃ | CH₃ | S | Br | OH |
| Ia1.444 | CH₂ | CH₃ | CH₃ | S | NO₂ | OH |
| Ia1.445 | CH₂ | CH₃ | CH₃ | S | CHF₂ | OH |
| Ia1.446 | CH₂ | CH₃ | CH₃ | S | CF₃ | OH |
| Ia1.447 | CH₂ | CH₃ | CH₃ | S | OCH₃ | OH |
| Ia1.448 | CH₂ | CH₃ | CH₃ | S | OCH₂CH₃ | OH |
| Ia1.449 | CH₂ | CH₃ | CH₃ | S | OCHF₂ | OH |
| Ia1.450 | CH₂ | CH₃ | CH₃ | S | OCF₃ | OH |
| Ia1.451 | CH₂ | H | H | NCH₃ | SCH₃ | OH |
| Ia1.452 | CH₂ | H | H | NCH₃ | SCH₂CH₃ | OH |
| Ia1.453 | CH₂ | H | H | NCH₃ | SO₂CH₃ | OH |
| Ia1.454 | CH₂ | H | H | NCH₃ | SO₂CH₂CH₃ | OH |
| Ia1.455 | CH₂ | H | H | NCH₃ | SO₂CH(CH₃)₂ | OH |
| Ia1.456 | CH₂ | H | H | NCH₃ | SO₂(CH₂)₂CH₃ | OH |
| Ia1.457 | CH₂ | H | H | NCH₃ | Cl | OH |
| Ia1.458 | CH₂ | H | H | NCH₃ | Br | OH |
| Ia1.459 | CH₂ | H | H | NCH₃ | NO₂ | OH |
| Ia1.460 | CH₂ | H | H | NCH₃ | CHF₂ | OH |
| Ia1.461 | CH₂ | H | H | NCH₃ | CF₃ | OH |
| Ia1.462 | CH₂ | H | H | NCH₃ | OCH₃ | OH |
| Ia1.463 | CH₂ | H | H | NCH₃ | OCH₂CH₃ | OH |
| Ia1.464 | CH₂ | H | H | NCH₃ | OCHF₂ | OH |
| Ia1.465 | CH₂ | H | H | NCH₃ | OCF₃ | OH |
| Ia1.466 | CH₂ | CH₃ | H | NCH₃ | SCH₃ | OH |
| Ia1.467 | CH₂ | CH₃ | H | NCH₃ | SCH₂CH₃ | OH |
| Ia1.468 | CH₂ | CH₃ | H | NCH₃ | SO₂CH₃ | OH |
| Ia1.469 | CH₂ | CH₃ | H | NCH₃ | SO₂CH₂CH₃ | OH |
| Ia1.470 | CH₂ | CH₃ | H | NCH₃ | SO₂CH(CH₃)₂ | OH |
| Ia1.471 | CH₂ | CH₃ | H | NCH₃ | SO₂(CH₂)₂CH₃ | OH |
| Ia1.472 | CH₂ | CH₃ | H | NCH₃ | Cl | OH |
| Ia1.473 | CH₂ | CH₃ | H | NCH₃ | Br | OH |
| Ia1.474 | CH₂ | CH₃ | H | NCH₃ | NO₂ | OH |
| Ia1.475 | CH₂ | CH₃ | H | NCH₃ | CHF₂ | OH |
| Ia1.476 | CH₂ | CH₃ | H | NCH₃ | CF₃ | OH |
| Ia1.477 | CH₂ | CH₃ | H | NCH₃ | OCH₃ | OH |
| Ia1.478 | CH₂ | CH₃ | H | NCH₃ | OCH₂CH₃ | OH |
| Ia1.479 | CH₂ | CH₃ | H | NCH₃ | OCHF₂ | OH |
| Ia1.480 | CH₂ | CH₃ | H | NCH₃ | OCF₃ | OH |
| Ia1.481 | CH₂ | CH₃ | CH₃ | NCH₃ | SCH₃ | OH |
| Ia1.482 | CH₂ | CH₃ | CH₃ | NCH₃ | SCH₂CH₃ | OH |
| Ia1.483 | CH₂ | CH₃ | CH₃ | NCH₃ | SO₂CH₃ | OH |
| Ia1.484 | CH₂ | CH₃ | CH₃ | NCH₃ | SO₂CH₂CH₃ | OH |
| Ia1.485 | CH₂ | CH₃ | CH₃ | NCH₃ | SO₂CH(CH₃)₂ | OH |
| Ia1.486 | CH₂ | CH₃ | CH₃ | NCH₃ | SO₂(CH₂)₂CH₃ | OH |
| Ia1.487 | CH₂ | CH₃ | CH₃ | NCH₃ | Cl | OH |
| Ia1.488 | CH₂ | CH₃ | CH₃ | NCH₃ | Br | OH |
| Ia1.489 | CH₂ | CH₃ | CH₃ | NCH₃ | NO₂ | OH |
| Ia1.490 | CH₂ | CH₃ | CH₃ | NCH₃ | CHF₂ | OH |
| Ia1.491 | CH₂ | CH₃ | CH₃ | NCH₃ | CF₃ | OH |
| Ia1.492 | CH₂ | CH₃ | CH₃ | NCH₃ | OCH₃ | OH |
| Ia1.493 | CH₂ | CH₃ | CH₃ | NCH₃ | OCH₂CH₃ | OH |
| Ia1.494 | CH₂ | CH₃ | CH₃ | NCH₃ | OCHF₂ | OH |
| Ia1.495 | CH₂ | CH₃ | CH₃ | NCH₃ | OCF₃ | OH |
| Ia1.496 | CHCH₃ | H | H | O | SCH₃ | OH |
| Ia1.497 | CHCH₃ | H | H | O | SCH₂CH₃ | OH |
| Ia1.498 | CHCH₃ | H | H | O | SO₂CH₃ | OH |
| Ia1.499 | CHCH₃ | H | H | O | SO₂CH₂CH₃ | OH |
| Ia1.500 | CHCH₃ | H | H | O | SO₂CH(CH₃)₂ | OH |
| Ia1.501 | CHCH₃ | H | H | O | SO₂(CH₂)₂CH₃ | OH |
| Ia1.502 | CHCH₃ | H | H | O | Cl | OH |
| Ia1.503 | CHCH₃ | H | H | O | Br | OH |
| Ia1.504 | CHCH₃ | H | H | O | NO₂ | OH |
| Ia1.505 | CHCH₃ | H | H | O | CHF₂ | OH |
| Ia1.506 | CHCH₃ | H | H | O | CF₃ | OH |
| Ia1.507 | CHCH₃ | H | H | O | OCH₃ | OH |
| Ia1.508 | CHCH₃ | H | H | O | OCH₂CH₃ | OH |
| Ia1.509 | CHCH₃ | H | H | O | OCHF₂ | OH |
| Ia1.510 | CHCH₃ | H | H | O | OCF₃ | OH |
| Ia1.511 | CHCH₃ | CH₃ | H | O | SCH₃ | OH |
| Ia1.512 | CHCH₃ | CH₃ | H | O | SCH₂CH₃ | OH |
| Ia1.513 | CHCH₃ | CH₃ | H | O | SO₂CH₃ | OH |
| Ia1.514 | CHCH₃ | CH₃ | H | O | SO₂CH₂CH₃ | OH |
| Ia1.515 | CHCH₃ | CH₃ | H | O | SO₂CH(CH₃)₂ | OH |
| Ia1.516 | CHCH₃ | CH₃ | H | O | SO₂(CH₂)₂CH₃ | OH |
| Ia1.517 | CHCH₃ | CH₃ | H | O | Cl | OH |
| Ia1.518 | CHCH₃ | CH₃ | H | O | Br | OH |
| Ia1.519 | CHCH₃ | CH₃ | H | O | NO₂ | OH |
| Ia1.520 | CHCH₃ | CH₃ | H | O | CHF₂ | OH |
| Ia1.521 | CHCH₃ | CH₃ | H | O | CF₃ | OH |
| Ia1.522 | CHCH₃ | CH₃ | H | O | OCH₃ | OH |
| Ia1.523 | CHCH₃ | CH₃ | H | O | OCH₂CH₃ | OH |
| Ia1.524 | CHCH₃ | CH₃ | H | O | OCHF₂ | OH |
| Ia1.525 | CHCH₃ | CH₃ | H | O | OCF₃ | OH |
| Ia1.526 | CHCH₃ | CH₃ | CH₃ | O | SCH₃ | OH |
| Ia1.527 | CHCH₃ | CH₃ | CH₃ | O | SCH₂CH₃ | OH |
| Ia1.528 | CHCH₃ | CH₃ | CH₃ | O | SO₂CH₃ | OH |
| Ia1.529 | CHCH₃ | CH₃ | CH₃ | O | SO₂CH₂CH₃ | OH |
| Ia1.530 | CHCH₃ | CH₃ | CH₃ | O | SO₂CH(CH₃)₂ | OH |
| Ia1.531 | CHCH₃ | CH₃ | CH₃ | O | SO₂(CH₂)₂CH₃ | OH |
| Ia1.532 | CHCH₃ | CH₃ | CH₃ | O | Cl | OH |
| Ia1.533 | CHCH₃ | CH₃ | CH₃ | O | Br | OH |
| Ia1.534 | CHCH₃ | CH₃ | CH₃ | O | NO₂ | OH |
| Ia1.535 | CHCH₃ | CH₃ | CH₃ | O | CHF₂ | OH |
| Ia1.536 | CHCH₃ | CH₃ | CH₃ | O | CF₃ | OH |
| Ia1.537 | CHCH₃ | CH₃ | CH₃ | O | OCH₃ | OH |
| Ia1.538 | CHCH₃ | CH₃ | CH₃ | O | OCH₂CH₃ | OH |
| Ia1.539 | CHCH₃ | CH₃ | CH₃ | O | OCHF₂ | OH |
| Ia1.540 | CHCH₃ | CH₃ | CH₃ | O | OCF₃ | OH |
| Ia1.541 | CHCH₃ | H | H | S | SCH₃ | OH |
| Ia1.542 | CHCH₃ | H | H | S | SCH₂CH₃ | OH |
| Ia1.543 | CHCH₃ | H | H | S | SO₂CH₃ | OH |
| Ia1.544 | CHCH₃ | H | H | S | SO₂CH₂CH₃ | OH |
| Ia1.545 | CHCH₃ | H | H | S | SO₂CH(CH₃)₂ | OH |
| Ia1.546 | CHCH₃ | H | H | S | SO₂(CH₂)₂CH₃ | OH |
| Ia1.547 | CHCH₃ | H | H | S | Cl | OH |
| Ia1.548 | CHCH₃ | H | H | S | Br | OH |
| Ia1.549 | CHCH₃ | H | H | S | NO₂ | OH |
| Ia1.550 | CHCH₃ | H | H | S | CHF₂ | OH |
| Ia1.551 | CHCH₃ | H | H | S | CF₃ | OH |
| Ia1.552 | CHCH₃ | H | H | S | OCH₃ | OH |
| Ia1.553 | CHCH₃ | H | H | S | OCH₂CH₃ | OH |
| Ia1.554 | CHCH₃ | H | H | S | OCHF₂ | OH |
| Ia1.555 | CHCH₃ | H | H | S | OCF₃ | OH |
| Ia1.556 | CHCH₃ | CH₃ | H | S | SCH₃ | OH |
| Ia1.557 | CHCH₃ | CH₃ | H | S | SCH₂CH₃ | OH |
| Ia1.558 | CHCH₃ | CH₃ | H | S | SO₂CH₃ | OH |
| Ia1.559 | CHCH₃ | CH₃ | H | S | SO₂CH₂CH₃ | OH |
| Ia1.560 | CHCH₃ | CH₃ | H | S | SO₂CH(CH₃)₂ | OH |
| Ia1.561 | CHCH₃ | CH₃ | H | S | SO₂(CH₂)₂CH₃ | OH |
| Ia1.562 | CHCH₃ | CH₃ | H | S | Cl | OH |
| Ia1.563 | CHCH₃ | CH₃ | H | S | Br | OH |
| Ia1.564 | CHCH₃ | CH₃ | H | S | NO₂ | OH |
| Ia1.565 | CHCH₃ | CH₃ | H | S | CHF₂ | OH |
| Ia1.566 | CHCH₃ | CH₃ | H | S | CF₃ | OH |
| Ia1.567 | CHCH₃ | CH₃ | H | S | OCH₃ | OH |
| Ia1.568 | CHCH₃ | CH₃ | H | S | OCH₂CH₃ | OH |
| Ia1.569 | CHCH₃ | CH₃ | H | S | OCHF₂ | OH |
| Ia1.570 | CHCH₃ | CH₃ | H | S | OCF₃ | OH |
| Ia1.571 | CHCH₃ | CH₃ | CH₃ | S | SCH₃ | OH |
| Ia1.572 | CHCH₃ | CH₃ | CH₃ | S | SCH₂CH₃ | OH |
| Ia1.573 | CHCH₃ | CH₃ | CH₃ | S | SO₂CH₃ | OH |
| Ia1.574 | CHCH₃ | CH₃ | CH₃ | S | SO₂CH₂CH₃ | OH |
| Ia1.575 | CHCH₃ | CH₃ | CH₃ | S | SO₂CH(CH₃)₂ | OH |
| Ia1.576 | CHCH₃ | CH₃ | CH₃ | S | SO₂(CH₂)₂CH₃ | OH |
| Ia1.577 | CHCH₃ | CH₃ | CH₃ | S | Cl | OH |
| Ia1.578 | CHCH₃ | CH₃ | CH₃ | S | Br | OH |
| Ia1.579 | CHCH₃ | CH₃ | CH₃ | S | NO₂ | OH |
| Ia1.580 | CHCH₃ | CH₃ | CH₃ | S | CHF₂ | OH |
| Ia1.581 | CHCH₃ | CH₃ | CH₃ | S | CF₃ | OH |
| Ia1.582 | CHCH₃ | CH₃ | CH₃ | S | OCH₃ | OH |
| Ia1.583 | CHCH₃ | CH₃ | CH₃ | S | OCH₂CH₃ | OH |
| Ia1.584 | CHCH₃ | CH₃ | CH₃ | S | OCHF₂ | OH |
| Ia1.585 | CHCH₃ | CH₃ | CH₃ | S | OCF₃ | OH |
| Ia1.586 | CHCH₃ | H | H | NCH₃ | SCH₃ | OH |
| Ia1.587 | CHCH₃ | H | H | NCH₃ | SCH₂CH₃ | OH |
| Ia1.588 | CHCH₃ | H | H | NCH₃ | SO₂CH₃ | OH |
| Ia1.589 | CHCH₃ | H | H | NCH₃ | SO₂CH₂CH₃ | OH |
| Ia1.590 | CHCH₃ | H | H | NCH₃ | SO₂CH(CH₃)₂ | OH |
| Ia1.591 | CHCH₃ | H | H | NCH₃ | SO₂(CH₂)₂CH₃ | OH |
| Ia1.592 | CHCH₃ | H | H | NCH₃ | Cl | OH |
| Ia1.593 | CHCH₃ | H | H | NCH₃ | Br | OH |
| Ia1.594 | CHCH₃ | H | H | NCH₃ | NO₂ | OH |
| Ia1.595 | CHCH₃ | H | H | NCH₃ | CHF₂ | OH |
| Ia1.596 | CHCH₃ | H | H | NCH₃ | CF₃ | OH |
| Ia1.597 | CHCH₃ | H | H | NCH₃ | OCH₃ | OH |
| Ia1.598 | CHCH₃ | H | H | NCH₃ | OCH₂CH₃ | OH |
| Ia1.599 | CHCH₃ | H | H | NCH₃ | OCHF₂ | OH |
| Ia1.600 | CHCH₃ | H | H | NCH₃ | OCF₃ | OH |
| Ia1.601 | CHCH₃ | CH₃ | H | NCH₃ | SCH₃ | OH |
| Ia1.602 | CHCH₃ | CH₃ | H | NCH₃ | SCH₂CH₃ | OH |
| Ia1.603 | CHCH₃ | CH₃ | H | NCH₃ | SO₂CH₃ | OH |
| Ia1.604 | CHCH₃ | CH₃ | H | NCH₃ | SO₂CH₂CH₃ | OH |
| Ia1.605 | CHCH₃ | CH₃ | H | NCH₃ | SO₂CH(CH₃)₂ | OH |
| Ia1.606 | CHCH₃ | CH₃ | H | NCH₃ | SO₂(CH₂)₂CH₃ | OH |
| Ia1.607 | CHCH₃ | CH₃ | H | NCH₃ | Cl | OH |
| Ia1.608 | CHCH₃ | CH₃ | H | NCH₃ | Br | OH |
| Ia1.609 | CHCH₃ | CH₃ | H | NCH₃ | NO₂ | OH |
| Ia1.610 | CHCH₃ | CH₃ | H | NCH₃ | CHF₂ | OH |
| Ia1.611 | CHCH₃ | CH₃ | H | NCH₃ | CF₃ | OH |
| Ia1.612 | CHCH₃ | CH₃ | H | NCH₃ | OCH₃ | OH |
| Ia1.613 | CHCH₃ | CH₃ | H | NCH₃ | OCH₂CH₃ | OH |
| Ia1.614 | CHCH₃ | CH₃ | H | NCH₃ | OCHF₂ | OH |
| Ia1.615 | CHCH₃ | CH₃ | H | NCH₃ | OCF₃ | OH |
| Ia1.616 | CHCH₃ | CH₃ | CH₃ | NCH₃ | SCH₃ | OH |
| Ia1.617 | CHCH₃ | CH₃ | CH₃ | NCH₃ | SCH₂CH₃ | OH |
| Ia1.618 | CHCH₃ | CH₃ | CH₃ | NCH₃ | SO₂CH₃ | OH |
| Ia1.619 | CHCH₃ | CH₃ | CH₃ | NCH₃ | SO₂CH₂CH₃ | OH |
| Ia1.620 | CHCH₃ | CH₃ | CH₃ | NCH₃ | SO₂CH(CH₃)₂ | OH |
| Ia1.621 | CHCH₃ | CH₃ | CH₃ | NCH₃ | SO₂(CH₂)₂CH₃ | OH |
| Ia1.622 | CHCH₃ | CH₃ | CH₃ | NCH₃ | Cl | OH |
| Ia1.623 | CHCH₃ | CH₃ | CH₃ | NCH₃ | Br | OH |
| Ia1.624 | CHCH₃ | CH₃ | CH₃ | NCH₃ | NO₂ | OH |
| Ia1.625 | CHCH₃ | CH₃ | CH₃ | NCH₃ | CHF₂ | OH |
| Ia1.626 | CHCH₃ | CH₃ | CH₃ | NCH₃ | CF₃ | OH |
| Ia1.627 | CHCH₃ | CH₃ | CH₃ | NCH₃ | OCH₃ | OH |
| Ia1.628 | CHCH₃ | CH₃ | CH₃ | NCH₃ | OCH₂CH₃ | OH |
| Ia1.629 | CHCH₃ | CH₃ | CH₃ | NCH₃ | OCHF₂ | OH |
| Ia1.630 | CHCH₃ | CH₃ | CH₃ | NCH₃ | OCF₃ | OH |
| Ia1.631 | C(CH₃)₂ | H | H | O | SCH₃ | OH |
| Ia1.632 | C(CH₃)₂ | H | H | O | SCH₂CH₃ | OH |
| Ia1.633 | C(CH₃)₂ | H | H | O | SO₂CH₃ | OH |
| Ia1.634 | C(CH₃)₂ | H | H | O | SO₂CH₂CH₃ | OH |
| Ia1.635 | C(CH₃)₂ | H | H | O | SO₂CH(CH₃)₂ | OH |
| Ia1.636 | C(CH₃)₂ | H | H | O | SO₂(CH₂)₂CH₃ | OH |
| Ia1.637 | C(CH₃)₂ | H | H | O | Cl | OH |
| Ia1.638 | C(CH₃)₂ | H | H | O | Br | OH |
| Ia1.639 | C(CH₃)₂ | H | H | O | NO₂ | OH |
| Ia1.640 | C(CH₃)₂ | H | H | O | CHF₂ | OH |
| Ia1.641 | C(CH₃)₂ | H | H | O | CF₃ | OH |
| Ia1.642 | C(CH₃)₂ | H | H | O | OCH₃ | OH |
| Ia1.643 | C(CH₃)₂ | H | H | O | OCH₂CH₃ | OH |
| Ia1.644 | C(CH₃)₂ | H | H | O | OCHF₂ | OH |
| Ia1.645 | C(CH₃)₂ | H | H | O | OCF₃ | OH |
| Ia1.646 | C(CH₃)₂ | CH₃ | H | O | SCH₃ | OH |
| Ia1.647 | C(CH₃)₂ | CH₃ | H | O | SCH₂CH₃ | OH |
| Ia1.648 | C(CH₃)₂ | CH₃ | H | O | SO₂CH₃ | OH |
| Ia1.649 | C(CH₃)₂ | CH₃ | H | O | SO₂CH₂CH₃ | OH |
| Ia1.650 | C(CH₃)₂ | CH₃ | H | O | SO₂CH(CH₃)₂ | OH |
| Ia1.651 | C(CH₃)₂ | CH₃ | H | O | SO₂(CH₂)₂CH₃ | OH |
| Ia1.652 | C(CH₃)₂ | CH₃ | H | O | Cl | OH |
| Ia1.653 | C(CH₃)₂ | CH₃ | H | O | Br | OH |
| Ia1.654 | C(CH₃)₂ | CH₃ | H | O | NO₂ | OH |
| Ia1.655 | C(CH₃)₂ | CH₃ | H | O | CHF₂ | OH |
| Ia1.656 | C(CH₃)₂ | CH₃ | H | O | CF₃ | OH |
| Ia1.657 | C(CH₃)₂ | CH₃ | H | O | OCH₃ | OH |
| Ia1.658 | C(CH₃)₂ | CH₃ | H | O | OCH₂CH₃ | OH |
| Ia1.659 | C(CH₃)₂ | CH₃ | H | O | OCHF₂ | OH |
| Ia1.660 | C(CH₃)₂ | CH₃ | H | O | OCF₃ | OH |
| Ia1.661 | C(CH₃)₂ | CH₃ | CH₃ | O | SCH₃ | OH |
| Ia1.662 | C(CH₃)₂ | CH₃ | CH₃ | O | SCH₂CH₃ | OH |
| Ia1.663 | C(CH₃)₂ | CH₃ | CH₃ | O | SO₂CH₃ | OH |
| Ia1.664 | C(CH₃)₂ | CH₃ | CH₃ | O | SO₂CH₂CH₃ | OH |
| Ia1.665 | C(CH₃)₂ | CH₃ | CH₃ | O | SO₂CH(CH₃)₂ | OH |
| Ia1.666 | C(CH₃)₂ | CH₃ | CH₃ | O | SO₂(CH₂)₂CH₃ | OH |
| Ia1.667 | C(CH₃)₂ | CH₃ | CH₃ | O | Cl | OH |
| Ia1.668 | C(CH₃)₂ | CH₃ | CH₃ | O | Br | OH |
| Ia1.669 | C(CH₃)₂ | CH₃ | CH₃ | O | NO₂ | OH |
| Ia1.670 | C(CH₃)₂ | CH₃ | CH₃ | O | CHF₂ | OH |
| Ia1.671 | C(CH₃)₂ | CH₃ | CH₃ | O | CF₃ | OH |
| Ia1.672 | C(CH₃)₂ | CH₃ | CH₃ | O | OCH₃ | OH |
| Ia1.673 | C(CH₃)₂ | CH₃ | CH₃ | O | OCH₂CH₃ | OH |
| Ia1.674 | C(CH₃)₂ | CH₃ | CH₃ | O | OCHF₂ | OH |
| Ia1.675 | C(CH₃)₂ | CH₃ | CH₃ | O | OCF₃ | OH |
| Ia1.676 | C(CH₃)₂ | H | H | S | SCH₃ | OH |
| Ia1.677 | C(CH₃)₂ | H | H | S | SCH₂CH₃ | OH |
| Ia1.678 | C(CH₃)₂ | H | H | S | SO₂CH₃ | OH |
| Ia1.679 | C(CH₃)₂ | H | H | S | SO₂CH₂CH₃ | OH |
| Ia1.680 | C(CH₃)₂ | H | H | S | SO₂CH(CH₃)₂ | OH |
| Ia1.681 | C(CH₃)₂ | H | H | S | SO₂(CH₂)₂CH₃ | OH |
| Ia1.682 | C(CH₃)₂ | H | H | S | Cl | OH |
| Ia1.683 | C(CH₃)₂ | H | H | S | Br | OH |
| Ia1.684 | C(CH₃)₂ | H | H | S | NO₂ | OH |
| Ia1.685 | C(CH₃)₂ | H | H | S | CHF₂ | OH |
| Ia1.686 | C(CH₃)₂ | H | H | S | CF₃ | OH |
| Ia1.687 | C(CH₃)₂ | H | H | S | OCH₃ | OH |
| Ia1.688 | C(CH₃)₂ | H | H | S | OCH₂CH₃ | OH |
| Ia1.689 | C(CH₃)₂ | H | H | S | OCHF₂ | OH |
| Ia1.690 | C(CH₃)₂ | H | H | S | OCF₃ | OH |
| Ia1.691 | C(CH₃)₂ | CH₃ | H | S | SCH₃ | OH |
| Ia1.692 | C(CH₃)₂ | CH₃ | H | S | SCH₂CH₃ | OH |
| Ia1.693 | C(CH₃)₂ | CH₃ | H | S | SO₂CH₃ | OH |
| Ia1.694 | C(CH₃)₂ | CH₃ | H | S | SO₂CH₂CH₃ | OH |
| Ia1.695 | C(CH₃)₂ | CH₃ | H | S | SO₂CH(CH₃)₂ | OH |
| Ia1.696 | C(CH₃)₂ | CH₃ | H | S | SO₂(CH₂)₂CH₃ | OH |
| Ia1.697 | C(CH₃)₂ | CH₃ | H | S | Cl | OH |
| Ia1.698 | C(CH₃)₂ | CH₃ | H | S | Br | OH |
| Ia1.699 | C(CH₃)₂ | CH₃ | H | S | NO₂ | OH |
| Ia1.700 | C(CH₃)₂ | CH₃ | H | S | CHF₂ | OH |
| Ia1.701 | C(CH₃)₂ | CH₃ | H | S | CF₃ | OH |
| Ia1.702 | C(CH₃)₂ | CH₃ | H | S | OCH₃ | OH |
| Ia1.703 | C(CH₃)₂ | CH₃ | H | S | OCH₂CH₃ | OH |
| Ia1.704 | C(CH₃)₂ | CH₃ | H | S | OCHF₂ | OH |
| Ia1.705 | C(CH₃)₂ | CH₃ | H | S | OCF₃ | OH |
| Ia1.706 | C(CH₃)₂ | CH₃ | CH₃ | S | SCH₃ | OH |
| Ia1.707 | C(CH₃)₂ | CH₃ | CH₃ | S | SCH₂CH₃ | OH |
| Ia1.708 | C(CH₃)₂ | CH₃ | CH₃ | S | SO₂CH₃ | OH |
| Ia1.709 | C(CH₃)₂ | CH₃ | CH₃ | S | SO₂CH₂CH₃ | OH |
| Ia1.710 | C(CH₃)₂ | CH₃ | CH₃ | S | SO₂CH(CH₃)₂ | OH |
| Ia1.711 | C(CH₃)₂ | CH₃ | CH₃ | S | SO₂(CH₂)₂CH₃ | OH |
| Ia1.712 | C(CH₃)₂ | CH₃ | CH₃ | S | Cl | OH |
| Ia1.713 | C(CH₃)₂ | CH₃ | CH₃ | S | Br | OH |
| Ia1.714 | C(CH₃)₂ | CH₃ | CH₃ | S | NO₂ | OH |
| Ia1.715 | C(CH₃)₂ | CH₃ | CH₃ | S | CHF₂ | OH |
| Ia1.716 | C(CH₃)₂ | CH₃ | CH₃ | S | CF₃ | OH |
| Ia1.717 | C(CH₃)₂ | CH₃ | CH₃ | S | OCH₃ | OH |
| Ia1.718 | C(CH₃)₂ | CH₃ | CH₃ | S | OCH₂CH₃ | OH |
| Ia1.719 | C(CH₃)₂ | CH₃ | CH₃ | S | OCHF₂ | OH |
| Ia1.720 | C(CH₃)₂ | CH₃ | CH₃ | S | OCF₃ | OH |
| Ia1.721 | C(CH₃)₂ | H | H | NCH₃ | SCH₃ | OH |
| Ia1.722 | C(CH₃)₂ | H | H | NCH₃ | SCH₂CH₃ | OH |
| Ia1.723 | C(CH₃)₂ | H | H | NCH₃ | SO₂CH₃ | OH |
| Ia1.724 | C(CH₃)₂ | H | H | NCH₃ | SO₂CH₂CH₃ | OH |
| Ia1.725 | C(CH₃)₂ | H | H | NCH₃ | SO₂CH(CH₃)₂ | OH |
| Ia1.726 | C(CH₃)₂ | H | H | NCH₃ | SO₂(CH₂)₂CH₃ | OH |
| Ia1.727 | C(CH₃)₂ | H | H | NCH₃ | Cl | OH |
| Ia1.728 | C(CH₃)₂ | H | H | NCH₃ | Br | OH |
| Ia1.729 | C(CH₃)₂ | H | H | NCH₃ | NO₂ | OH |
| Ia1.730 | C(CH₃)₂ | H | H | NCH₃ | CHF₂ | OH |
| Ia1.731 | C(CH₃)₂ | H | H | NCH₃ | CF₃ | OH |
| Ia1.732 | C(CH₃)₂ | H | H | NCH₃ | OCH₃ | OH |
| Ia1.733 | C(CH₃)₂ | H | H | NCH₃ | OCH₂CH₃ | OH |
| Ia1.734 | C(CH₃)₂ | H | H | NCH₃ | OCHF₂ | OH |
| Ial. 735 | C(CH₃)₂ | H | H | NCH₃ | OCF₃ | OH |
| Ia1.736 | C(CH₃)₂ | CH₃ | H | NCH₃ | SCH₃ | OH |
| Ia1.737 | C(CH₃)₂ | CH₃ | H | NCH₃ | SCH₂CH₃ | OH |
| Ia1.738 | C(CH₃)₂ | CH₃ | H | NCH₃ | SO₂CH₃ | OH |
| Ia1.739 | C(CH₃)₂ | CH₃ | H | NCH₃ | SO₂CH₂CH₃ | OH |
| Ia1.740 | C(CH₃)₂ | CH₃ | H | NCH₃ | SO₂CH(CH₃)₂ | OH |
| Ia1.741 | C(CH₃)₂ | CH₃ | H | NCH₃ | SO₂(CH₂)₂CH₃ | OH |
| Ia1.742 | C(CH₃)₂ | CH₃ | H | NCH₃ | Cl | OH |
| Ia1.743 | C(CH₃)₂ | CH₃ | H | NCH₃ | Br | OH |
| Ia1.744 | C(CH₃)₂ | CH₃ | H | NCH₃ | NO₂ | OH |
| Ia1.745 | C(CH₃)₂ | CH₃ | H | NCH₃ | CHF₂ | OH |
| Ia1.746 | C(CH₃)₂ | CH₃ | H | NCH₃ | CF₃ | OH |
| Ia1.747 | C(CH₃)₂ | CH₃ | H | NCH₃ | OCH₃ | OH |
| Ia1.748 | C(CH₃)₂ | CH₃ | H | NCH₃ | OCH₂CH₃ | OH |
| Ia1.749 | C(CH₃)₂ | CH₃ | H | NCH₃ | OCHF₂ | OH |
| Ia1.750 | C(CH₃)₂ | CH₃ | H | NCH₃ | OCF₃ | OH |
| Ia1.751 | C(CH₃)₂ | CH₃ | CH₃ | NCH₃ | SCH₃ | OH |
| Ia1.752 | C(CH₃)₂ | CH₃ | CH₃ | NCH₃ | SCH₂CH₃ | OH |
| Ia1.753 | C(CH₃)₂ | CH₃ | CH₃ | NCH₃ | SO₂CH₃ | OH |
| Ia1.754 | C(CH₃)₂ | CH₃ | CH₃ | NCH₃ | SO₂CH₂CH₃ | OH |
| Ia1.755 | C(CH₃)₂ | CH₃ | CH₃ | NCH₃ | SO₂CH(CH₃)₂ | OH |
| Ia1.756 | C(CH₃)₂ | CH₃ | CH₃ | NCH₃ | SO₂(CH₂)₂CH₃ | OH |
| Ia1.757 | C(CH₃)₂ | CH₃ | CH₃ | NCH₃ | Cl | OH |
| Ia1.758 | C(CH₃)₂ | CH₃ | CH₃ | NCH₃ | Br | OH |
| Ia1.759 | C(CH₃)₂ | CH₃ | CH₃ | NCH₃ | NO₂ | OH |
| Ia1.760 | C(CH₃)₂ | CH₃ | CH₃ | NCH₃ | CHF₂ | OH |
| Ia1.761 | C(CH₃)₂ | CH₃ | CH₃ | NCH₃ | CF₃ | OH |
| Ia1.762 | C(CH₃)₂ | CH₃ | CH₃ | NCH₃ | OCH₃ | OH |
| Ia1.763 | C(CH₃)₂ | CH₃ | CH₃ | NCH₃ | OCH₂CH₃ | OH |
| Ia1.764 | C(CH₃)₂ | CH₃ | CH₃ | NCH₃ | OCHF₂ | OH |
| Ia1.765 | C(CH₃)₂ | CH₃ | CH₃ | NCH₃ | OCF₃ | OH |

Ebenso außerordentlichst bevorzugt sind die Verbindungen der Formel Ia2, insbesondere die Verbindungen Ia2.1 bis Ia2.765, die sich von den entsprechenden Verbindungen Ia1.1 bis Ia1.765 dadurch unterscheiden, daß R¹⁴ für Methyl steht.

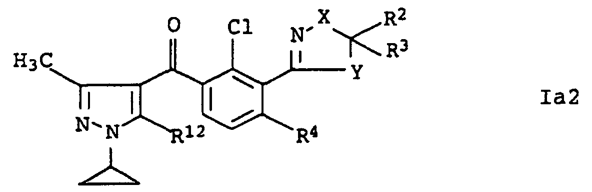

Ebenso außerordentlichst bevorzugt sind die Verbindungen der Formel Ia3, insbesondere die Verbindungen Ia3.1 bis Ia3.765, die sich von den Verbindungen Ia1.1 bis Ia1.765 dadurch unterscheiden, daß R¹³ für Cyclopentyl steht.

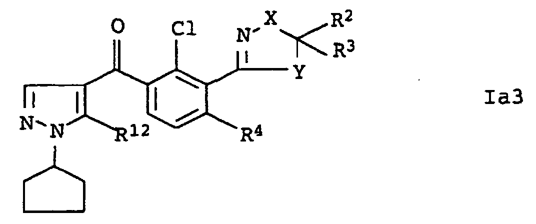

Ebenso außerordentlichst bevorzugt sind die Verbindungen der Formel Ia4, insbesondere die Verbindungen Ia4.1 bis Ia4.765, die sich von den Verbindungen Ia1.1 bis Ia1.765 dadurch unterscheiden, daß R¹³ für Cyclopentyl und R¹⁴ für Methyl stehen.

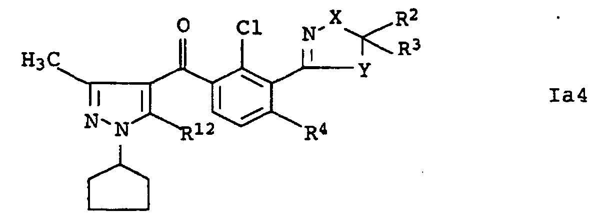

Ebenso außerordentlichst bevorzugt sind die Verbindungen der Formel Ia5, insbesondere die Verbindungen Ia5.1 bis Ia5.765, die sich von den Verbindungen Ia1.1 bis Ia1.765 dadurch unterscheiden, daß R¹³ für Cyclohexyl steht.

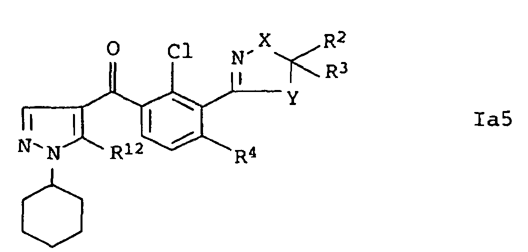

Ebenso außerordentlichst bevorzugt sind die Verbindungen der Formel Ia6, insbesondere die Verbindungen Ia6.1 bis Ia6.765, die sich von den Verbindungen Ia1.1 bis Ia1.765 dadurch unterscheiden, daß R¹³ für Cyclohexyl und R¹⁴ für Methyl stehen.

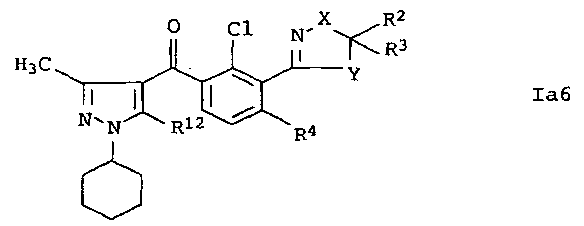

Ebenso außerordentlichst bevorzugt sind die Verbindungen der Formel Ia7, insbesondere die Verbindungen Ia7.1 bis Ia7.765, die sich von den entsprechenden Verbindungen Ia1.1 bis Ia1.765 dadurch unterscheiden, daß R¹ für Methyl steht.

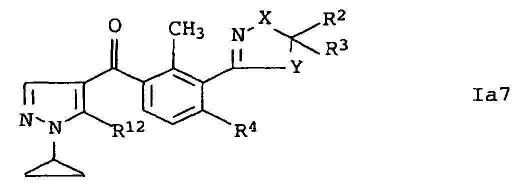

Ebenso außerordentlichst bevorzugt sind die Verbindungen der Formel Ia8, insbesondere die Verbindungen Ia8.1 bis Ia8.765, die sich von den entsprechenden Verbindungen Ia1.1 bis Ia1.765 dadurch unterscheiden, daß R¹ für Methyl und R¹⁴ für Methyl stehen.

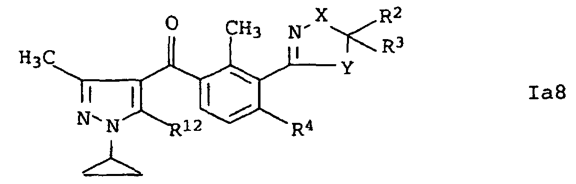

Ebenso außerordentlichst bevorzugt sind die Verbindungen der Formel Ia9, insbesondere die Verbindungen Ia9.1 bis Ia9.765, die sich von den Verbindungen Ia1.1 bis Ia1.765 dadurch unterscheiden, daß R¹ für Methyl und R¹³ für Cyclopentyl stehen.

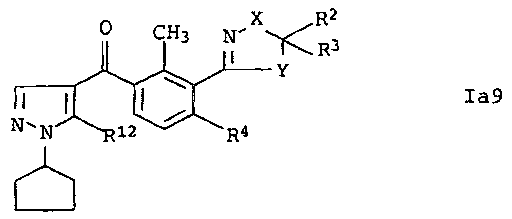

Ebenso außerordentlichst bevorzugt sind die Verbindungen der Formel Ia10, insbesondere die Verbindungen Ia10.1 bis Ia10.765, die sich von den Verbindungen Ia1.1 bis Ia1.765 dadurch unterscheiden, daß R¹ für Methyl, R¹³ für Cyclopentyl und R¹⁴ für Methyl stehen.

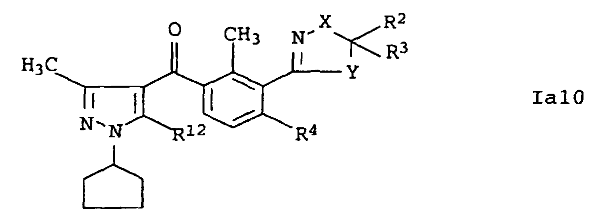

Ebenso außerordentlichst bevorzugt sind die Verbindungen der Formel Ia11, insbesondere die Verbindungen Ia11.1 bis Ia11.765, die sich von den Verbindungen Ia1.1 bis Ia1.765 dadurch unterscheiden, daß R¹ für Methyl und R¹³ für Cyclohexyl stehen.

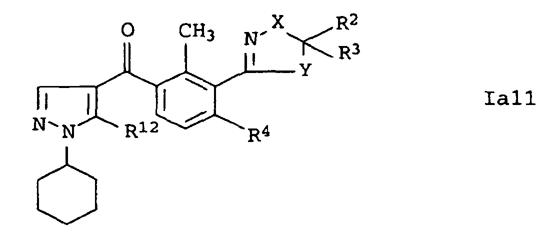

Ebenso außerordentlichst bevorzugt sind die Verbindungen der Formel Ia12, insbesondere die Verbindungen Ia12.1 bis Ia12.765, die sich von den Verbindungen Ia1.1 bis Ia1.765 dadurch unterscheiden, daß R¹ für Methyl R¹³ für Cyclohexyl und R¹⁴ für Methyl stehen.

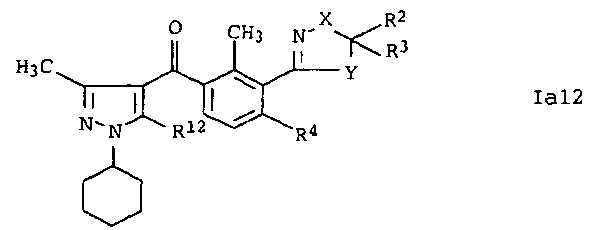

Ebenso außerordentlichst bevorzugt sind die Verbindungen der Formel Ia13, insbesondere die Verbindungen Ia13.1 bis Ia13.765, die sich von den entsprechenden Verbindungen Ia1.1 bis Ia1.765 dadurch unterscheiden, daß R¹ für Methoxy steht.

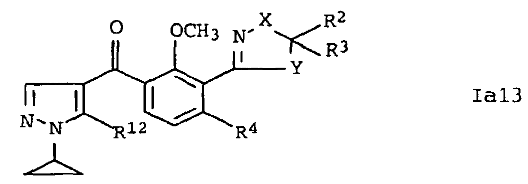

Ebenso außerordentlichst bevorzugt sind die Verbindungen der Formel Ia14, insbesondere die Verbindungen Ia14.1 bis Ia14.765, die sich von den entsprechenden Verbindungen Ia1.1 bis Ia1.765 dadurch unterscheiden, daß R¹ für Methoxy und R¹⁴ für Methyl stehen.

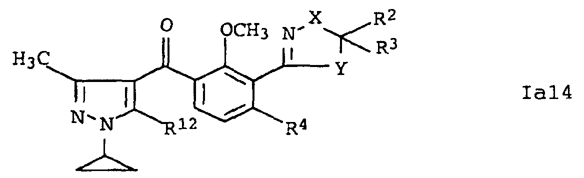

Ebenso außerordentlichst bevorzugt sind die Verbindungen der Formel Ia15, insbesondere die Verbindungen Ia15.1 bis Ia15.765, die sich von den Verbindungen Ia1.1 bis Ia1.765 dadurch unterscheiden, daß R¹ für Methoxy und R¹³ für Cyclopentyl stehen.

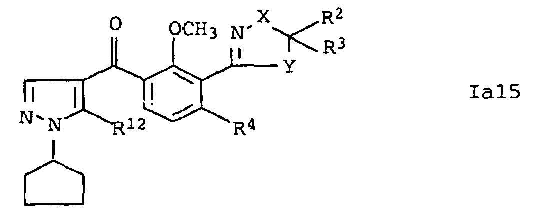

Ebenso außerordentlichst bevorzugt sind die Verbindungen der Formel Ia16, insbesondere die Verbindungen Ia16.1 bis Ia16.765, die sich von den Verbindungen Ia1.1 bis Ia1.765 dadurch unterscheiden, daß R¹ für Methoxy, R¹³ für Cyclopentyl und R¹⁴ für Methyl stehen.

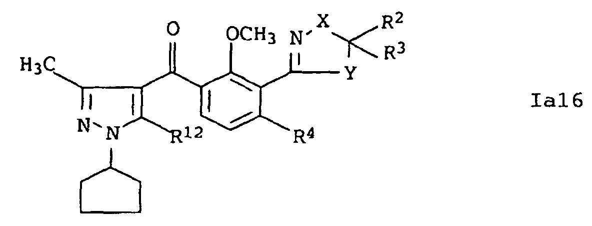

Ebenso außerordentlichst bevorzugt sind die Verbindungen der Formel Ia17, insbesondere die Verbindungen Ia17.1 bis Ia17.765, die sich von den Verbindungen Ia1.1 bis Ia1.765 dadurch unterscheiden, daß R¹ für Methoxy und R¹³ für Cyclohexyl stehen.

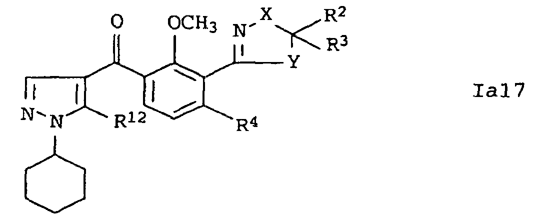

Ebenso außerordentlichst bevorzugt sind die Verbindungen der Formel Ia18, insbesondere die Verbindungen Ia18.1 bis Ia18.765, die sich von den Verbindungen Ia1.1 bis Ia1.765 dadurch unterscheiden, daß R¹ für Methoxy R¹³ für Cyclohexyl und R¹⁴ für Methyl stehen.

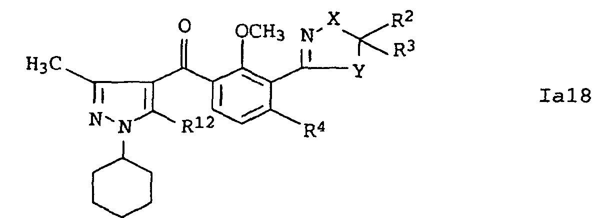

Ebenso außerordentlich bevorzugt sind die Verbindungen der Formel Ib1 (≡ I mit R¹ = Cl, R³ und R⁹ bzw. R³ und R¹¹ bilden gemeinsam eine Bindung aus, R¹³ = cyclo-C₃H₅ und R⁵ und R¹⁴ = H) , insbesondere die Verbindungen Ib1.1 bis Ib1.420 der Tabelle 2, wobei die Restedefinitionen X, Y und R¹ bis R¹⁴ nicht nur in Kombination miteinander, sondern auch jeweils für sich allein betrachtet für die erfindungsgemäßen Verbindungen eine besondere Bedeutung haben.

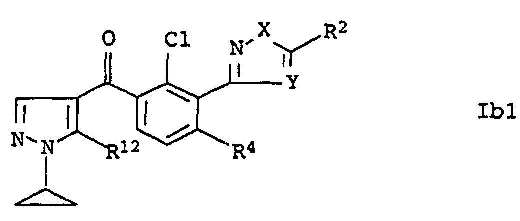

**Tabelle 2:**

| Nr. | X | R² | Y | R⁴ | R¹² |
|---|---|---|---|---|---|
| Ib1.1 | O | H | CH | SCH₃ | OH |
| Ib1.2 | O | H | CH | SCH₂CH₃ | OH |
| Ib1.3 | O | H | CH | SO₂CH₃ | OH |
| Ib1.4 | O | H | CH | SO₂CH₂CH₃ | OH |
| Ib1.5 | O | H | CH | SO₂CH(CH₃)₂ | OH |
| Ib1.6 | O | H | CH | SO₂(CH₂)₂CH₃ | OH |
| Ib1.7 | O | H | CH | Cl | OH |
| Ib1.8 | O | H | CH | Br | OH |
| Ib1.9 | O | H | CH | NO₂ | OH |
| Ib1.10 | O | H | CH | CHF₂ | OH |
| Ib1.11 | O | H | CH | CF₃ | OH |
| Ib1.12 | O | H | CH | OCH₃ | OH |
| Ib1.13 | O | H | CH | OCH₂CH₃ | OH |
| Ib1.14 | O | H | CH | OCHF₂ | OH |
| Ib1.15 | O | H | CH | OCF₃ | OH |
| Ib1.16 | O | CH₃ | CH | SCH₃ | OH |
| Ib1.17 | O | CH₃ | CH | SCH₂CH₃ | OH |
| Ib1.18 | O | CH₃ | CH | SO₂CH₃ | OH |
| Ib1.19 | O | CH₃ | CH | SO₂CH₂CH₃ | OH |
| Ib1.20 | O | CH₃ | CH | SO₂CH(CH₃)₂ | OH |
| Ib1.21 | O | CH₃ | CH | SO₂(CH₂)₂CH₃ | OH |
| Ib1.22 | O | CH₃ | CH | Cl | OH |
| Ib1.23 | O | CH₃ | CH | Br | OH |
| Ib1.24 | O | CH₃ | CH | NO₂ | OH |
| Ib1.25 | O | CH₃ | CH | CHF₂ | OH |
| Ib1.26 | O | CH₃ | CH | CF₃ | OH |
| Ib1.27 | O | CH₃ | CH | OCH₃ | OH |
| Ib1.28 | O | CH₃ | CH | OCH₂CH₃ | OH |
| Ib1.29 | O | CH₃ | CH | OCHF₂ | OH |
| Ib1.30 | O | CH₃ | CH | OCF₃ | OH |
| Ib1.31 | O | CH₂CH₃ | CH | SCH₃ | OH |
| Ib1.32 | O | CH₂CH₃ | CH | SCH₂CH₃ | OH |
| Ib1.33 | O | CH₂CH₃ | CH | SO₂CH₃ | OH |
| Ib1.34 | O | CH₂CH₃ | CH | SO₂CH₂CH₃ | OH |
| Ib1.35 | O | CH₂CH₃ | CH | SO₂CH(CH₃)₂ | OH |
| Ib1.36 | O | CH₂CH₃ | CH | SO₂(CH₂)₂CH₃ | OH |
| Ib1.37 | O | CH₂CH₃ | CH | Cl | OH |
| Ib1.38 | O | CH₂CH₃ | CH | Br | OH |
| Ib1.39 | O | CH₂CH₃ | CH | NO₂ | OH |
| Ib1.40 | O | CH₂CH₃ | CH | CHF₂ | OH |
| Ib1.41 | O | CH₂CH₃ | CH | CF₃ | OH |
| Ib1.42 | O | CH₂CH₃ | CH | OCH₃ | OH |
| Ib1.43 | O | CH₂CH₃ | CH | OCH₂CH₃ | OH |
| Ib1.44 | O | CH₂CH₃ | CH | OCHF₂ | OH |
| Ib1.45 | O | CH₂CH₃ | CH | OCF₃ | OH |
| Ib1.46 | O | CH₂Cl | CH | SCH₃ | OH |
| Ib1.47 | O | CH₂Cl | CH | SCH₂CH₃ | OH |
| Ib1.48 | O | CH₂Cl | CH | SO₂CH₃ | OH |
| Ib1.49 | O | CH₂Cl | CH | SO₂CH₂CH₃ | OH |
| Ib1.50 | O | CH₂Cl | CH | SO₂CH(CH₃)₂ | OH |
| Ib1.51 | O | CH₂Cl | CH | SO₂(CH₂)₂CH₃ | OH |
| Ib1.52 | O | CH₂Cl | CH | Cl | OH |
| Ib1.53 | O | CH₂Cl | CH | Br | OH |
| Ib1.54 | O | CH₂Cl | CH | NO₂ | OH |
| Ib1.55 | O | CH₂Cl | CH | CHF₂ | OH |
| Ib1.56 | O | CH₂Cl | CH | CF₃ | OH |
| Ib1.57 | O | CH₂Cl | CH | OCH₃ | OH |
| Ib1.58 | O | CH₂Cl | CH | OCH₂CH₃ | OH |
| Ib1.59 | O | CH₂Cl | CH | OCHF₂ | OH |
| Ib1.60 | O | CH₂Cl | CH | OCF₃ | OH |
| Ib1.61 | O | H | C(CH₃) | SCH₃ | OH |
| Ib1.62 | O | H | C(CH₃) | SCH₂CH₃ | OH |
| Ib1.63 | O | H | C(CH₃) | SO₂CH₃ | OH |
| Ib1.64 | O | H | C(CH₃) | SO₂CH₂CH₃ | OH |
| Ib1.65 | O | H | C(CH₃) | SO₂CH(CH₃)₂ | OH |
| Ib1.66 | O | H | C(CH₃) | SO₂(CH₂)₂CH₃ | OH |
| Ib1.67 | O | H | C(CH₃) | Cl | OH |
| Ib1.68 | O | H | C(CH₃) | Br | OH |
| Ib1.69 | O | H | C(CH₃) | NO₂ | OH |
| Ib1.70 | O | H | C(CH₃) | CHF₂ | OH |
| Ib1.71 | O | H | C(CH₃) | CF₃ | OH |
| Ib1.72 | O | H | C(CH₃) | OCH₃ | OH |
| Ib1.73 | O | H | C(CH₃) | OCH₂CH₃ | OH |
| Ib1.74 | O | H | C(CH₃) | OCHF₂ | OH |
| Ib1.75 | O | H | C(CH₃) | OCF₃ | OH |
| Ib1.76 | O | CH₃ | C(CH₃) | SCH₃ | OH |
| Ib1.77 | O | CH₃ | C(CH₃) | SCH₂CH₃ | OH |
| Ib1.78 | O | CH₃ | C(CH₃) | SO₂CH₃ | OH |
| Ib1.79 | O | CH₃ | C(CH₃) | SO₂CH₂CH₃ | OH |
| Ib1.80 | O | CH₃ | C(CH₃) | SO₂CH(CH₃)₂ | OH |
| Ib1.81 | O | CH₃ | C(CH₃) | SO₂(CH₂)₂CH₃ | OH |
| Ib1.82 | O | CH₃ | C(CH₃) | Cl | OH |
| Ib1.83 | O | CH₃ | C(CH₃) | Br | OH |
| Ib1.84 | O | CH₃ | C(CH₃) | NO₂ | OH |
| Ib1.85 | O | CH₃ | C(CH₃) | CHF₂ | OH |
| Ib1.86 | O | CH₃ | C(CH₃) | CF₃ | OH |
| Ib1.87 | O | CH₃ | C(CH₃) | OCH₃ | OH |
| Ib1.88 | O | CH₃ | C(CH₃) | OCH₂CH₃ | OH |
| Ib1.89 | O | CH₃ | C(CH₃) | OCHF₂ | OH |
| Ib1.90 | O | CH₃ | C(CH₃) | OCF₃ | OH |
| Ib1.91 | O | CH₂CH₃ | C(CH₃) | SCH₃ | OH |
| Ib1.92 | O | CH₂CH₃ | C(CH₃) | SCH₂CH₃ | OH |
| Ib1.93 | O | CH₂CH₃ | C(CH₃) | SO₂CH₃ | OH |
| Ib1.94 | O | CH₂CH₃ | C(CH₃) | SO₂CH₂CH₃ | OH |
| Ib1.95 | O | CH₂CH₃ | C(CH₃) | SO₂CH(CH₃)₂ | OH |
| Ib1.96 | O | CH₂CH₃ | C(CH₃) | SO₂(CH₂)₂CH₃ | OH |
| Ib1.97 | O | CH₂CH₃ | C(CH₃) | Cl | OH |
| Ib1.98 | O | CH₂CH₃ | C(CH₃) | Br | OH |
| Ib1.99 | O | CH₂CH₃ | C(CH₃) | NO₂ | OH |
| Ib1.100 | O | CH₂CH₃ | C(CH₃) | CHF₂ | OH |
| Ib1.101 | O | CH₂CH₃ | C(CH₃) | CF₃ | OH |
| Ib1.102 | O | CH₂CH₃ | C(CH₃) | OCH₃ | OH |
| Ib1.103 | O | CH₂CH₃ | C(CH₃) | OCH₂CH₃ | OH |
| Ib1.104 | O | CH₂CH₃ | C(CH₃) | OCHF₂ | OH |
| Ib1.105 | O | CH₂CH₃ | C(CH₃) | OCF₃ | OH |
| Ib1.106 | O | CH₂Cl | C(CH₃) | SCH₃ | OH |
| Ib1.107 | O | CH₂Cl | C(CH₃) | SCH₂CH₃ | OH |
| Ib1.108 | O | CH₂Cl | C(CH₃) | SO₂CH₃ | OH |
| Ib1.109 | O | CH₂Cl | C(CH₃) | SO₂CH₂CH₃ | OH |
| Ib1.110 | O | CH₂Cl | C(CH₃) | SO₂CH(CH₃)₂ | OH |
| Ib1.111 | O | CH₂Cl | C(CH₃) | SO₂(CH₂)₂CH₃ | OH |
| Ib1.112 | O | CH₂Cl | C(CH₃) | Cl | OH |
| Ib1.113 | O | CH₂Cl | C(CH₃) | Br | OH |
| Ib1.114 | O | CH₂Cl | C(CH₃) | NO₂ | OH |
| Ib1.115 | O | CH₂Cl | C(CH₃) | CHF₂ | OH |
| Ib1.116 | O | CH₂Cl | C(CH₃) | CF₃ | OH |
| Ib1.117 | O | CH₂Cl | C(CH₃) | OCH₃ | OH |
| Ib1.118 | O | CH₂Cl | C(CH₃) | OCH₂CH₃ | OH |
| Ib1.119 | O | CH₂Cl | C(CH₃) | OCHF₂ | OH |
| Ib1.120 | O | CH₂Cl | C(CH₃) | OCF₃ | OH |
| Ib1.121 | O | H | C(CH₂CH₃) | SCH₃ | OH |
| Ib1.122 | O | H | C(CH₂CH₃) | SCH₂CH₃ | OH |
| Ib1.123 | O | H | C(CH₂CH₃) | SO₂CH₃ | OH |
| Ib1.124 | O | H | C(CH₂CH₃) | SO₂CH₂CH₃ | OH |
| Ib1.125 | O | H | C(CH₂CH₃) | SO₂CH(CH₃)₂ | OH |
| Ib1.126 | O. | H | C(CH₂CH₃) | SO₂(CH₂)₂CH₃ | OH |
| Ib1.127 | O | H | C(CH₂CH₃) | Cl | OH |
| Ib1.128 | O | H | C(CH₂CH₃) | Br | OH |
| Ib1.129 | O | H | C(CH₂CH₃) | NO₂ | OH |
| Ib1.130 | O | H | C(CH₂CH₃) | CHF₂ | OH |
| Ib1.131 | O | H | C(CH₂CH₃) | CF₃ | OH |
| Ib1.132 | O | H | C(CH₂CH₃) | OCH₃ | OH |
| Ib1.133 | O | H | C(CH₂CH₃) | OCH₂CH₃ | OH |
| Ib1.134 | O | H | C(CH₂CH₃) | OCHF₂ | OH |
| Ib1.135 | O | H | C(CH₂CH₃) | OCF₃ | OH |
| Ib1.136 | O | CH₃ | C(CH₂CH₃) | SCH₃ | OH |
| Ib1.137 | O | CH₃ | C(CH₂CH₃) | SCH₂CH₃ | OH |
| Ib1.138 | O | CH₃ | C(CH₂CH₃) | SO₂CH₃ | OH |
| Ib1.139 | O | CH₃ | C(CH₂CH₃) | SO₂CH₂CH₃ | OH |
| Ib1.140 | O | CH₃ | C(CH₂CH₃) | SO₂CH(CH₃)₂ | OH |
| Ib1.141 | O | CH₃ | C(CH₂CH₃) | SO₂(CH₂)₂CH₃ | OH |
| Ib1.142 | O | CH₃ | C(CH₂CH₃) | Cl | OH |
| Ib1.143 | O | CH₃ | C(CH₂CH₃) | Br | OH |
| Ib1.144 | O | CH₃ | C(CH₂CH₃) | NO₂ | OH |
| Ib1.145 | O | CH₃ | C(CH₂CH₃) | CHF₂ | OH |
| Ib1.146 | O | CH₃ | C(CH₂CH₃) | CF₃ | OH |
| Ib1.147 | O | CH₃ | C (CH₂CH₃) | OCH₃ | OH |
| Ib1.148 | O | CH₃ | C(CH₂CH₃) | OCH₂CH₃ | OH |
| Ib1.149 | O | CH₃ | C(CH₂CH₃) | OCHF₂ | OH |
| Ib1.150 | O | CH₃ | C(CH₂CH₃) | OCF₃ | OH |
| Ib1.151 | O | CH₂CH₃ | C(CH₂CH₃) | SCH₃ | OH |
| Ib1.152 | O | CH₂CH₃ | C(CH₂CH₃) | SCH₂CH₃ | OH |
| Ib1.153 | O | CH₂CH₃ | C(CH₂CH₃) | SO₂CH₃ | OH |
| Ib1.154 | O | CH₂CH₃ | C(CH₂CH₃) | SO₂CH₂CH₃ | OH |
| Ib1.155 | O | CH₂CH₃ | C(CH₂CH₃) | SO₂CH(CH₃)₂ | OH |
| Ib1.156 | O | CH₂CH₃ | C(CH₂CH₃) | SO₂(CH₂)₂CH₃ | OH |
| Ib1.157 | O | CH₂CH₃ | C(CH₂CH₃) | Cl | OH |
| Ib1.158 | O | CH₂CH₃ | C(CH₂CH₃) | Br | OH |
| Ib1.159 | O | CH₂CH₃ | C(CH₂CH₃) | NO₂ | OH |
| Ib1.160 | O | CH₂CH₃ | C(CH₂CH₃) | CHF₂ | OH |
| Ib1.161 | O | CH₂CH₃ | C(CH₂CH₃) | CF₃ | OH |
| Ib1.162 | O | CH₂CH₃ | C(CH₂CH₃) | OCH₃ | OH |
| Ib1.163 | O | CH₂CH₃ | C(CH₂CH₃) | OCH₂CH₃ | OH |
| Ib1.164 | O | CH₂CH₃ | C(CH₂CH₃) | OCHF₂ | OH |
| Ib1.165 | O | CH₂CH₃ | C(CH₂CH₃) | OCF₃ | OH |
| Ib1.166 | O | CH₂Cl | C(CH₂CH₃) | SCH₃ | OH |
| Ib1.167 | O | CH₂Cl | C(CH₂CH₃) | SCH₂CH₃ | OH |
| Ib1.168 | O | CH₂Cl | C(CH₂CH₃) | SO₂CH₃ | OH |
| Ib1.169 | O | CH₂Cl | C(CH₂CH₃) | SO₂CH₂CH₃ | OH |
| Ib1.170 | O | CH₂Cl | C(CH₂CH₃) | SO₂CH(CH₃)₂ | OH |
| Ib1.171 | O | CH₂Cl | C(CH₂CH₃) | SO₂(CH₂)₂CH₃ | OH |
| Ib1.172 | O | CH₂Cl | C(CH₂CH₃) | Cl | OH |
| Ib1.173 | O | CH₂Cl | C(CH₂CH₃) | Br | OH |
| Ib1.174 | O | CH₂Cl | C(CH₂CH₃) | NO₂ | OH |
| Ib1.175 | O | CH₂Cl | C(CH₂CH₃) | CHF₂ | OH |
| Ib1.176 | O | CH₂Cl | C(CH₂CH₃) | CF₃ | OH |
| Ib1.177 | O | CH₂Cl | C(CH₂CH₃) | OCH₃ | OH |
| Ib1.178 | O | CH₂Cl | C(CH₂CH₃) | OCH₂CH₃ | OH |
| Ib1.179 | O | CH₂Cl | C(CH₂CH₃) | OCHF₂ | OH |
| Ib1.180 | O | CH₂Cl | C(CH₂CH₃) | OCF₃ | OH |
| Ib1.181 | NCH₃ | H | CH | SCH₃ | OH |
| Ib1.182 | NCH₃ | H | CH | SCH₂CH₃ | OH |
| Ib1.183 | NCH₃ | H | CH | SO₂CH₃ | OH |
| Ib1.184 | NCH₃ | H | CH | SO₂CH₂CH₃ | OH |
| Ib1.185 | NCH₃ | H | CH | SO₂CH(CH₃)₂ | OH |
| Ib1.186 | NCH₃ | H | CH | SO₂(CH₂)₂CH₃ | OH |
| Ib1.187 | NCH₃ | H | CH | Cl | OH |
| Ib1.188 | NCH₃ | H | CH | Br | OH |
| Ib1.189 | NCH₃ | H | CH | NO₂ | OH |
| Ib1.190 | NCH₃ | H | CH | CHF₂ | OH |
| Ib1.191 | NCH₃ | H | CH | CF₃ | OH |
| Ib1.192 | NCH₃ | H | CH | OCH₃ | OH |
| Ib1.193 | NCH₃ | H | CH | OCH₂CH₃ | OH |
| Ib1.194 | NCH₃ | H | CH | OCHF₂ | OH |
| Ib1.195 | NCH₃ | H | CH | OCF₃ | OH |
| Ib1.196 | NCH₃ | CH₃ | CH | SCH₃ | OH |
| Ib1.197 | NCH₃ | CH₃ | CH | SCH₂CH₃ | OH |
| Ib1.198 | NCH₃ | CH₃ | CH | SO₂CH₃ | OH |
| Ib1.199 | NCH₃ | CH₃ | CH | SO₂CH₂CH₃ | OH |
| Ib1.200 | NCH₃ | CH₃ | CH | SO₂CH(CH₃)₂ | OH |
| Ib1.201 | NCH₃ | CH₃ | CH | SO₂(CH₂)₂CH₃ | OH |
| Ib1.202 | NCH₃ | CH₃ | CH | Cl | OH |
| Ib1.203 | NCH₃ | CH₃ | CH | Br | OH |
| Ib1.204 | NCH₃ | CH₃ | CH | NO₂ | OH |
| Ib1.205 | NCH₃ | CH₃ | CH | CHF₂ | OH |
| Ib1.206 | NCH₃ | CH₃ | CH | CF₃ | OH |
| Ib1.207 | NCH₃ | CH₃ | CH | OCH₃ | OH |
| Ib1.208 | NCH₃ | CH₃ | CH | OCH₂CH₃ | OH |
| Ib1.209 | NCH₃ | CH₃ | CH | OCHF₂ | OH |
| Ib1.210 | NCH₃ | CH₃ | CH | OCF₃ | OH |
| Ib1.211 | NCH₃ | CH₂CH₃ | CH | SCH₃ | OH |
| Ib1.212 | NCH₃ | CH₂CH₃ | CH | SCH₂CH₃ | OH |
| Ib1.213 | NCH₃ | CH₂CH₃ | CH | SO₂CH₃ | OH |
| Ib1.214 | NCH₃ | CH₂CH₃ | CH | SO₂CH₂CH₃ | OH |
| Ib1.215 | NCH₃ | CH₂CH₃ | CH | SO₂CH(CH₃)₂ | OH |
| Ib1.216 | NCH₃ | CH₂CH₃ | CH | SO₂(CH₂)₂CH₃ | OH |
| Ib1.217 | NCH₃ | CH₂CH₃ | CH | Cl | OH |
| Ib1.218 | NCH₃ | CH₂CH₃ | CH | Br | OH |
| Ib1.219 | NCH₃ | CH₂CH₃ | CH | NO₂ | OH |
| Ib1.220 | NCH₃ | CH₂CH₃ | CH | CHF₂ | OH |
| Ib1.221 | NCH₃ | CH₂CH₃ | CH | CF₃ | OH |
| Ib1.222 | NCH₃ | CH₂CH₃ | CH | OCH₃ | OH |
| Ib1.223 | NCH₃ | CH₂CH₃ | CH | OCH₂CH₃ | OH |
| Ib1.224 | NCH₃ | CH₂CH₃ | CH | OCHF₂ | OH |
| Ib1.225 | NCH₃ | CH₂CH₃ | CH | OCF₃ | OH |
| Ib1.226 | NCH₃ | CH₂Cl | CH | SCH₃ | OH |
| Ib1.227 | NCH₃ | CH₂Cl | CH | SCH₂CH₃ | OH |
| Ib1.228 | NCH₃ | CH₂Cl | CH | SO₂CH₃ | OH |
| Ib1.229 | NCH₃ | CH₂Cl | CH | SO₂CH₂CH₃ | OH |
| Ib1.230 | NCH₃ | CH₂Cl | CH | SO₂CH(CH₃)₂ | OH |
| Ib1.231 | NCH₃ | CH₂Cl | CH | SO₂(CH₂)₂CH₃ | OH |
| Ib1.232 | NCH₃ | CH₂Cl | CH | Cl | OH |
| Ib1.233 | NCH₃ | CH₂Cl | CH | Br | OH |
| Ib1.234 | NCH₃ | CH₂Cl | CH | NO₂ | OH |
| Ib1.235 | NCH₃ | CH₂Cl | CH | CHF₂ | OH |
| Ib1.236 | NCH₃ | CH₂Cl | CH | CF₃ | OH |
| Ib1.237 | NCH₃ | CH₂Cl | CH | OCH₃ | OH |
| Ib1.238 | NCH₃ | CH₂Cl | CH | OCH₂CH₃ | OH |
| Ib1.239 | NCH₃ | CH₂Cl | CH | OCHF₂ | OH |
| Ib1.240 | NCH₃ | CH₂Cl | CH | OCF₃ | OH |
| Ib1.241 | NCH₃ | H | C(CH₃) | SCH₃ | OH |
| Ib1.242 | NCH₃ | H | C(CH₃) | SCH₂CH₃ | OH |
| Ib1.243 | NCH₃ | H | C(CH₃) | SO₂CH₃ | OH |
| Ib1.244 | NCH₃ | H | C(CH₃) | SO₂CH₂CH₃ | OH |
| Ib1.245 | NCH₃ | H | C(CH₃) | SO₂CH(CH₃)₂ | OH |
| Ib1.246 | NCH₃ | H | C(CH₃) | SO₂(CH₂)₂CH₃ | OH |
| Ib1.247 | NCH₃ | H | C(CH₃) | Cl | OH |
| Ib1.248 | NCH₃ | H | C(CH₃) | Br | OH |
| Ib1.249 | NCH₃ | H | C(CH₃) | NO₂ | OH |
| Ib1.250 | NCH₃ | H | C(CH₃) | CHF₂ | OH |
| Ib1.251 | NCH₃ | H | C(CH₃) | CF₃ | OH |
| Ib1.252 | NCH₃ | H | C(CH₃) | OCH₃ | OH |
| Ib1.253 | NCH₃ | H | C(CH₃) | OCH₂CH₃ | OH |
| Ib1.254 | NCH₃ | H | C(CH₃) | OCHF₂ | OH |
| Ib1.255 | NCH₃ | H | C(CH₃) | OCF₃ | OH |
| Ib1.256 | NCH₃ | CH₃ | C(CH₃) | SCH₃ | OH |
| Ib1.257 | NCH₃ | CH₃ | C(CH₃) | SCH₂CH₃ | OH |
| Ib1.258 | NCH₃ | CH₃ | C(CH₃) | SO₂CH₃ | OH |
| Ib1.259 | NCH₃ | CH₃ | C(CH₃) | SO₂CH₂CH₃ | OH |
| Ib1.260 | NCH₃ | CH₃ | C(CH₃) | SO₂CH(CH₃)₂ | OH |
| Ib1.261 | NCH₃ | CH₃ | C(CH₃) | SO₂(CH₂)₂CH₃ | OH |
| Ib1.262 | NCH₃ | CH₃ | C(CH₃) | Cl | OH |
| Ib1.263 | NCH₃ | CH₃ | C(CH₃) | Br | OH |
| Ib1.264 | NCH₃ | CH₃ | C(CH₃) | NO₂ | OH |
| Ib1.265 | NCH₃ | CH₃ | C(CH₃) | CHF₂ | OH |
| Ib1.266 | NCH₃ | CH₃ | C(CH₃) | CF₃ | OH |
| Ib1.267 | NCH₃ | CH₃ | C(CH₃) | OCH₃ | OH |
| Ib1.268 | NCH₃ | CH₃ | C(CH₃) | OCH₂CH₃ | OH |
| Ib1.269 | NCH₃ | CH₃ | C(CH₃) | OCHF₂ | OH |
| Ib1.270 | NCH₃ | CH₃ | C(CH₃) | OCF₃ | OH |
| Ib1.271 | NCH₃ | CH₂CH₃ | C(CH₃) | SCH₃ | OH |
| Ib1.272 | NCH₃ | CH₂CH₃ | C(CH₃) | SCH₂CH₃ | OH |
| Ib1.273 | NCH₃ | CH₂CH₃ | C(CH₃) | SO₂CH₃ | OH |
| Ib1.274 | NCH₃ | CH₂CH₃ | C(CH₃) | SO₂CH₂CH₃ | OH |
| Ib1.275 | NCH₃ | CH₂CH₃ | C(CH₃) | SO₂CH(CH₃)₂ | OH |
| Ib1.276 | NCH₃ | CH₂CH₃ | C(CH₃) | SO₂(CH₂)₂CH₃ | OH |
| Ib1.277 | NCH₃ | CH₂CH₃ | C(CH₃) | Cl | OH |
| Ib1.278 | NCH₃ | CH₂CH₃ | C(CH₃) | Br | OH |
| Ib1.279 | NCH₃ | CH₂CH₃ | C(CH₃) | NO₂ | OH |
| Ib1.280 | NCH₃ | CH₂CH₃ | C(CH₃) | CHF₂ | OH |
| Ib1.281 | NCH₃ | CH₂CH₃ | C(CH₃) | CF₃ | OH |
| Ib1.282 | NCH₃ | CH₂CH₃ | C(CH₃) | OCH₃ | OH |
| Ib1.283 | NCH₃ | CH₂CH₃ | C(CH₃) | OCH₂CH₃ | OH |
| Ib1.284 | NCH₃ | CH₂CH₃ | C(CH₃) | OCHF₂ | OH |
| Ib1.285 | NCH₃ | CH₂CH₃ | C(CH₃) | OCF₃ | OH |
| Ib1.286 | NCH₃ | CH₂Cl | C(CH₃) | SCH₃ | OH |
| Ib1.287 | NCH₃ | CH₂Cl | C(CH₃) | SCH₂CH₃ | OH |
| Ib1.288 | NCH₃ | CH₂Cl | C(CH₃) | SO₂CH₃ | OH |
| Ib1.289 | NCH₃ | CH₂Cl | C(CH₃) | SO₂CH₂CH₃ | OH |
| Ib1.290 | NCH₃ | CH₂Cl | C(CH₃) | SO₂CH(CH₃)₂ | OH |
| Ib1.291 | NCH₃ | CH₂Cl | C(CH₃) | SO₂(CH₂)₂CH₃ | OH |
| Ib1.292 | NCH₃ | CH₂Cl | C(CH₃) | Cl | OH |
| Ib1.293 | NCH₃ | CH₂Cl | C(CH₃) | Br | OH |
| Ib1.294 | NCH₃ | CH₂Cl | C(CH₃) | NO₂ | OH |
| Ib1.295 | NCH₃ | CH₂Cl | C(CH₃) | CHF₂ | OH |
| Ib1.296 | NCH₃ | CH₂Cl | C(CH₃) | CF₃ | OH |
| Ib1.297 | NCH₃ | CH₂Cl | C(CH₃) | OCH₃ | OH |
| Ib1.298 | NCH₃ | CH₂Cl | C(CH₃) | OCH₂CH₃ | OH |
| Ib1.299 | NCH₃ | CH₂Cl | C(CH₃) | OCHF₂ | OH |
| Ib1.300 | NCH₃ | CH₂Cl | C(CH₃) | OCF₃ | OH |
| Ib1.301 | NCH₃ | H | C(CH₂CH₃) | SCH₃ | OH |
| Ib1.302 | NCH₃ | H | C(CH₂CH₃) | SCH₂CH₃ | OH |
| Ib1.303 | NCH₃ | H | C(CH₂CH₃) | SO₂CH₃ | OH |
| Ib1.304 | NCH₃ | H | C(CH₂CH₃) | SO₂CH₂CH₃ | OH |
| Ib1.305 | NCH₃ | H | C(CH₂CH₃) | SO₂CH(CH₃)₂ | OH |
| Ib1.306 | NCH₃ | H | C(CH₂CH₃) | SO₂(CH₂)₂CH₃ | OH |
| Ib1.307 | NCH₃ | H | C(CH₂CH₃) | Cl | OH |
| Ib1.308 | NCH₃ | H | C(CH₂CH₃) | Br | OH |
| Ib1.309 | NCH₃ | H | C(CH₂CH₃) | NO₂ | OH |
| Ib1.310 | NCH₃ | H | C(CH₂CH₃) | CHF₂ | OH |
| Ib1.311 | NCH₃ | H | C(CH₂CH₃) | CF₃ | OH |
| Ib1.312 | NCH₃ | H | C(CH₂CH₃) | OCH₃ | OH |
| Ib1.313 | NCH₃ | H | C(CH₂CH₃) | OCH₂CH₃ | OH |
| Ib1.314 | NCH₃ | H | C(CH₂CH₃) | OCHF₂ | OH |
| Ib1.315 | NCH₃ | H | C(CH₂CH₃) | OCF₃ | OH |
| Ib1.316 | NCH₃ | CH₃ | C(CH₂CH₃) | SCH₃ | OH |
| Ib1.317 | NCH₃ | CH₃ | C(CH₂CH₃) | SCH₂CH₃ | OH |
| Ib1.318 | NCH₃ | CH₃ | C(CH₂CH₃) | SO₂CH₃ | OH |
| Ib1.319 | NCH₃ | CH₃ | C(CH₂CH₃) | SO₂CH₂CH₃ | OH |
| Ib1.320 | NCH₃ | CH₃ | C(CH₂CH₃) | SO₂CH(CH₃)₂ | OH |
| Ib1.321 | NCH₃ | CH₃ | C(CH₂CH₃) | SO₂(CH₂)₂CH₃ | OH |
| Ib1.322 | NCH₃ | CH₃ | C(CH₂CH₃) | Cl | OH |
| Ib1.323 | NCH₃ | CH₃ | C(CH₂CH₃) | Br | OH |
| Ib1.324 | NCH₃ | CH₃ | C(CH₂CH₃) | NO₂ | OH |
| Ib1.325 | NCH₃ | CH₃ | C(CH₂CH₃) | CHF₂ | OH |
| Ib1.326 | NCH₃ | CH₃ | C(CH₂CH₃) | CF₃ | OH |
| Ib1.327 | NCH₃ | CH₃ | C(CH₂CH₃) | OCH₃ | OH |
| Ib1.328 | NCH₃ | CH₃ | C(CH₂CH₃) | OCH₂CH₃ | OH |
| Ib1.329 | NCH₃ | CH₃ | C(CH₂CH₃) | OCHF₂ | OH |
| Ib1.330 | NCH₃ | CH₃ | C(CH₂CH₃) | OCF₃ | OH |
| Ib1.331 | NCH₃ | CH₂CH₃ | C(CH₂CH₃) | SCH₃ | OH |
| Ib1.332 | NCH₃ | CH₂CH₃ | C(CH₂CH₃) | SCH₂CH₃ | OH |
| Ib1.333 | NCH₃ | CH₂CH₃ | C(CH₂CH₃) | SO₂CH₃ | OH |
| Ib1.334 | NCH₃ | CH₂CH₃ | C(CH₂CH₃) | SO₂CH₂CH₃ | OH |
| Ib1.335 | NCH₃ | CH₂CH₃ | C(CH₂CH₃) | SO₂CH(CH₃)₂ | OH |
| Ib1.336 | NCH₃ | CH₂CH₃ | C(CH₂CH₃) | SO₂(CH₂)₂CH₃ | OH |
| Ib1.337 | NCH₃ | CH₂CH₃ | C(CH₂CH₃) | Cl | OH |
| Ib1.338 | NCH₃ | CH₂CH₃ | C(CH₂CH₃) | Br | OH |
| Ib1.339 | NCH₃ | CH₂CH₃ | C(CH₂CH₃) | NO₂ | OH |
| Ib1.340 | NCH₃ | CH₂CH₃ | C(CH₂CH₃) | CHF₂ | OH |
| Ib1.341 | NCH₃ | CH₂CH₃ | C(CH₂CH₃) | CF₃ | OH |
| Ib1.342 | NCH₃ | CH₂CH₃ | C(CH₂CH₃) | OCH₃ | OH |
| Ib1.343 | NCH₃ | CH₂CH₃ | C(CH₂CH₃) | OCH₂CH₃ | OH |
| Ib1.344 | NCH₃ | CH₂CH₃ | C(CH₂CH₃) | OCHF₂ | OH |
| Ib1.345 | NCH₃ | CH₂CH₃ | C(CH₂CH₃) | OCF₃ | OH |
| Ib1.346 | NCH₃ | CH₂Cl | C(CH₂CH₃) | SCH₃ | OH |
| Ib1.347 | NCH₃ | CH₂Cl | C(CH₂CH₃) | SCH₂CH₃ | OH |
| Ib1.348 | NCH₃ | CH₂Cl | C(CH₂CH₃) | SO₂CH₃ | OH |
| Ib1.349 | NCH₃ | CH₂Cl | C(CH₂CH₃) | SO₂CH₂CH₃ | OH |
| Ib1.350 | NCH₃ | CH₂Cl | C(CH₂CH₃) | SO₂CH(CH₃)₂ | OH |
| Ib1.351 | NCH₃ | CH₂Cl | C(CH₂CH₃) | SO₂(CH₂)₂CH₃ | OH |
| Ib1.352 | NCH₃ | CH₂Cl | C(CH₂CH₃) | Cl | OH |
| Ib1.353 | NCH₃ | CH₂Cl | C(CH₂CH₃) | Br | OH |
| Ib1.354 | NCH₃ | CH₂Cl | C(CH₂CH₃) | NO₂ | OH |
| Ib1.355 | NCH₃ | CH₂Cl | C(CH₂CH₃) | CHF₂ | OH |
| Ib1.356 | NCH₃ | CH₂Cl | C(CH₂CH₃) | CF₃ | OH |
| Ib1.357 | NCH₃ | CH₂Cl | C(CH₂CH₃) | OCH₃ | OH |
| Ib1.358 | NCH₃ | CH₂Cl | C(CH₂CH₃) | OCH₂CH₃ | OH |
| Ib1.359 | NCH₃ | CH₂Cl | C(CH₂CH₃) | OCHF₂ | OH |
| Ib1.360 | NCH₃ | CH₂Cl | C(CH₂CH₃) | OCF₃ | OH |
| Ib1.361 | NCH₃ | H | N | SCH₃ | OH |
| Ib1.362 | NCH₃ | H | N | SCH₂CH₃ | OH |
| Ib1.363 | NCH₃ | H | N | SO₂CH₃ | OH |
| Ib1.364 | NCH₃ | H | N | SO₂CH₂CH₃ | OH |
| Ib1.365 | NCH₃ | H | N | SO₂CH(CH₃)₂ | OH |
| Ib1.366 | NCH₃ | H | N | SO₂(CH₂)₂CH₃ | OH |
| Ib1.367 | NCH₃ | H | N | Cl | OH |
| Ib1.368 | NCH₃ | H | N | Br | OH |
| Ib1.369 | NCH₃ | H | N | NO₂ | OH |
| Ib1.370 | NCH₃ | H | N | CHF₂ | OH |
| Ib1.371 | NCH₃ | H | N | CF₃ | OH |
| Ib1.372 | NCH₃ | H | N | OCH₃ | OH |
| Ib1.373 | NCH₃ | H | N | OCH₂CH₃ | OH |
| Ib1.374 | NCH₃ | H | N | OCHF₂ | OH |
| Ib1.375 | NCH₃ | H | N | OCF₃ | OH |
| Ib1.376 | NCH₃ | CH₃ | N | SCH₃ | OH |
| Ib1.377 | NCH₃ | CH₃ | N | SCH₂CH₃ | OH |
| Ib1.378 | NCH₃ | CH₃ | N | SO₂CH₃ | OH |
| Ib1.379 | NCH₃ | CH₃ | N | SO₂CH₂CH₃ | OH |
| Ib1.380 | NCH₃ | CH₃ | N | SO₂CH(CH₃)₂ | OH |
| Ib1.381 | NCH₃ | CH₃ | N | SO₂(CH₂)₂CH₃ | OH |
| Ib1.382 | NCH₃ | CH₃ | N | Cl | OH |
| Ib1.383 | NCH₃ | CH₃ | N | Br | OH |
| Ib1.384 | NCH₃ | CH₃ | N | NO₂ | OH |
| Ib1.385 | NCH₃ | CH₃ | N | CHF₂ | OH |
| Ib1.386 | NCH₃ | CH₃ | N | CF₃ | OH |
| Ib1.387 | NCH₃ | CH₃ | N | OCH₃ | OH |
| Ib1.388 | NCH₃ | CH₃ | N | OCH₂CH₃ | OH |
| Ib1.389 | NCH₃ | CH₃ | N | OCHF₂ | OH |
| Ib1.390 | NCH₃ | CH₃ | N | OCF₃ | OH |
| Ib1.391 | NCH₃ | CH₂CH₃ | N | SCH₃ | OH |
| Ib1.392 | NCH₃ | CH₂CH₃ | N | SCH₂CH₃ | OH |
| Ib1.393 | NCH₃ | CH₂CH₃ | N | SO₂CH₃ | OH |
| Ib1.394 | NCH₃ | CH₂CH₃ | N | SO₂CH₂CH₃ | OH |
| Ib1.395 | NCH₃ | CH₂CH₃ | N | SO₂CH(CH₃)₂ | OH |
| Ib1.396 | NCH₃ | CH₂CH₃ | N | SO₂(CH₂)₂CH₃ | OH |
| Ib1.397 | NCH₃ | CH₂CH₃ | N | Cl | OH |
| Ib1.398 | NCH₃ | CH₂CH₃ | N | Br | OH |
| Ib1.399 | NCH₃ | CH₂CH₃ | N | NO₂ | OH |
| Ib1.400 | NCH₃ | CH₂CH₃ | N | CHF₂ | OH |
| Ib1.401 | NCH₃ | CH₂CH₃ | N | CF₃ | OH |
| Ib1.402 | NCH₃ | CH₂CH₃ | N | OCH₃ | OH |
| Ib1.403 | NCH₃ | CH₂CH₃ | N | OCH₂CH₃ | OH |
| Ib1.404 | NCH₃ | CH₂CH₃ | N | OCHF₂ | OH |
| Ib1.405 | NCH₃ | CH₂CH₃ | N | OCF₃ | OH |
| Ib1.406 | NCH₃ | CH₂Cl | N | SCH₃ | OH |
| Ib1.407 | NCH₃ | CH₂Cl | N | SCH₂CH₃ | OH |
| Ib1.408 | NCH₃ | CH₂Cl | N | SO₂CH₃ | OH |
| Ib1.409 | NCH₃ | CH₂Cl | N | SO₂CH₂CH₃ | OH |
| Ib1.410 | NCH₃ | CH₂Cl | N | SO₂CH(CH₃)₂ | OH |
| Ib1.411 | NCH₃ | CH₂Cl | N | SO₂(CH₂)₂CH₃ | OH |
| Ib1.412 | NCH₃ | CH₂Cl | N | Cl | OH |
| Ib1.413 | NCH₃ | CH₂Cl | N | Br | OH |
| Ib1.414 | NCH₃ | CH₂Cl | N | NO₂ | OH |
| Ib1.415 | NCH₃ | CH₂Cl | N | CHF₂ | OH |
| Ib1.416 | NCH₃ | CH₂Cl | N | CF₃ | OH |
| Ib1.417 | NCH₃ | CH₂Cl | N | OCH₃ | OH |
| Ib1.418 | NCH₃ | CH₂Cl | N | OCH₂CH₃ | OH |
| Ib1.419 | NCH₃ | CH₂Cl | N | OCHF₂ | OH |
| Ib1.420 | NCH₃ | CH₂Cl | N | OCF₃ | OH |

Ebenso außerordentlichst bevorzugt sind die Verbindungen der Formel Ib2, insbesondere die Verbindungen Ib2.1 bis Ia2.420, die sich von den entsprechenden Verbindungen Ib1.1 bis Ib1.420 dadurch unterscheiden, daß R¹⁴ für Methyl steht.

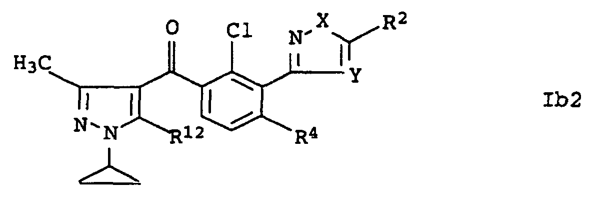

Ebenso außerordentlichst bevorzugt sind die Verbindungen der Formel Ib3, insbesondere die Verbindungen Ib3.1 bis Ib3.420, die sich von den Verbindungen Ib1.1 bis Ib1.420 dadurch unterscheiden, daß R¹³ für Cyclopentyl steht.

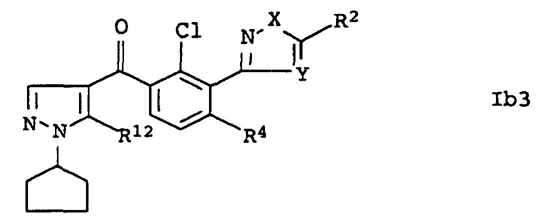

Ebenso außerordentlichst bevorzugt sind die Verbindungen der Formel Ib4, insbesondere die Verbindungen Ib4.1 bis Ib4.420, die sich von den Verbindungen Ia1.1 bis Ib1.420 dadurch unterscheiden, daß R¹³ für Cyclopentyl und R¹⁴ für Methyl stehen.

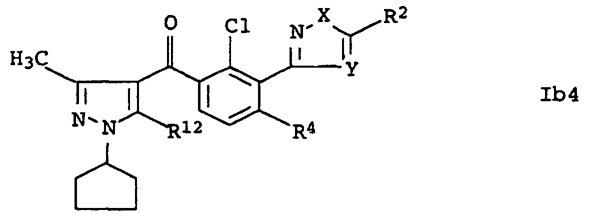

Ebenso außerordentlichst bevorzugt sind die Verbindungen der Formel Ib5, insbesondere die Verbindungen Ib5.1 bis Ib5.420, die sich von den Verbindungen Ib1.1 bis Ib1.420 dadurch unterscheiden, daß R¹³ für Cyclohexyl steht.

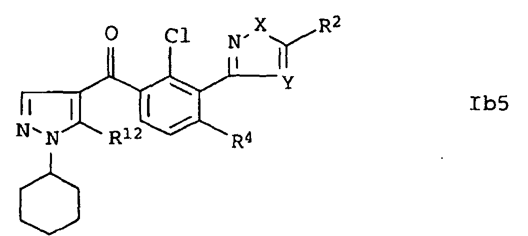

Ebenso außerordentlichst bevorzugt sind die Verbindungen der Formel Ib6, insbesondere die Verbindungen Ib6.1 bis Ib6.420, die sich von den Verbindungen Ib1.1 bis Ib1.420 dadurch unterscheiden, daß R¹³ für Cyclohexyl und R¹⁴ für Methyl stehen.

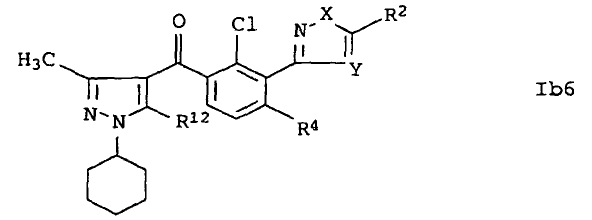

Ebenso außerordentlichst bevorzugt sind die Verbindungen der Formel Ib7, insbesondere die Verbindungen Ib7.1 bis Ib7.420, die sich von den entsprechenden Verbindungen Ib1.1 bis Ib1.420 dadurch unterscheiden, daß R¹ für Methyl steht.

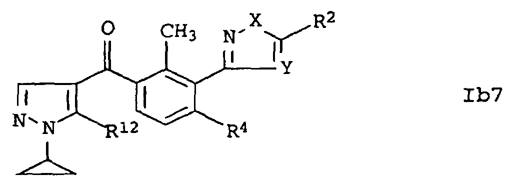

Ebenso außerordentlichst bevorzugt sind die Verbindungen der Formel Ib8, insbesondere die Verbindungen Ib8.1 bis Ib8.420, die sich von den entsprechenden Verbindungen Ib1.1 bis Ib1.420 dadurch unterscheiden, daß R¹ für Methyl und R¹⁴ für Methyl stehen.

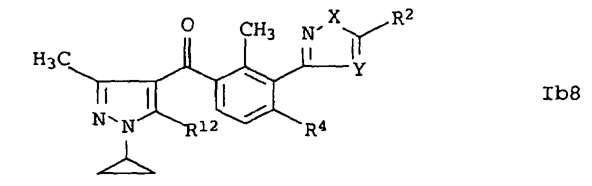

Ebenso außerordentlichst bevorzugt sind die Verbindungen der Formel Ib9, insbesondere die Verbindungen Ib9.1 bis Ib9.420, die sich von den Verbindungen Ib1.1 bis Ib1.420 dadurch unterscheiden, daß R¹ für Methyl und R¹³ für Cyclopentyl stehen.

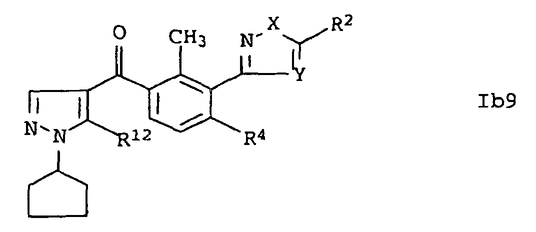

Ebenso außerordentlichst bevorzugt sind die Verbindungen der Formel Ib10, insbesondere die Verbindungen Ib10.1 bis Ib10.420, die sich von den Verbindungen Ib1.1 bis Ib1.420 dadurch unterscheiden, daß R¹ für Methyl, R¹³ für Cyclopentyl und R¹⁴ für Methyl stehen.

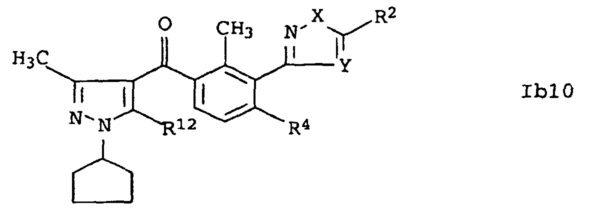

Ebenso außerordentlichst bevorzugt sind die Verbindungen der Formel Ib11, insbesondere die Verbindungen Ib11.1 bis Ib11.420, die sich von den Verbindungen Ib1.1 bis Ib1.420 dadurch unterscheiden, daß R¹ für Methyl und R¹³ für Cyclohexyl stehen.

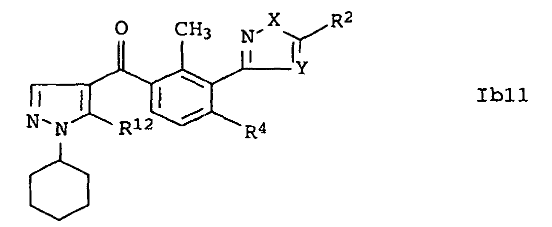

Ebenso außerordentlichst bevorzugt sind die Verbindungen der Formel Ib12, insbesondere die Verbindungen Ib12.1 bis Ib12.420, die sich von den Verbindungen Ib1.1 bis Ib1.420 dadurch unterscheiden, daß R¹ für Methyl R¹³ für Cyclohexyl und R¹⁴ für Methyl stehen.

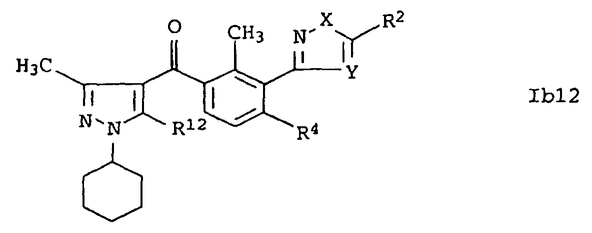

Ebenso außerordentlichst bevorzugt sind die Verbindungen der Formel Ib13, insbesondere die Verbindungen Ib13.1 bis Ib13.420, die sich von den entsprechenden Verbindungen Ib1.1 bis Ib1.420 dadurch unterscheiden, daß R¹ für Methoxy steht.

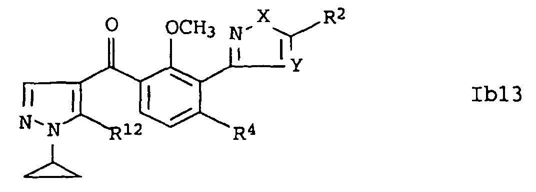

Ebenso außerordentlichst bevorzugt sind die Verbindungen der Formel Ib14, insbesondere die Verbindungen Ib14.1 bis Ib14.420, die sich von den entsprechenden Verbindungen Ib1.1 bis Ib1.900 dadurch unterscheiden, daß R¹ für Methoxy und R¹⁴ für Methyl stehen.

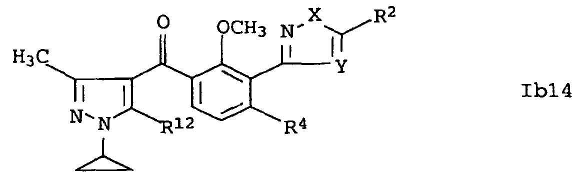

Ebenso außerordentlichst bevorzugt sind die Verbindungen der Formel Ib15, insbesondere die Verbindungen Ib15.1 bis Ib15.420, die sich von den Verbindungen Ib1.1 bis Ib1.420 dadurch unterscheiden, daß R¹ für Methoxy und R¹³ für Cyclopentyl stehen.

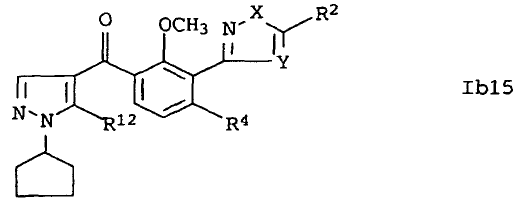

Ebenso außerordentlichst bevorzugt sind die Verbindungen der Formel Ib16, insbesondere die Verbindungen Ib16.1 bis Ib16.420, die sich von den Verbindungen Ib1.1 bis Ib1.420 dadurch unterscheiden, daß R¹ für Methoxy, R¹³ für Cyclopentyl und R¹⁴ für Methyl stehen.

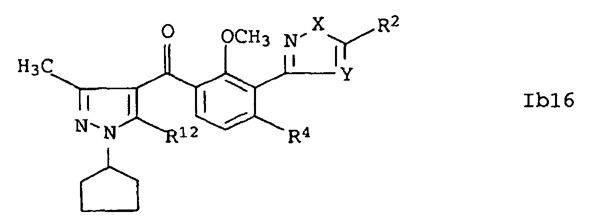

Ebenso außerordentlichst bevorzugt sind die Verbindungen der Formel Ib17, insbesondere die Verbindungen Ib17.1 bis Ib17.420, die sich von den Verbindungen Ib1.1 bis Ib1.420 dadurch unterscheiden, daß R¹ für Methoxy und R¹³ für Cyclohexyl stehen.

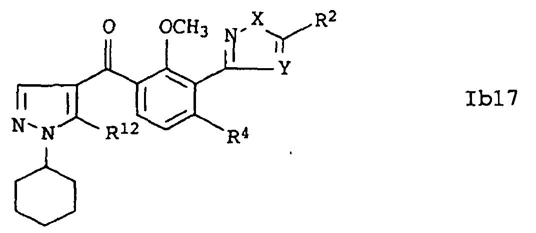

Ebenso außerordentlichst bevorzugt sind die Verbindungen der Formel Ib18, insbesondere die Verbindungen Ib18.1 bis Ib18.420, die sich von den Verbindungen Ib1.1 bis Ib1.420 dadurch unterscheiden, daß R¹ für Methoxy, R¹³ für Cyclohexyl und R¹⁴ für Methyl stehen.

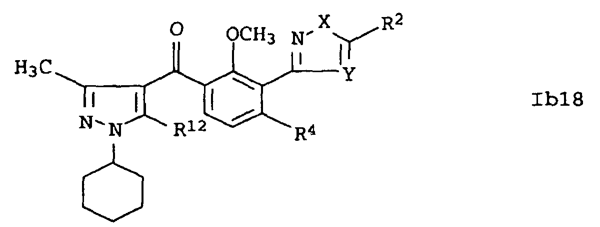

Ebenso außerordentlich bevorzugt sind die Verbindungen der Formel Ic1 (≡ I mit R¹ = Cl, R³ und R⁶ bzw. R³ und R⁸ bilden gemeinsam eine Bindung aus, R¹³ = cyclo-C₃H₅ und R⁵ und R¹⁴ = H), insbesondere die Verbindungen Ic1.1-Ic1.720 der Tabelle 3, wobei die Restedefinitionen nicht nur in Kombination miteinander sondern auch jeweils für sich allein betrachtet für die erfindungsgemäßen Verbindungen eine besondere Bedeutung haben.

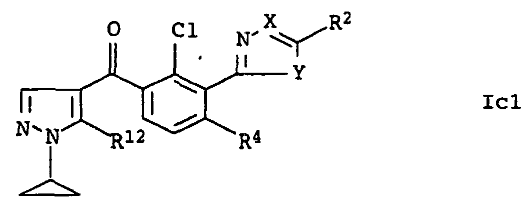

**Tabelle 3:**

| Nr. | X | R² | Y | R⁴ | R⁸ |
|---|---|---|---|---|---|
| Ic1.1 | CH | H | O | SCH₃ | OH |
| Ic1.2 | CH | H | O | SCH₂CH₃ | OH |
| Ic1.3 | CH | H | O | SO₂CH₃ | OH |
| Ic1.4 | CH | H | O | SO₂CH₂CH₃ | OH |
| Ic1.5 | CH | H | O | SO₂CH(CH₃)₂ | OH |
| Ic1.6 | CH | H | O | SO₂(CH₂)₂CH₃ | OH |
| Ic1.7 | CH | H | O | Cl | OH |
| Ic1.8 | CH | H | O | Br | OH |
| Ic1.9 | CH | H | O | NO₂ | OH |
| Ic1.10 | CH | H | O | CHF₂ | OH |
| Ic1.11 | CH | H | O | CF₃ | OH |
| Ic1.12 | CH | H | O | OCH₃ | OH |
| Ic1.13 | CH | H | O | OCH₂CH₃ | OH |
| Ic1.14 | CH | H | O | OCHF₂ | OH |
| Ic1.15 | CH | H | O | OCF₃ | OH |
| Ic1.16 | CH | CH₃ | O | SCH₃ | OH |
| Ic1.17 | CH | CH₃ | O | SCH₂CH₃ | OH |
| Ic1.18 | CH | CH₃ | O | SO₂CH₃ | OH |
| Ic1.19 | CH | CH₃ | O | SO₂CH₂CH₃ | OH |
| Ic1.20 | CH | CH₃ | O | SO₂CH(CH₃)₂ | OH |
| Ic1.21 | CH | CH₃ | O | SC₂(CH₂)₂CH₃ | OH |
| Ic1.22 | CH | CH₃ | O | Cl | OH |
| Ic1.23 | CH | CH₃ | O | Br | OH |
| Ic1.24 | CH | CH₃ | O | NO₂ | OH |
| Ic1.25 | CH | CH₃ | O | CHF₂ | OH |
| Ic1.26 | CH | CH₃ | O | CF₃ | OH |
| Ic1.27 | CH | CH₃ | O | OCH₃ | OH |
| Ic1.28 | CH | CH₃ | O | OCH₂CH₃ | OH |
| Ic1.29 | CH | CH₃ | O | OCHF₂ | OH |
| Ic1.30 | CH | CH₃ | O | OCF₃ | OH |
| Ic1.31 | CH | CH₂CH₃ | O | SCH₃ | OH |
| Ic1.32 | CH | CH₂CH₃ | O | SCH₂CH₃ | OH |
| Ic1.33 | CH | CH₂CH₃ | O | SO₂CH₃ | OH |
| Ic1.34 | CH | CH₂CH₃ | O | SO₂CH₂CH₃ | OH |
| Ic1.35 | CH | CH₂CH₃ | O | SO₂CH(CH₃)₂ | OH |
| Ic1.36 | CH | CH₂CH₃ | O | SO₂(CH₂)₂CH₃ | OH |
| Ic1.37 | CH | CH₂CH₃ | O | Cl | OH |
| Ic1.38 | CH | CH₂CH₃ | O | Br | OH |
| Ic1.39 | CH | CH₂CH₃ | O | NO₂ | OH |
| Ic1.40 | CH | CH₂CH₃ | O | CHF₂ | OH |
| Ic1.41 | CH | CH₂CH₃ | O | CF₃ | OH |
| Ic1.42 | CH | CH₂CH₃ | O | OCH₃ | OH |
| Ic1.43 | CH | CH₂CH₃ | O | OCH₂CH₃ | OH |
| Ic1.44 | CH | CH₂CH₃ | O | OCHF₂ | OH |
| Ic1.45 | CH | CH₂CH₃ | O | OCF₃ | OH |
| Ic1.46 | CH | CH₂Cl | O | SCH₃ | OH |
| Ic1.47 | CH | CH₂Cl | O | SCH₂CH₃ | OH |
| Ic1.48 | CH | CH₂Cl | O | SO₂CH₃ | OH |
| Ic1.49 | CH | CH₂Cl | O | SO₂CH₂CH₃ | OH |
| Ic1.50 | CH | CH₂Cl | O | SO₂CH(CH₃)₂ | OH |
| Ic1.51 | CH | CH₂Cl | O | SO₂(CH₂)₂CH₃ | OH |
| Ic1.52 | CH | CH₂Cl | O | Cl | OH |
| Ic1.53 | CH | CH₂Cl | O | Br | OH |
| Ic1.54 | CH | CH₂Cl | O | NO₂ | OH |
| Ic1.55 | CH | CH₂Cl | O | CHF₂ | OH |
| Ic1.56 | CH | CH₂Cl | O | CF₃ | OH |
| Ic1.57 | CH | CH₂Cl | O | OCH₃ | OH |
| Ic1.58 | CH | CH₂Cl | O | OCH₂CH₃ | OH |
| Ic1.59 | CH | CH₂Cl | O | OCHF₂ | OH |
| Ic1.60 | CH | CH₂Cl | O | OCF₃ | OH |
| Ic1.61 | CH | H | S | SCH₃ | OH |
| Ic1.62 | CH | H | S | SCH₂CH₃ | OH |
| Ic1.63 | CH | H | S | SO₂CH₃ | OH |
| Ic1.64 | CH | H | S | SO₂CH₂CH₃ | OH |
| Ic1.65 | CH | H | S | SO₂CH(CH₃)₂ | OH |
| Ic1.66 | CH | H | S | SO₂(CH₂)₂CH₃ | OH |
| Ic1.67 | CH | H | S | Cl | OH |
| Ic1.68 | CH | H | S | Br | OH |
| Ic1.69 | CH | H | S | NO₂ | OH |
| Ic1.70 | CH | H | S | CHF₂ | OH |
| Ic1.71 | CH | H | S | CF₃ | OH |
| Ic1.72 | CH | H | S | OCH₃ | OH |
| Ic1.73 | CH | H | S | OCH₂CH₃ | OH |
| Ic1.74 | CH | H | S | OCHF₂ | OH |
| Ic1.75 | CH | H | S | OCF₃ | OH |
| Ic1.76 | CH | CH₃ | S | SCH₃ | OH |
| Ic1.77 | CH | CH₃ | S | SCH₂CH₃ | OH |
| Ic1.78 | CH | CH₃ | S | SO₂CH₃ | OH |
| Ic1.79 | CH | CH₃ | S | SO₂CH₂CH₃ | OH |
| Ic1.80 | CH | CH₃ | S | SO₂CH(CH₃)₂ | OH |
| Ic1.81 | CH | CH₃ | S | SO₂(CH₂)₂CH₃ | OH |
| Ic1.82 | CH | CH₃ | S | Cl | OH |
| Ic1.83 | CH | CH₃ | S | Br | OH |
| Ic1.84 | CH | CH₃ | S | NO₂ | OH |
| Ic1.85 | CH | CH₃ | S | CHF₂ | OH |
| Ic1.86 | CH | CH₃ | S | CF₃ | OH |
| Ic1.87 | CH | CH₃ | S | OCH₃ | OH |
| Ic1.88 | CH | CH₃ | S | OCH₂CH₃ | OH |
| Ic1.89 | CH | CH₃ | S | OCHF₂ | OH |
| Ic1.90 | CH | CH₃ | S | OCF₃ | OH |
| Ic1.91 | CH | CH₂CH₃ | S | SCH₃ | OH |
| Ic1.92 | CH | CH₂CH₃ | S | SCH₂CH₃ | OH |
| Ic1.93 | CH | CH₂CH₃ | S | SO₂CH₃ | OH |
| Ic1.94 | CH | CH₂CH₃ | S | SO₂CH₂CH₃ | OH |
| Ic1.95 | CH | CH₂CH₃ | S | SO₂CH(CH₃)₂ | OH |
| Ic1.96 | CH | CH₂CH₃ | S | SO₂(CH₂)₂CH₃ | OH |
| Ic1.97 | CH | CH₂CH₃ | S | Cl | OH |
| Ic1.98 | CH | CH₂CH₃ | S | Br | OH |
| Ic1.99 | CH | CH₂CH₃ | S | NO₂ | OH |
| Ic1.100 | CH | CH₂CH₃ | S | CHF₂ | OH |
| Ic1.101 | CH | CH₂CH₃ | S | CF₃ | OH |
| Ic1.102 | CH | CH₂CH₃ | S | OCH₃ | OH |
| Ic1.103 | CH | CH₂CH₃ | S | OCH₂CH₃ | OH |
| Ic1.104 | CH | CH₂CH₃ | S | OCHF₂ | OH |
| Ic1.105 | CH | CH₂CH₃ | S | OCF₃ | OH |
| Ic1.106 | CH | CH₂Cl | S | SCH₃ | OH |
| Ic1.107 | CH | CH₂Cl | S | SCH₂CH₃ | OH |
| Ic1.108 | CH | CH₂Cl | S | SO₂CH₃ | OH |
| Ic1.109 | CH | CH₂Cl | S | SO₂CH₂CH₃ | OH |
| Ic1.110 | CH | CH₂Cl | S | SO₂CH(CH₃)₂ | OH |
| Ic1.111 | CH | CH₂Cl | S | SO₂(CH₂)₂CH₃ | OH |
| Ic1.112 | CH | CH₂Cl | S | Cl | OH |
| Ic1.113 | CH | CH₂Cl | S | Br | OH |
| Ic1.114 | CH | CH₂Cl | S | NO₂ | OH |
| Ic1.115 | CH | CH₂Cl | S | CHF₂ | OH |
| Ic1.116 | CH | CH₂Cl | S | CF₃ | OH |
| Ic1.117 | CH | CH₂Cl | S | OCH₃ | OH |
| Ic1.118 | CH | CH₂Cl | S | OCH₂CH₃ | OH |
| Ic1.119 | CH | CH₂Cl | S | OCHF₂ | OH |
| Ic1.120 | CH | CH₂Cl | S | OCF₃ | OH |
| Ic1.121 | CH | H | NCH₃ | SCH₃ | OH |
| Ic1.122 | CH | H | NCH₃ | SCH₂CH₃ | OH |
| Ic1.123 | CH | H | NCH₃ | SO₂CH₃ | OH |
| Ic1.124 | CH | H | NCH₃ | SO₂CH₂CH₃ | OH |
| Ic1.125 | CH | H | NCH₃ | SO₂CH(CH₃)₂ | OH |
| Ic1.126 | CH | H | NCH₃ | SO₂(CH₂)₂CH₃ | OH |
| Ic1.127 | CH | H | NCH₃ | Cl | OH |
| Ic1.128 | CH | H | NCH₃ | Br | OH |
| Ic1.129 | CH | H | NCH₃ | NO₂ | OH |
| Ic1.130 | CH | H | NCH₃ | CHF₂ | OH |
| Ic1.131 | CH | H | NCH₃ | CF₃ | OH |
| Ic1.132 | CH | H | NCH₃ | OCH₃ | OH |
| Ic1.133 | CH | H | NCH₃ | OCH₂CH₃ | OH |
| Ic1.134 | CH | H | NCH₃ | OCHF₂ | OH |
| Ic1.135 | CH | H | NCH₃ | OCF₃ | OH |
| Ic1.136 | CH | CH₃ | NCH₃ | SCH₃ | OH |
| Ic1.137 | CH | CH₃ | NCH₃ | SCH₂CH₃ | OH |
| Ic1.138 | CH | CH₃ | NCH₃ | SO₂CH₃ | OH |
| Ic1.139 | CH | CH₃ | NCH₃ | SO₂CH₂CH₃ | OH |
| Ic1.140 | CH | CH₃ | NCH₃ | SO₂CH(CH₃)₂ | OH |
| Ic1.141 | CH | CH₃ | NCH₃ | SO₂(CH₂)₂CH₃ | OH |
| Ic1.142 | CH | CH₃ | NCH₃ | Cl | OH |
| Ic1.143 | CH | CH₃ | NCH₃ | Br | OH |
| Ic1.144 | CH | CH₃ | NCH₃ | NO₂ | OH |
| Ic1.145 | CH | CH₃ | NCH₃ | CHF₂ | OH |
| Ic1.146 | CH | CH₃ | NCH₃ | CF₃ | OH |
| Ic1.147 | CH | CH₃ | NCH₃ | OCH₃ | OH |
| Ic1.148 | CH | CH₃ | NCH₃ | OCH₂CH₃ | OH |
| Ic1.149 | CH | CH₃ | NCH₃ | OCHF₂ | OH |
| Ic1.150 | CH | CH₃ | NCH₃ | OCF₃ | OH |
| Ic1.151 | CH | CH₂CH₃ | NCH₃ | SCH₃ | OH |
| Ic1.152 | CH | CH₂CH₃ | NCH₃ | SCH₂CH₃ | OH |
| Ic1.153 | CH | CH₂CH₃ | NCH₃ | SO₂CH₃ | OH |
| Ic1.154 | CH | CH₂CH₃ | NCH₃ | SO₂CH₂CH₃ | OH |
| Ic1.155 | CH | CH₂CH₃ | NCH₃ | SO₂CH(CH₃)₂ | OH |
| Ic1.156 | CH | CH₂CH₃ | NCH₃ | SO₂(CH₂)₂CH₃ | OH |
| Ic1.157 | CH | CH₂CH₃ | NCH₃ | Cl | OH |
| Ic1.158 | CH | CH₂CH₃ | NCH₃ | Br | OH |
| Ic1.159 | CH | CH₂CH₃ | NCH₃ | NO₂ | OH |
| Ic1.160 | CH | CH₂CH₃ | NCH₃ | CHF₂ | OH |
| Ic1.161 | CH | CH₂CH₃ | NCH₃ | CF₃ | OH |
| Ic1.162 | CH | CH₂CH₃ | NCH₃ | OCH₃ | OH |
| Ic1.163 | CH | CH₂CH₃ | NCH₃ | OCH₂CH₃ | OH |
| Ic1.164 | CH | CH₂CH₃ | NCH₃ | OCHF₂ | OH |
| Ic1.165 | CH | CH₂CH₃ | NCH₃ | OCF₃ | OH |
| Ic1.166 | CH | CH₂Cl | NCH₃ | SCH₃ | OH |
| Ic1.167 | CH | CH₂Cl | NCH₃ | SCH₂CH₃ | OH |
| Ic1.168 | CH | CH₂Cl | NCH₃ | SO₂CH₃ | OH |
| Ic1.169 | CH | CH₂Cl | NCH₃ | SO₂CH₂CH₃ | OH |
| Ic1.170 | CH | CH₂Cl | NCH₃ | SO₂CH(CH₃)₂ | OH |
| Ic1.171 | CH | CH₂Cl | NCH₃ | SO₂(CH₂)₂CH₃ | OH |
| Ic1.172 | CH | CH₂Cl | NCH₃ | Cl | OH |
| Ic1.173 | CH | CH₂Cl | NCH₃ | Br | OH |
| Ic1.174 | CH | CH₂Cl | NCH₃ | NO₂ | OH |
| Ic1.175 | CH | CH₂Cl | NCH₃ | CHF₂ | OH |
| Ic1.176 | CH | CH₂Cl | NCH₃ | CF₃ | OH |
| Ic1.177 | CH | CH₂Cl | NCH₃ | OCH₃ | OH |
| Ic1.178 | CH | CH₂Cl | NCH₃ | OCH₂CH₃ | OH |
| Ic1.179 | CH | CH₂Cl | NCH₃ | OCHF₂ | OH |
| Ic1.180 | CH | CH₂Cl | NCH₃ | OCF₃ | OH |
| Ic1.181 | C(CH₃) | H | O | SCH₃ | OH |
| Ic1.182 | C(CH₃) | H | O | SCH₂CH₃ | OH |
| Ic1.183 | C(CH₃) | H | O | SO₂CH₃ | OH |
| Ic1.184 | C(CH₃) | H | O | SO₂CH₂CH₃ | OH |
| Ic1.185 | C(CH₃) | H | O | SO₂CH(CH₃)₂ | OH |
| Ic1.186 | C(CH₃) | H | O | SO₂(CH₂)₂CH₃ | OH |
| Ic1.187 | C(CH₃) | H | O | Cl | OH |
| Ic1.188 | C(CH₃) | H | O | Br | OH |
| Ic1.189 | C(CH₃) | H | O | NO₂ | OH |
| Ic1.190 | C(CH₃) | H | O | CHF₂ | OH |
| Ic1.191 | C(CH₃) | H | O | CF₃ | OH |
| Ic1.192 | C(CH₃) | H | O | OCH₃ | OH |
| Ic1.193 | C(CH₃) | H | O | OCH₂CH₃ | OH |
| Ic1.194 | C(CH₃) | H | O | OCHF₂ | OH |
| Ic1.195 | C(CH₃) | H | O | OCF₃ | OH |
| Ic1.196 | C(CH₃) | CH₃ | O | SCH₃ | OH |
| Ic1.197 | C(CH₃) | CH₃ | O | SCH₂CH₃ | OH |
| Ic1.198 | C(CH₃) | CH₃ | O | SO₂CH₃ | OH |
| Ic1.199 | C(CH₃) | CH₃ | O | SO₂CH₂CH₃ | OH |
| Ic1.200 | C(CH₃) | CH₃ | O | SO₂CH(CH₃)₂ | OH |
| Ic1.201 | C(CH₃) | CH₃ | O | SO₂(CH₂)₂CH₃ | OH |
| Ic1.202 | C(CH₃) | CH₃ | O | Cl | OH |
| Ic1.203 | C(CH₃) | CH₃ | O | Br | OH |
| Ic1.204 | C(CH₃) | CH₃ | O | NO₂ | OH |
| Ic1.205 | C(CH₃) | CH₃ | O | CHF₂ | OH |
| Ic1.206 | C(CH₃) | CH₃ | O | CF₃ | OH |
| Ic1.207 | C(CH₃) | CH₃ | O | OCH₃ | OH |
| Ic1.208 | C(CH₃) | CH₃ | O | OCH₂CH₃ | OH |
| Ic1.209 | C(CH₃) | CH₃ | O | OCHF₂ | OH |
| Ic1.210 | C(CH₃) | CH₃ | O | OCF₃ | OH |
| Ic1.211 | C(CH₃) | CH₂CH₃ | O | SCH₃ | OH |
| Ic1.212 | C(CH₃) | CH₂CH₃ | O | SCH₂CH₃ | OH |
| Ic1.213 | C(CH₃) | CH₂CH₃ | O | SO₂CH₃ | OH |
| Ic1.214 | C(CH₃) | CH₂CH₃ | O | SO₂CH₂CH₃ | OH |
| Ic1.215 | C(CH₃) | CH₂CH₃ | O | SO₂CH(CH₃)₂ | OH |
| Ic1.216 | C(CH₃) | CH₂CH₃ | O | SO₂(CH₂)₂CH₃ | OH |
| Ic1.217 | C(CH₃) | CH₂CH₃ | O | Cl | OH |
| Ic1.218 | C(CH₃) | CH₂CH₃ | O | Br | OH |
| Ic1.219 | C(CH₃) | CH₂CH₃ | O | NO₂ | OH |
| Ic1.220 | C(CH₃) | CH₂CH₃ | O | CHF₂ | OH |
| Ic1.221 | C(CH₃) | CH₂CH₃ | O | CF₃ | OH |
| Ic1.222 | C(CH₃) | CH₂CH₃ | O | OCH₃ | OH |
| Ic1.223 | C(CH₃) | CH₂CH₃ | O | OCH₂CH₃ | OH |
| Ic1.224 | C(CH₃) | CH₂CH₃ | O | OCHF₂ | OH |
| Ic1.225 | C(CH₃) | CH₂CH₃ | O | OCF₃ | OH |
| Ic1.226 | C(CH₃) | CH₂Cl | O | SCH₃ | OH |
| Ic1.227 | C(CH₃) | CH₂Cl | O | SCH₂CH₃ | OH |
| Ic1.228 | C(CH₃) | CH₂Cl | O | SO₂CH₃ | OH |
| Ic1.229 | C(CH₃) | CH₂Cl | O | SO₂CH₂CH₃ | OH |
| Ic1.230 | C(CH₃) | CH₂Cl | O | SO₂CH(CH₃)₂ | OH |
| Ic1.231 | C(CH₃) | CH₂Cl | O | SO₂(CH₂)₂CH₃ | OH |
| Ic1.232 | C(CH₃) | CH₂Cl | O | Cl | OH |
| Ic1.233 | C(CH₃) | CH₂Cl | O | Br | OH |
| Ic1.234 | C(CH₃) | CH₂Cl | O | NO₂ | OH |
| Ic1.235 | C(CH₃) | CH₂Cl | O | CHF₂ | OH |
| Ic1.236 | C(CH₃) | CH₂Cl | O | CF₃ | OH |
| Ic1.237 | C(CH₃) | CH₂Cl | O | OCH₃ | OH |
| Ic1.238 | C(CH₃) | CH₂Cl | O | OCH₂CH₃ | OH |
| Ic1.239 | C(CH₃) | CH₂Cl | O | OCHF₂ | OH |
| Ic1.240 | C(CH₃) | CH₂Cl | O | OCF₃ | OH |
| Ic1.241 | C(CH₃) | H | S | SCH₃ | OH |
| Ic1.242 | C(CH₃) | H | S | SCH₂CH₃ | OH |
| Ic1.243 | C(CH₃) | H | S | SO₂CH₃ | OH |
| Ic1.244 | C(CH₃) | H | S | SO₂CH₂CH₃ | OH |
| Ic1.245 | C(CH₃) | H | S | SO₂CH(CH₃)₂ | OH |
| Ic1.246 | C(CH₃) | H | S | SO₂(CH₂)₂CH₃ | OH |
| Ic1.247 | C(CH₃) | H | S | Cl | OH |
| Ic1.248 | C(CH₃) | H | S | Br | OH |
| Ic1.249 | C(CH₃) | H | S | NO₂ | OH |
| Ic1.250 | C(CH₃) | H | S | CHF₂ | OH |
| Ic1.251 | C(CH₃) | H | S | CF₃ | OH |
| Ic1.252 | C(CH₃) | H | S | OCH₃ | OH |
| Ic1.253 | C(CH₃) | H | S | OCH₂CH₃ | OH |
| Ic1.254 | C(CH₃) | H | S | OCHF₂ | OH |
| Ic1.255 | C(CH₃) | H | S | OCF₃ | OH |
| Ic1.256 | C(CH₃) | CH₃ | S | SCH₃ | OH |
| Ic1.257 | C(CH₃) | CH₃ | S | SCH₂CH₃ | OH |
| Ic1.258 | C(CH₃) | CH₃ | S | SO₂CH₃ | OH |
| Ic1.259 | C(CH₃) | CH₃ | S | SO₂CH₂CH₃ | OH |
| Ic1.260 | C(CH₃) | CH₃ | S | SO₂CH(CH₃)₂ | OH |
| Ic1.261 | C(CH₃) | CH₃ | S | SO₂(CH₂)₂CH₃ | OH |
| Ic1.262 | C(CH₃) | CH₃ | S | Cl | OH |
| Ic1.263 | C(CH₃) | CH₃ | S | Br | OH |
| Ic1.264 | C(CH₃) | CH₃ | S | NO₂ | OH |
| Ic1.265 | C(CH₃) | CH₃ | S | CHF₂ | OH |
| Ic1.266 | C(CH₃) | CH₃ | S | CF₃ | OH |
| Ic1.267 | C(CH₃) | CH₃ | S | OCH₃ | OH |
| Ic1.268 | C(CH₃) | CH₃ | S | OCH₂CH₃ | OH |
| Ic1.269 | C(CH₃) | CH₃ | S | OCHF₂ | OH |
| Ic1.270 | C(CH₃) | CH₃ | S | OCF₃ | OH |
| Ic1.271 | C(CH₃) | CH₂CH₃ | S | SCH₃ | OH |
| Ic1.272 | C(CH₃) | CH₂CH₃ | S | SCH₂CH₃ | OH |
| Ic1.273 | C(CH₃) | CH₂CH₃ | S | SO₂CH₃ | OH |
| Ic1.274 | C(CH₃) | CH₂CH₃ | S | SO₂CH₂CH₃ | OH |
| Ic1.275 | C(CH₃) | CH₂CH₃ | S | SO₂CH(CH₃)₂ | OH |
| Ic1.276 | C(CH₃) | CH₂CH₃ | S | SO₂(CH₂)₂CH₃ | OH |
| Ic1.277 | C(CH₃) | CH₂CH₃ | S | Cl | OH |
| Ic1.278 | C(CH₃) | CH₂CH₃ | S | Br | OH |
| Ic1.279 | C(CH₃) | CH₂CH₃ | S | NO₂ | OH |
| Ic1.280 | C(CH₃) | CH₂CH₃ | S | CHF₂ | OH |
| Ic1.281 | C(CH₃) | CH₂CH₃ | S | CF₃ | OH |
| Ic1.282 | C(CH₃) | CH₂CH₃ | S | OCH₃ | OH |
| Ic1.283 | C(CH₃) | CH₂CH₃ | S | OCH₂CH₃ | OH |
| Ic1.284 | C(CH₃) | CH₂CH₃ | S | OCHF₂ | OH |
| Ic1.285 | C(CH₃) | CH₂CH₃ | S | OCF₃ | OH |
| Ic1.286 | C(CH₃) | CH₂Cl | S | SCH₃ | OH |
| Ic1.287 | C(CH₃) | CH₂Cl | S | SCH₂CH₃ | OH |
| Ic1.288 | C(CH₃) | CH₂Cl | S | SO₂CH₃ | OH |
| Ic1.289 | C(CH₃) | CH₂Cl | S | SO₂CH₂CH₃ | OH |
| Ic1.290 | C(CH₃) | CH₂Cl | S | SO₂CH(CH₃)₂ | OH |
| Ic1.291 | C(CH₃) | CH₂Cl | S | SO₂(CH₂)₂CH₃ | OH |
| Ic1.292 | C(CH₃) | CH₂Cl | S | Cl | OH |
| Ic1.293 | C(CH₃) | CH₂Cl | S | Br | OH |
| Ic1.294 | C(CH₃) | CH₂Cl | S | NO₂ | OH |
| Ic1.295 | C(CH₃) | CH₂Cl | S | CHF₂ | OH |
| Ic1.296 | C(CH₃) | CH₂Cl | S | CF₃ | OH |
| Ic1.297 | C(CH₃) | CH₂Cl | S | OCH₃ | OH |
| Ic1.298 | C(CH₃) | CH₂Cl | S | OCH₂CH₃ | OH |
| Ic1.299 | C(CH₃) | CH₂Cl | S | OCHF₂ | OH |
| Ic1.300 | C(CH₃) | CH₂Cl | S | OCF₃ | OH |
| Ic1.301 | C(CH₃) | H | NCH₃ | SCH₃ | OH |
| Ic1.302 | C(CH₃) | H | NCH₃ | SCH₂CH₃ | OH |
| Ic1.303 | C(CH₃) | H | NCH₃ | SO₂CH₃ | OH |
| Ic1.304 | C(CH₃) | H | NCH₃ | SO₂CH₂CH₃ | OH |
| Ic1.305 | C(CH₃) | H | NCH₃ | SO₂CH(CH₃)₂ | OH |
| Ic1.306 | C(CH₃) | H | NCH₃ | SO₂(CH₂)₂CH₃ | OH |
| Ic1.307 | C(CH₃) | H | NCH₃ | Cl | OH |
| Ic1.308 | C(CH₃) | H | NCH₃ | Br | OH |
| Ic1.309 | C(CH₃) | H | NCH₃ | NO₂ | OH |
| Ic1.310 | C(CH₃) | H | NCH₃ | CHF₂ | OH |
| Ic1.311 | C(CH₃) | H | NCH₃ | CF₃ | OH |
| Ic1.312 | C(CH₃) | H | NCH₃ | OCH₃ | OH |
| Ic1.313 | C(CH₃) | H | NCH₃ | OCH₂CH₃ | OH |
| Ic1.314 | C(CH₃) | H | NCH₃ | OCHF₂ | OH |
| Ic1.315 | C(CH₃) | H | NCH₃ | OCF₃ | OH |
| Ic1.316 | C(CH₃) | CH₃ | NCH₃ | SCH₃ | OH |
| Ic1.317 | C(CH₃) | CH₃ | NCH₃ | SCH₂CH₃ | OH |
| Ic1.318 | C(CH₃) | CH₃ | NCH₃ | SO₂CH₃ | OH |
| Ic1.319 | C(CH₃) | CH₃ | NCH₃ | SO₂CH₂CH₃ | OH |
| Ic1.320 | C(CH₃) | CH₃ | NCH₃ | SO₂CH(CH₃)₂ | OH |
| Ic1.321 | C(CH₃) | CH₃ | NCH₃ | SO₂(CH₂)₂CH₃ | OH |
| Ic1.322 | C(CH₃) | CH₃ | NCH₃ | Cl | OH |
| Ic1.323 | C(CH₃) | CH₃ | NCH₃ | Br | OH |
| Ic1.324 | C(CH₃) | CH₃ | NCH₃ | NO₂ | OH |
| Ic1.325 | C(CH₃) | CH₃ | NCH₃ | CHF₂ | OH |
| Ic1.326 | C(CH₃) | CH₃ | NCH₃ | CF₃ | OH |
| Ic1.327 | C(CH₃) | CH₃ | NCH₃ | OCH₃ | OH |
| Ic1.328 | C(CH₃) | CH₃ | NCH₃ | OCH₂CH₃ | OH |
| Ic1.329 | C(CH₃) | CH₃ | NCH₃ | OCHF₂ | OH |
| Ic1.330 | C(CH₃) | CH₃ | NCH₃ | OCF₃ | OH |
| Ic1.331 | C(CH₃) | CH₂CH₃ | NCH₃ | SCH₃ | OH |
| Ic1.332 | C(CH₃) | CH₂CH₃ | NCH₃ | SCH₂CH₃ | OH |
| Ic1.333 | C(CH₃) | CH₂CH₃ | NCH₃ | SO₂CH₃ | OH |
| Ic1.334 | C(CH₃) | CH₂CH₃ | NCH₃ | SO₂CH₂CH₃ | OH |
| Ic1.335 | C(CH₃) | CH₂CH₃ | NCH₃ | SO₂CH(CH₃)₂ | OH |
| Ic1.336 | C(CH₃) | CH₂CH₃ | NCH₃ | SO₂(CH₂)₂CH₃ | OH |
| Ic1.337 | C(CH₃) | CH₂CH₃ | NCH₃ | Cl | OH |
| Ic1.338 | C(CH₃) | CH₂CH₃ | NCH₃ | Br | OH |
| Ic1.339 | C(CH₃) | CH₂CH₃ | NCH₃ | NO₂ | OH |
| Ic1.340 | C(CH₃) | CH₂CH₃ | NCH₃ | CHF₂ | OH |
| Ic1.341 | C(CH₃) | CH₂CH₃ | NCH₃ | CF₃ | OH |
| Ic1.342 | C(CH₃) | CH₂CH₃ | NCH₃ | OCH₃ | OH |
| Ic1.343 | C(CH₃) | CH₂CH₃ | NCH₃ | OCH₂CH₃ | OH |
| Ic1.344 | C(CH₃) | CH₂CH₃ | NCH₃ | OCHF₂ | OH |
| Ic1.345 | C(CH₃) | CH₂CH₃ | NCH₃ | OCF₃ | OH |
| Ic1.346 | C(CH₃) | CH₂Cl | NCH₃ | SCH₃ | OH |
| Ic1.347 | C(CH₃) | CH₂Cl | NCH₃ | SCH₂CH₃ | OH |
| Ic1.348 | C(CH₃) | CH₂Cl | NCH₃ | SO₂CH₃ | OH |
| Ic1.349 | C(CH₃) | CH₂Cl | NCH₃ | SO₂CH₂CH₃ | OH |
| Ic1.350 | C(CH₃) | CH₂Cl | NCH₃ | SO₂CH(CH₃)₂ | OH |
| Ic1.351 | C(CH₃) | CH₂Cl | NCH₃ | SO₂(CH₂)₂CH₃ | OH |
| Ic1.352 | C(CH₃) | CH₂Cl | NCH₃ | Cl | OH |
| Ic1.353 | C(CH₃) | CH₂Cl | NCH₃ | Br | OH |
| Ic1.354 | C(CH₃) | CH₂Cl | NCH₃ | NO₂ | OH |
| Ic1.355 | C(CH₃) | CH₂Cl | NCH₃ | CHF₂ | OH |
| Ic1.356 | C(CH₃) | CH₂Cl | NCH₃ | CF₃ | OH |
| Ic1.357 | C(CH₃) | CH₂Cl | NCH₃ | OCH₃ | OH |
| Ic1.358 | C(CH₃) | CH₂Cl | NCH₃ | OCH₂CH₃ | OH |
| Ic1.359 | C(CH₃) | CH₂Cl | NCH₃ | OCHF₂ | OH |
| Ic1.360 | C(CH₃) | CH₂Cl | NCH₃ | OCF₃ | OH |
| Ic1.361 | C(CH₂CH₃) | H | O | SCH₃ | OH |
| Ic1.362 | C(CH₂CH₃) | H | O | SCH₂CH₃ | OH |
| Ic1.363 | C(CH₂CH₃) | H | O | SO₂CH₃ | OH |
| Ic1.364 | C(CH₂CH₃) | H | O | SO₂CH₂CH₃ | OH |
| Ic1.365 | C(CH₂CH₃) | H | O | SO₂CH(CH₃)₂ | OH |
| Ic1.366 | C(CH₂CH₃) | H | O | SO₂(CH₂)₂CH₃ | OH |
| Ic1.367 | C(CH₂CH₃) | H | O | Cl | OH |
| Ic1.368 | C(CH₂CH₃) | H | O | Br | OH |
| Ic1.369 | C(CH₂CH₃) | H | O | NO₂ | OH |
| Ic1.370 | C(CH₂CH₃) | H | O | CHF₂ | OH |
| Ic1.371 | C(CH₂CH₃) | H | O | CF₃ | OH |
| Ic1.372 | C(CH₂CH₃) | H | O | OCH₃ | OH |
| Ic1.373 | C(CH₂CH₃) | H | O | OCH₂CH₃ | OH |
| Ic1.374 | C(CH₂CH₃) | H | O | OCHF₂ | OH |
| Ic1.375 | C(CH₂CH₃) | H | O | OCF₃ | OH |
| Ic1.376 | C(CH₂CH₃) | CH₃ | O | SCH₃ | OH |
| Ic1.377 | C(CH₂CH₃) | CH₃ | O | SCH₂CH₃ | OH |
| Ic1.378 | C(CH₂CH₃) | CH₃ | O | SO₂CH₃ | OH |
| Ic1.379 | C(CH₂CH₃) | CH₃ | O | SO₂CH₂CH₃ | OH |
| Ic1.380 | C(CH₂CH₃) | CH₃ | O | SO₂CH(CH₃)₂ | OH |
| Ic1.381 | C(CH₂CH₃) | CH₃ | O | SO₂(CH₂)₂CH₃ | OH |
| Ic1.382 | C(CH₂CH₃) | CH₃ | O | Cl | OH |
| Ic1.383 | C(CH₂CH₃) | CH₃ | O | Br | OH |
| Ic1.384 | C(CH₂CH₃) | CH₃ | O | NO₂ | OH |
| Ic1.385 | C(CH₂CH₃) | CH₃ | O | CHF₂ | OH |
| Ic1.386 | C(CH₂CH₃) | CH₃ | O | CF₃ | OH |
| Ic1.387 | C(CH₂CH₃) | CH₃ | O | OCH₃ | OH |
| Ic1.388 | C(CH₂CH₃) | CH₃ | O | OCH₂CH₃ | OH |
| Ic1.389 | C(CH₂CH₃) | CH₃ | O | OCHF₂ | OH |
| Ic1.390 | C(CH₂CH₃) | CH₃ | O | OCF₃ | OH |
| Ic1.391 | C(CH₂CH₃) | CH₂CH₃ | O | SCH₃ | OH |
| Ic1.392 | C(CH₂CH₃) | CH₂CH₃ | O | SCH₂CH₃ | OH |
| Ic1.393 | C(CH₂CH₃) | CH₂CH₃ | O | SO₂CH₃ | OH |
| Ic1.394 | C(CH₂CH₃) | CH₂CH₃ | O | SO₂CH₂CH₃ | OH |
| Ic1.395 | C(CH₂CH₃) | CH₂CH₃ | O | SO₂CH(CH₃)₂ | OH |
| Ic1.396 | C(CH₂CH₃) | CH₂CH₃ | O | SO₂(CH₂)₂CH₃ | OH |
| Ic1.397 | C(CH₂CH₃) | CH₂CH₃ | O | Cl | OH |
| Ic1.398 | C(CH₂CH₃) | CH₂CH₃ | O | Br | OH |
| Ic1.399 | C(CH₂CH₃) | CH₂CH₃ | O | NO₂ | OH |
| Ic1.400 | C(CH₂CH₃) | CH₂CH₃ | O | CHF₂ | OH |
| Ic1.401 | C(CH₂CH₃) | CH₂CH₃ | O | CF₃ | OH |
| Ic1.402 | C(CH₂CH₃) | CH₂CH₃ | O | OCH₃ | OH |
| Ic1.403 | C(CH₂CH₃) | CH₂CH₃ | O | OCH₂CH₃ | OH |
| Ic1.404 | C(CH₂CH₃) | CH₂CH₃ | O | OCHF₂ | OH |
| Ic1.405 | C(CH₂CH₃) | CH₂CH₃ | O | OCF₃ | OH |
| Ic1.406 | C(CH₂CH₃) | CH₂Cl | O | SCH₃ | OH |
| Ic1.407 | C(CH₂CH₃) | CH₂Cl | O | SCH₂CH₃ | OH |
| Ic1.408 | C(CH₂CH₃) | CH₂Cl | O | SO₂CH₃ | OH |
| Ic1.409 | C(CH₂CH₃) | CH₂Cl | O | SO₂CH₂CH₃ | OH |
| Ic1.410 | C(CH₂CH₃) | CH₂Cl | O | SO₂CH(CH₃)₂ | OH |
| Ic1.411 | C(CH₂CH₃) | CH₂Cl | O | SO₂(CH₂)₂CH₃ | OH |
| Ic1.412 | C(CH₂CH₃) | CH₂Cl | O | Cl | OH |
| Ic1.413 | C(CH₂CH₃) | CH₂Cl | O | Br | OH |
| Ic1.414 | C(CH₂CH₃) | CH₂Cl | O | NO₂ | OH |
| Ic1.415 | C(CH₂CH₃) | CH₂Cl | O | CHF₂ | OH |
| Ic1.416 | C(CH₂CH₃) | CH₂Cl | O | CF₃ | OH |
| Ic1.417 | C(CH₂CH₃) | CH₂Cl | O | OCH₃ | OH |
| Ic1.418 | C(CH₂CH₃) | CH₂Cl | O | OCH₂CH₃ | OH |
| Ic1.419 | C(CH₂CH₃) | CH₂Cl | O | OCHF₂ | OH |
| Ic1.420 | C(CH₂CH₃) | CH₂Cl | O | OCF₃ | OH |
| Ic1.421 | C(CH₂CH₃) | H | S | SCH₃ | OH |
| Ic1.422 | C(CH₂CH₃) | H | S | SCH₂CH₃ | OH |
| Ic1.423 | C(CH₂CH₃) | H | S | SO₂CH₃ | OH |
| Ic1.424 | C(CH₂CH₃) | H | S | SO₂CH₂CH₃ | OH |
| Ic1.425 | C(CH₂CH₃) | H | S | SO₂CH(CH₃)₂ | OH |
| Ic1.426 | C(CH₂CH₃) | H | S | SO₂(CH₂)₂CH₃ | OH |
| Ic1.427 | C(CH₂CH₃) | H | S | Cl | OH |
| Ic1.428 | C(CH₂CH₃) | H | S | Br | OH |
| Ic1.429 | C(CH₂CH₃) | H | S | NO₂ | OH |
| Ic1.430 | C(CH₂CH₃) | H | S | CHF₂ | OH |
| Ic1.431 | C(CH₂CH₃) | H | S | CF₃ | OH |
| Ic1.432 | C(CH₂CH₃) | H | S | OCH₃ | OH |
| Ic1.433 | C(CH₂CH₃) | H | S | OCH₂CH₃ | OH |
| Ic1.434 | C(CH₂CH₃) | H | S | OCHF₂ | OH |
| Ic1.435 | C(CH₂CH₃) | H | S | OCF₃ | OH |
| Ic1.436 | C(CH₂CH₃) | CH₃ | S | SCH₃ | OH |
| Ic1.437 | C(CH₂CH₃) | CH₃ | S | SCH₂CH₃ | OH |
| Ic1.438 | C(CH₂CH₃) | CH₃ | S | SO₂CH₃ | OH |
| Ic1.439 | C(CH₂CH₃) | CH₃ | S | SO₂CH₂CH₃ | OH |
| Ic1.440 | C(CH₂CH₃) | CH₃ | S | SO₂CH(CH₃)₂ | OH |
| Ic1.441 | C(CH₂CH₃) | CH₃ | S | SO₂(CH₂)₂CH₃ | OH |
| Ic1.442 | C(CH₂CH₃) | CH₃ | S | Cl | OH |
| Ic1.443 | C(CH₂CH₃) | CH₃ | S | Br | OH |
| Ic1.444 | C(CH₂CH₃) | CH₃ | S | NO₂ | OH |
| Ic1.445 | C(CH₂CH₃) | CH₃ | S | CHF₂ | OH |
| Ic1.446 | C(CH₂CH₃) | CH₃ | S | CF₃ | OH |
| Ic1.447 | C(CH₂CH₃) | CH₃ | S | OCH₃ | OH |
| Ic1.448 | C(CH₂CH₃) | CH₃ | S | OCH₂CH₃ | OH |
| Ic1.449 | C(CH₂CH₃) | CH₃ | S | OCHF2 | OH |
| Ic1.450 | C(CH₂CH₃) | CH₃ | S | OCF₃ | OH |
| Ic1.451 | C(CH₂CH₃) | CH₂CH₃ | S | SCH₃ | OH |
| Ic1.452 | C(CH₂CH₃) | CH₂CH₃ | S | SCH₂CH₃ | OH |
| Ic1.453 | C(CH₂CH₃) | CH₂CH₃ | S | SO₂CH₃ | OH |
| Ic1.454 | C(CH₂CH₃) | CH₂CH₃ | S | SO₂CH₂CH₃ | OH |
| Ic1.455 | C(CH₂CH₃) | CH₂CH₃ | S | SO₂CH(CH₃)₂ | OH |
| Ic1.456 | C(CH₂CH₃) | CH₂CH₃ | S | SO₂(CH₂)₂CH₃ | OH |
| Ic1.457 | C(CH₂CH₃) | CH₂CH₃ | S | Cl | OH |
| Ic1.458 | C (CH₂CH₃) | CH₂CH₃ | S | Br | OH |
| Ic1.459 | C(CH₂CH₃) | CH₂CH₃ | S | NO₂ | OH |
| Ic1.460 | C(CH₂CH₃) | CH₂CH₃ | S | CHF₂ | OH |
| Ic1.461 | C(CH₂CH₃) | CH₂CH₃ | S | CF₃ | OH |
| Ic1.462 | C(CH₂CH₃) | CH₂CH₃ | S | OCH₃ | OH |
| Ic1.463 | C(CH₂CH₃) | CH₂CH₃ | S | OCH₂CH₃ | OH |
| Ic1.464 | C(CH₂CH₃) | CH₂CH₃ | S | OCHF₂ | OH |
| Ic1.465 | C(CH₂CH₃) | CH₂CH₃ | S | OCF₃ | OH |
| Ic1.466 | C(CH₂CH₃) | CH₂Cl | S | SCH₃ | OH |
| Ic1.467 | C(CH₂CH₃) | CH₂Cl | S | SCH₂CH₃ | OH |
| Ic1.468 | C(CH₂CH₃) | CH₂Cl | S | SO₂CH₃ | OH |
| Ic1.469 | C(CH₂CH₃) | CH₂Cl | S | SO₂CH₂CH₃ | OH |
| Ic1.470 | C(CH₂CH₃) | CH₂Cl | S | SO₂CH(CH₃)₂ | OH |
| Ic1.471 | C(CH₂CH₃) | CH₂Cl | S | SO₂(CH₂)₂CH₃ | OH |
| Ic1.472 | C(CH₂CH₃) | CH₂Cl | S | Cl | OH |
| Ic1.473 | C(CH₂CH₃) | CH₂Cl | S | Br | OH |
| Ic1.474 | C(CH₂CH₃) | CH₂Cl | S | NO₂ | OH |
| Ic1.475 | C(CH₂CH₃) | CH₂Cl | S | CHF₂ | OH |
| Ic1.476 | C(CH₂CH₃) | CH₂Cl | S | CF₃ | OH |
| Ic1.477 | C(CH₂CH₃) | CH₂Cl | S | OCH₃ | OH |
| Ic1.478 | C(CH₂CH₃) | CH₂Cl | S | OCH₂CH₃ | OH |
| Ic1.479 | C(CH₂CH₃) | CH₂Cl | S | OCHF₂ | OH |
| Ic1.480 | C(CH₂CH₃) | CH₂Cl | S | OCF₃ | OH |
| Ic1.481 | C(CH₂CH₃) | H | NCH₃ | SCH₃ | OH |
| Ic1.482 | C(CH₂CH₃) | H | NCH₃ | SCH₂CH₃ | OH |
| Ic1.483 | C(CH₂CH₃) | H | NCH₃ | SO₂CH₃ | OH |
| Ic1.484 | C(CH₂CH₃) | H | NCH₃ | SO₂CH₂CH₃ | OH |
| Ic1.485 | C(CH₂CH₃) | H | NCH₃ | SO₂CH(CH₃)₂ | OH |
| Ic1.486 | C(CH₂CH₃) | H | NCH₃ | SO₂(CH₂)₂CH₃ | OH |
| Ic1.487 | C(CH₂CH₃) | H | NCH₃ | Cl | OH |
| Ic1.488 | C(CH₂CH₃) | H | NCH₃ | Br | OH |
| Ic1.489 | C(CH₂CH₃) | H | NCH₃ | NO₂ | OH |
| Ic1.490 | C(CH₂CH₃) | H | NCH₃ | CHF₂ | OH |
| Ic1.491 | C(CH₂CH₃) | H | NCH₃ | CF₃ | OH |
| Ic1.492 | C(CH₂CH₃) | H | NCH₃ | OCH₃ | OH |
| Ic1.493 | C(CH₂CH₃) | H | NCH₃ | OCH₂CH₃ | OH |
| Ic1.494 | C(CH₂CH₃) | H | NCH₃ | OCHF₂ | OH |
| Ic1.495 | C(CH₂CH₃) | H | NCH₃ | OCF₃ | OH |
| Ic1.496 | C(CH₂CH₃) | CH₃ | NCH₃ | SCH₃ | OH |
| Ic1.497 | C(CH₂CH₃) | CH₃ | NCH₃ | SCH₂CH₃ | OH |
| Ic1.498 | C(CH₂CH₃) | CH₃ | NCH₃ | SO₂CH₃ | OH |
| Ic1.499 | C(CH₂CH₃) | CH₃ | NCH₃ | SO₂CH₂CH₃ | OH |
| Ic1.500 | C(CH₂CH₃) | CH₃ | NCH₃ | SO₂CH(CH₃)₂ | OH |
| Ic1.501 | C(CH₂CH₃) | CH₃ | NCH₃ | SO₂(CH₂)₂CH₃ | OH |
| Ic1.502 | C(CH₂CH₃) | CH₃ | NCH₃ | Cl | OH |
| Ic1.503 | C(CH₂CH₃) | CH₃ | NCH₃ | Br | OH |
| Ic1.504 | C(CH₂CH₃) | CH₃ | NCH₃ | NO₂ | OH |
| Ic1.505 | C(CH₂CH₃) | CH₃ | NCH₃ | CHF₂ | OH |
| Ic1.506 | C(CH₂CH₃) | CH₃ | NCH₃ | CF₃ | OH |
| Ic1.507 | C(CH₂CH₃) | CH₃ | NCH₃ | OCH₃ | OH |
| Ic1.508 | C(CH₂CH₃) | CH₃ | NCH₃ | OCH₂CH₃ | OH |
| Ic1.509 | C(CH₂CH₃) | CH₃ | NCH₃ | OCHF₂ | OH |
| Ic1.510 | C(CH₂CH₃) | CH₃ | NCH₃ | OCF₃ | OH |
| Ic1.511 | C(CH₂CH₃) | CH₂CH₃ | NCH₃ | SCH₃ | OH |
| Ic1.512 | C(CH₂CH₃) | CH₂CH₃ | NCH₃ | SCH₂CH₃ | OH |
| Ic1.513 | C(CH₂CH₃) | CH₂CH₃ | NCH₃ | SO₂CH₃ | OH |
| Ic1.514 | C(CH₂CH₃) | CH₂CH₃ | NCH₃ | SO₂CH₂CH₃ | OH |
| Ic1.515 | C(CH₂CH₃) | CH₂CH₃ | NCH₃ | SO₂CH(CH₃)₂ | OH |
| Ic1.516 | C(CH₂CH₃) | CH₂CH₃ | NCH₃ | SO₂(CH₂)₂CH₃ | OH |
| Ic1.517 | C(CH₂CH₃) | CH₂CH₃ | NCH₃ | Cl | OH |
| Ic1.518 | C(CH₂CH₃) | CH₂CH₃ | NCH₃ | Br | OH |
| Ic1.519 | C(CH₂CH₃) | CH₂CH₃ | NCH₃ | NO₂ | OH |
| Ic1.520 | C(CH₂CH₃) | CH₂CH₃ | NCH₃ | CHF₂ | OH |
| Ic1.521 | C(CH₂CH₃) | CH₂CH₃ | NCH₃ | CF₃ | OH |
| Ic1.522 | C(CH₂CH₃) | CH₂CH₃ | NCH₃ | OCH₃ | OH |
| Ic1.523 | C(CH₂CH₃) | CH₂CH₃ | NCH₃ | OCH₂CH₃ | OH |
| Ic1.524 | C(CH₂CH₃) | CH₂CH₃ | NCH₃ | OCHF₂ | OH |
| Ic1.525 | C(CH₂CH₃) | CH₂CH₃ | NCH₃ | OCF₃ | OH |
| Ic1.526 | C(CH₂CH₃) | CH₂Cl | NCH₃ | SCH₃ | OH |
| Ic1.527 | C(CH₂CH₃) | CH₂Cl | NCH₃ | SCH₂CH₃ | OH |
| Ic1.528 | C(CH₂CH₃) | CH₂Cl | NCH₃ | SO₂CH₃ | OH |
| Ic1.529 | C(CH₂CH₃) | CH₂Cl | NCH₃ | SO₂CH₂CH₃ | OH |
| Ic1.530 | C(CH₂CH₃) | CH₂Cl | NCH₃ | SO₂CH(CH₃)₂ | OH |
| Ic1.531 | C(CH₂CH₃) | CH₂Cl | NCH₃ | SO₂(CH₂)₂CH₃ | OH |
| Ic1.532 | C(CH₂CH₃) | CH₂Cl | NCH₃ | Cl | OH |
| Ic1.533 | C(CH₂CH₃) | CH₂Cl | NCH₃ | Br | OH |
| Ic1.534 | C(CH₂CH₃) | CH₂Cl | NCH₃ | NO₂ | OH |
| Ic1.535 | C(CH₂CH₃) | CH₂Cl | NCH₃ | CHF₂ | OH |
| Ic1.536 | C(CH₂CH₃) | CH₂Cl | NCH₃ | CF₃ | OH |
| Ic1.537 | C(CH₂CH₃) | CH₂Cl | NCH₃ | OCH₃ | OH |
| Ic1.538 | C(CH₂CH₃) | CH₂Cl | NCH₃ | OCH₂CH₃ | OH |
| Ic1.539 | C(CH₂CH₃) | CH₂Cl | NCH₃ | OCHF₂ | OH |
| Ic1.540 | C(CH₂CH₃) | CH₂Cl | NCH₃ | OCF₃ | OH |
| Ic1.541 | N | H | O | SCH₃ | OH |
| Ic1.542 | N | H | O | SCH₂CH₃ | OH |
| Ic1.543 | N | H | O | SO₂CH₃ | OH |
| Ic1.544 | N | H | O | SO₂CH₂CH₃ | OH |
| Ic1.545 | N | H | O | SO₂CH(CH₃)₂ | OH |
| Ic1.546 | N | H | O | SO₂(CH₂)₂CH₃ | OH |
| Ic1.547 | N | H | O | Cl | OH |
| Ic1.548 | N | H | O | Br | OH |
| Ic1.549 | N | H | O | NO₂ | OH |
| Ic1.550 | N | H | O | CHF₂ | OH |
| Ic1.551 | N | H | O | CF₃ | OH |
| Ic1.552 | N | H | O | OCH₃ | OH |
| Ic1.553 | N | H | O | OCH₂CH₃ | OH |
| Ic1.554 | N | H | O | OCHF₂ | OH |
| Ic1.555 | N | H | O | OCF₃ | OH |
| Ic1.556 | N | CH₃ | O | SCH₃ | OH |
| Ic1.557 | N | CH₃ | O | SCH₂CH₃ | OH |
| Ic1.558 | N | CH₃ | O | SO₂CH₃ | OH |
| Ic1.559 | N | CH₃ | O | SO₂CH₂CH₃ | OH |
| Ic1.560 | N | CH₃ | O | SO₂CH(CH₃)₂ | OH |
| Ic1.561 | N | CH₃ | O | SO₂(CH₂)₂CH₃ | OH |
| Ic1.562 | N | CH₃ | O | Cl | OH |
| Ic1.563 | N | CH₃ | O | Br | OH |
| Ic1.564 | N | CH₃ | O | NO₂ | OH |
| Ic1.565 | N | CH₃ | O | CHF₂ | OH |
| Ic1.566 | N | CH₃ | O | CF₃ | OH |
| Ic1.567 | N | CH₃ | O | OCH₃ | OH |
| Ic1.568 | N | CH₃ | O | OCH₂CH₃ | OH |
| Ic1.569 | N | CH₃ | O | OCHF₂ | OH |
| Ic1.570 | N | CH₃ | O | OCF₃ | OH |
| Ic1.571 | N | CH₂CH₃ | O | SCH₃ | OH |
| Ic1.572 | N | CH₂CH₃ | O | SCH₂CH₃ | OH |
| Ic1.573 | N | CH₂CH₃ | O | SO₂CH₃ | OH |
| Ic1.574 | N | CH₂CH₃ | O | SO₂CH₂CH₃ | OH |
| Ic1.575 | N | CH₂CH₃ | O | SO₂CH(CH₃)₂ | OH |
| Ic1.576 | N | CH₂CH₃ | O | SO₂(CH₂)₂CH₃ | OH |
| Ic1.577 | N | CH₂CH₃ | O | Cl | OH |
| Ic1.578 | N | CH₂CH₃ | O | Br | OH |
| Ic1.579 | N | CH₂CH₃ | O | NO₂ | OH |
| Ic1.580 | N | CH₂CH₃ | O | CHF₂ | OH |
| Ic1.581 | N | CH₂CH₃ | O | CF₃ | OH |
| Ic1.582 | N | CH₂CH₃ | O | OCH₃ | OH |
| Ic1.583 | N | CH₂CH₃ | O | OCH₂CH₃ | OH |
| Ic1.584 | N | CH₂CH₃ | O | OCHF₂ | OH |
| Ic1.585 | N | CH₂CH₃ | O | OCF₃ | OH |
| Ic1.586 | N | CH₂Cl | O | SCH₃ | OH |
| Ic1.587 | N | CH₂Cl | O | SCH₂CH₃ | OH |
| Ic1.588 | N | CH₂Cl | O | SO₂CH₃ | OH |
| Ic1.589 | N | CH₂Cl | O | SO₂CH₂CH₃ | OH |
| Ic1.590 | N | CH₂Cl | O | SO₂CH(CH₃)₂ | OH |
| Ic1.591 | N | CH₂Cl | O | SO₂(CH₂)₂CH₃ | OH |
| Ic1.592 | N | CH₂Cl | O | Cl | OH |
| Ic1.593 | N | CH₂Cl | O | Br | OH |
| Ic1.594 | N | CH₂Cl | O | NO₂ | OH |
| Ic1.595 | N | CH₂Cl | O | CHF₂ | OH |
| Ic1.596 | N | CH₂Cl | O | CF₃ | OH |
| Ic1.597 | N | CH₂Cl | O | OCH₃ | OH |
| Ic1.598 | N | CH₂Cl | O | OCH₂CH₃ | OH |
| Ic1.599 | N | CH₂Cl | O | OCHF₂ | OH |
| Ic1.600 | N | CH₂Cl | O | OCF₃ | OH |
| Ic1.601 | N | H | S | SCH₃ | OH |
| Ic1.602 | N | H | S | SCH₂CH₃ | OH |
| Ic1.603 | N | H | S | SO₂CH₃ | OH |
| Ic1.604 | N | H | S | SO₂CH₂CH₃ | OH |
| Ic1.605 | N | H | S | SO₂CH(CH₃)₂ | OH |
| Ic1.606 | N | H | S | SO₂(CH₂)₂CH₃ | OH |
| Ic1.607 | N | H | S | Cl | OH |
| Ic1.608 | N | H | S | Br | OH |
| Ic1.609 | N | H | S | NO₂ | OH |
| Ic1.610 | N | H | S | CHF₂ | OH |
| Ic1.611 | N | H | S | CF₃ | OH |
| Ic1.612 | N | H | S | OCH₃ | OH |
| Ic1.613 | N | H | S | OCH₂CH₃ | OH |
| Ic1.614 | N | H | S | OCHF₂ | OH |
| Ic1.615 | N | H | S | OCF₃ | OH |
| Ic1.616 | N | CH₃ | S | SCH₃ | OH |
| Ic1.617 | N | CH₃ | S | SCH₂CH₃ | OH |
| Ic1.618 | N | CH₃ | S | SO₂CH₃ | OH |
| Ic1.619 | N | CH₃ | S | SO₂CH₂CH₃ | OH |
| Ic1.620 | N | CH₃ | S | SO₂CH(CH₃)₂ | OH |
| Ic1.621 | N | CH₃ | S | SO₂(CH₂)₂CH₃ | OH |
| Ic1.622 | N | CH₃ | S | Cl | OH |
| Ic1.623 | N | CH₃ | S | Br | OH |
| Ic1.624 | N | CH₃ | S | NO₂ | OH |
| Ic1.625 | N | CH₃ | S | CHF₂ | OH |
| Ic1.626 | N | CH₃ | S | CF₃ | OH |
| Ic1.627 | N | CH₃ | S | OCH₃ | OH |
| Ic1.628 | N | CH₃ | S | OCH₂CH₃ | OH |
| Ic1.629 | N | CH₃ | S | OCHF₂ | OH |
| Ic1.630 | N | CH₃ | S | OCF₃ | OH |
| Ic1.631 | N | CH₂CH₃ | S | SCH₃ | OH |
| Ic1.632 | N | CH₂CH₃ | S | SCH₂CH₃ | OH |
| Ic1.633 | N | CH₂CH₃ | S | SO₂CH₃ | OH |
| Ic1.634 | N | CH₂CH₃ | S | SO₂CH₂CH₃ | OH |
| Ic1.635 | N | CH₂CH₃ | S | SO₂CH(CH₃)₂ | OH |
| Ic1.636 | N | CH₂CH₃ | S | SO₂(CH₂)₂CH₃ | OH |
| Ic1.637 | N | CH₂CH₃ | S | Cl | OH |
| Ic1.638 | N | CH₂CH₃ | S | Br | OH |
| Ic1.639 | N | CH₂CH₃ | S | NO₂ | OH |
| Ic1.640 | N | CH₂CH₃ | S | CHF₂ | OH |
| Ic1.641 | N | CH₂CH₃ | S | CF₃ | OH |
| Ic1.642 | N | CH₂CH₃ | S | OCH₃ | OH |
| Ic1.643 | N | CH₂CH₃ | S | OCH₂CH₃ | OH |
| Ic1.644 | N | CH₂CH₃ | S | OCHF₂ | OH |
| Ic1.645 | N | CH₂CH₃ | S | OCF₃ | OH |
| Ic1.646 | N | CH₂Cl | S | SCH₃ | OH |
| Ic1.647 | N | CH₂Cl | S | SCH₂CH₃ | OH |
| Ic1.648 | N | CH₂Cl | S | SO₂CH₃ | OH |
| Ic1.649 | N | CH₂Cl | S | SO₂CH₂CH₃ | OH |
| Ic1.650 | N | CH₂Cl | S | SO₂CH(CH₃)₂ | OH |
| Ic1.651 | N | CH₂Cl | S | SO₂(CH₂)₂CH₃ | OH |
| Ic1.652 | N | CH₂Cl | S | Cl | OH |
| Ic1.653 | N | CH₂Cl | S | Br | OH |
| Ic1.654 | N | CH₂Cl | S | NO₂ | OH |
| Ic1.655 | N | CH₂Cl | S | CHF₂ | OH |
| Ic1.656 | N | CH₂Cl | S | CF₃ | OH |
| Ic1.657 | N | CH₂Cl | S | OCH₃ | OH |
| Ic1.658 | N | CH₂Cl | S | OCH₂CH₃ | OH |
| Ic1.659 | N | CH₂Cl | S | OCHF₂ | OH |
| Ic1.660 | N | CH₂Cl | S | OCF₃ | OH |
| Ic1.661 | N | H | NCH₃ | SCH₃ | OH |
| Ic1.662 | N | H | NCH₃ | SCH₂CH₃ | OH |
| Ic1.663 | N | H | NCH₃ | SO₂CH₃ | OH |
| Ic1.664 | N | H | NCH₃ | SO₂CH₂CH₃ | OH |
| Ic1.665 | N | H | NCH₃ | SO₂CH(CH₃)₂ | OH |
| Ic1.666 | N | H | NCH₃ | SO₂(CH₂)₂CH₃ | OH |
| Ic1.667 | N | H | NCH₃ | Cl | OH |
| Ic1.668 | N | H | NCH₃ | Br | OH |
| Ic1.669 | N | H | NCH₃ | NO₂ | OH |
| Ic1.670 | N | H | NCH₃ | CHF₂ | OH |
| Ic1.671 | N | H | NCH₃ | CF₃ | OH |
| Ic1.672 | N | H | NCH₃ | OCH₃ | OH |
| Ic1.673 | N | H | NCH₃ | OCH₂CH₃ | OH |
| Ic1.674 | N | H | NCH₃ | OCHF₂ | OH |
| Ic1.675 | N | H | NCH₃ | OCF₃ | OH |
| Ic1.676 | N | CH₃ | NCH₃ | SCH₃ | OH |
| Ic1.677 | N | CH₃ | NCH₃ | SCH₂CH₃ | OH |
| Ic1.678 | N | CH₃ | NCH₃ | SO₂CH₃ | OH |
| Ic1.679 | N | CH₃ | NCH₃ | SO₂CH₂CH₃ | OH |
| Ic1.680 | N | CH₃ | NCH₃ | SO₂CH(CH₃)₂ | OH |
| Ic1.681 | N | CH₃ | NCH₃ | SO₂(CH₂)₂CH₃ | OH |
| Ic1.682 | N | CH₃ | NCH₃ | Cl | OH |
| Ic1.683 | N | CH₃ | NCH₃ | Br | OH |
| Ic1.684 | N | CH₃ | NCH₃ | NO₂ | OH |
| Ic1.685 | N | CH₃ | NCH₃ | CHF₂ | OH |
| Ic1.686 | N | CH₃ | NCH₃ | CF₃ | OH |
| Ic1.687 | N | CH₃ | NCH₃ | OCH₃ | OH |
| Ic1.688 | N | CH₃ | NCH₃ | OCH₂CH₃ | OH |
| Ic1.689 | N | CH₃ | NCH₃ | OCHF₂ | OH |
| Ic1.690 | N | CH₃ | NCH₃ | OCF₃ | OH |
| Ic1.691 | N | CH₂CH₃ | NCH₃ | SCH₃ | OH |
| Ic1.692 | N | CH₂CH₃ | NCH₃ | SCH₂CH₃ | OH |
| Ic1.693 | N | CH₂CH₃ | NCH₃ | SO₂CH₃ | OH |
| Ic1.694 | N | CH₂CH₃ | NCH₃ | SO₂CH₂CH₃ | OH |
| Ic1.695 | N | CH₂CH₃ | NCH₃ | SO₂CH(CH₃)₂ | OH |
| Ic1.696 | N | CH₂CH₃ | NCH₃ | SO₂(CH₂)₂CH₃ | OH |
| Ic1.697 | N | CH₂CH₃ | NCH₃ | Cl | OH |
| Ic1.698 | N | CH₂CH₃ | NCH₃ | Br | OH |
| Ic1.699 | N | CH₂CH₃ | NCH₃ | NO₂ | OH |
| Ic1.700 | N | CH₂CH₃ | NCH₃ | CHF₂ | OH |
| Ic1.701 | N | CH₂CH₃ | NCH₃ | CF₃ | OH |
| Ic1.702 | N | CH₂CH₃ | NCH₃ | OCH₃ | OH |
| Ic1.703 | N | CH₂CH₃ | NCH₃ | OCH₂CH₃ | OH |
| Ic1.704 | N | CH₂CH₃ | NCH₃ | OCHF₂ | OH |
| Ic1.705 | N | CH₂CH₃ | NCH₃ | OCF₃ | OH |
| Ic1.706 | N | CH₂Cl | NCH₃ | SCH₃ | OH |
| Ic1.707 | N | CH₂Cl | NCH₃ | SCH₂CH₃ | OH |
| Ic1.708 | N | CH₂Cl | NCH₃ | SO₂CH₃ | OH |
| Ic1.709 | N | CH₂Cl | NCH₃ | SO₂CH₂CH₃ | OH |
| Ic1.710 | N | CH₂Cl | NCH₃ | SO₂CH(CH₃)₂ | OH |
| Ic1.711 | N | CH₂Cl | NCH₃ | SO₂(CH₂)₂CH₃ | OH |
| Ic1.712 | N | CH₂Cl | NCH₃ | Cl | OH |
| Ic1.713 | N | CH₂Cl | NCH₃ | Br | OH |
| Ic1.714 | N | CH₂Cl | NCH₃ | NO₂ | OH |
| Ic1.715 | N | CH₂Cl | NCH₃ | CHF₂ | OH |
| Ic1.716 | N | CH₂Cl | NCH₃ | CF₃ | OH |
| Ic1.717 | N | CH₂Cl | NCH₃ | OCH₃ | OH |
| Ic1.718 | N | CH₂Cl | NCH₃ | OCH₂CH₃ | OH |
| Ic1.719 | N | CH₂Cl | NCH₃ | OCHF₂ | OH |
| Ic1.720 | N | CH₂Cl | NCH₃ | OCF₃ | OH |

Ebenso außerordentlichst bevorzugt sind die Verbindungen der Formel Ic2, insbesondere die Verbindungen Ic2.1 bis Ic2.720, die sich von den entsprechenden Verbindungen Ic1.1 bis Ic1.720 dadurch unterscheiden, daß R¹⁴ für Methyl steht.

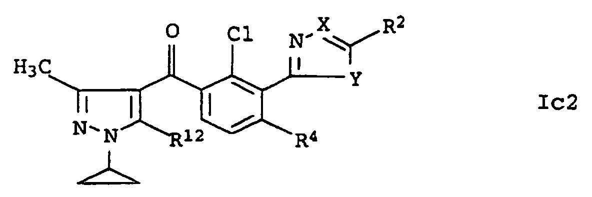

Ebenso außerordentlichst bevorzugt sind die Verbindungen der Formel Ic3, insbesondere die Verbindungen Ic3.1 bis Ic3.720, die sich von den Verbindungen Ic1.1 bis Ic1.720 dadurch unterscheiden, daß R¹³ für Cyclopentyl steht.

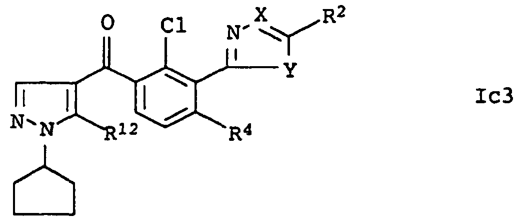

Ebenso außerordentlichst bevorzugt sind die Verbindungen der Formel Ic4, insbesondere die Verbindungen Ic4.1 bis Ic4.720, die sich von den Verbindungen Ic1.1 bis Ic1.720 dadurch unterscheiden, daß R¹³ für Cyclopentyl und R¹⁴ für Methyl stehen.

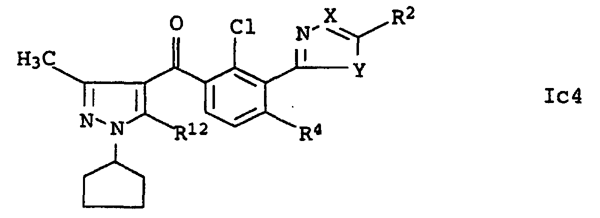

Ebenso außerordentlichst bevorzugt sind die Verbindungen der Formel Ic5, insbesondere die Verbindungen Ic5.1 bis Ic5.720, die sich von den Verbindungen Ic1.1 bis Icl.720 dadurch unterscheiden, daß R¹³ für Cyclohexyl steht.

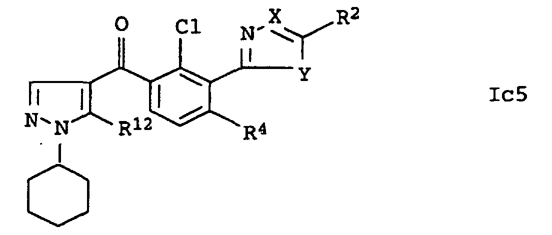

Ebenso außerordentlichst bevorzugt sind die Verbindungen der Formel Ic6, insbesondere die Verbindungen Ic6.1 bis Ic6.720, die sich von den Verbindungen Ic1.1 bis Ic1.720 dadurch unterscheiden, daß R¹³ für Cyclohexyl und R¹⁴ für Methyl stehen.

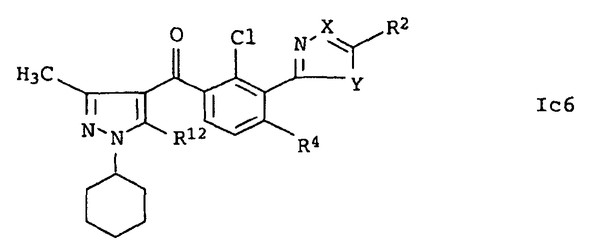

Ebenso außerordentlichst bevorzugt sind die Verbindungen der Formel Ic7, insbesondere die Verbindungen Ic7.1 bis Ic7.720, die sich von den entsprechenden Verbindungen Ic1.1 bis Ic1.720 dadurch unterscheiden, daß R¹ für Methyl steht.

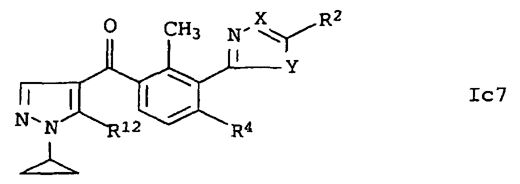

Ebenso außerordentlichst bevorzugt sind die Verbindungen der Formel Ic8, insbesondere die Verbindungen Ic8.1 bis Ic8.720, die sich von den entsprechenden Verbindungen Ic1.1 bis Ic1.720 dadurch unterscheiden, daß R¹ für Methyl und R¹⁴ für Methyl stehen.

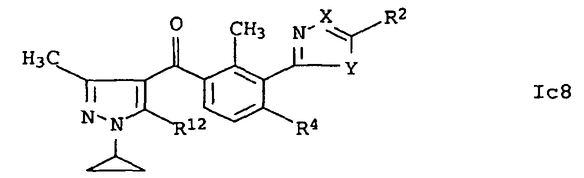

Ebenso außerordentlichst bevorzugt sind die Verbindungen der Formel Ic9, insbesondere die Verbindungen Ic9.1 bis Ic9.720, die sich von den Verbindungen Ic1.1 bis Ic1.720 dadurch unterscheiden, daß R¹ für Methyl und R¹³ für Cyclopentyl stehen.

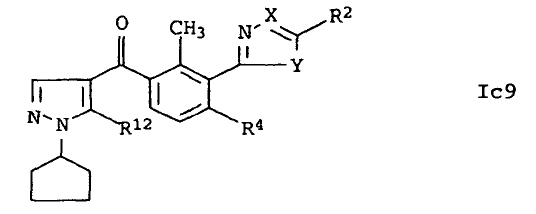

Ebenso außerordentlichst bevorzugt sind die Verbindungen der Formel Ic10, insbesondere die Verbindungen Ic10.1 bis Ic10.720, die sich von den Verbindungen Ic1.1 bis Ic1.720 dadurch unterscheiden, daß R¹ für Methyl, R¹³ für Cyclopentyl und R¹⁴ für Methyl stehen.

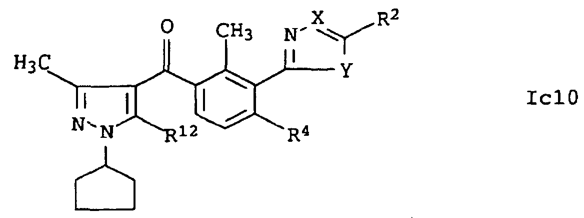

Ebenso außerordentlichst bevorzugt sind die Verbindungen der Formel Ic11, insbesondere die Verbindungen Ic11.1 bis Ic11.720, die sich von den Verbindungen Ic1.1 bis Ic1.720 dadurch unterscheiden, daß R¹ für Methyl und R¹³ für Cyclohexyl stehen.

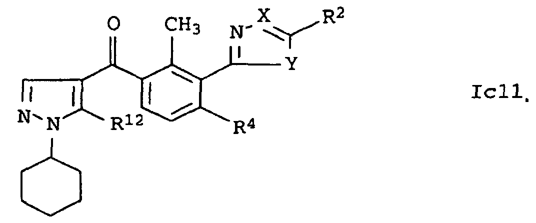

Ebenso außerordentlichst bevorzugt sind die Verbindungen der Formel Ic12, insbesondere die Verbindungen Ic12.1 bis Ic12.720, die sich von den Verbindungen Ic1.1 bis Ic1.720 dadurch unterscheiden, daß R¹ für Methyl, R¹³ für Cyclohexyl und R¹⁴ für Methyl stehen.

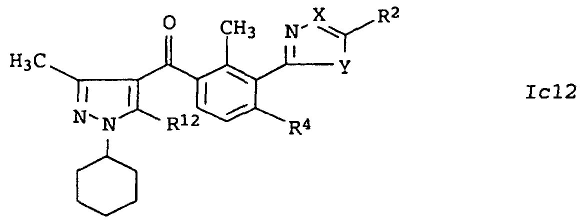

Ebenso außerordentlichst bevorzugt sind die Verbindungen der Formel Ic13, insbesondere die Verbindungen Ic13.1 bis Ic13.720, die sich von den entsprechenden Verbindungen Ic1.1 bis Ic1.720 dadurch unterscheiden, daß R¹ für Methoxy steht.

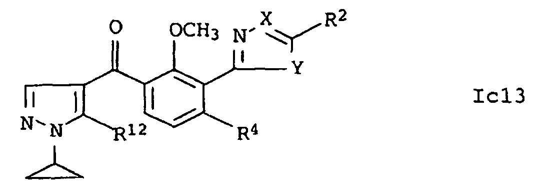

Ebenso außerordentlichst bevorzugt sind die Verbindungen der Formel Ic14, insbesondere die Verbindungen Ic14.1 bis Ic14.720, die sich von den entsprechenden Verbindungen Ic1.1 bis Ic1.720 dadurch unterscheiden, daß R¹ für Methoxy und R¹⁴ für Methyl stehen.

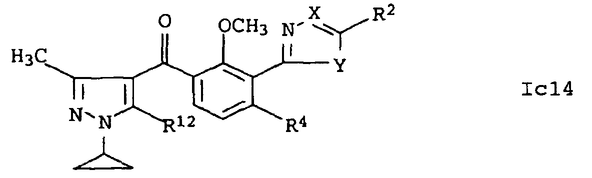

Ebenso außerordentlichst bevorzugt sind die Verbindungen der Formel Ic15, insbesondere die Verbindungen Ic15.1 bis Ic15.720, die sich von den Verbindungen Ic1.1 bis Ic1.720 dadurch unterscheiden, daß R¹ für Methoxy und R¹³ für Cyclopentyl stehen.

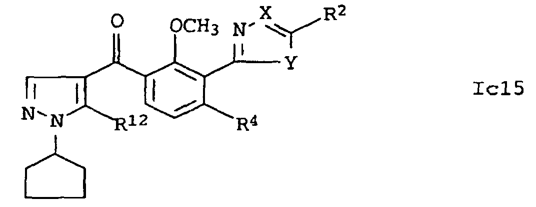

Ebenso außerordentlichst bevorzugt sind die Verbindungen der Formel Ic16, insbesondere die Verbindungen Ic16.1 bis Ic16.720, die sich von den Verbindungen Ic1.1 bis Ic1.720 dadurch unterscheiden, daß R¹ für Methoxy, R¹³ für Cyclopentyl und R¹⁴ für Methyl stehen.

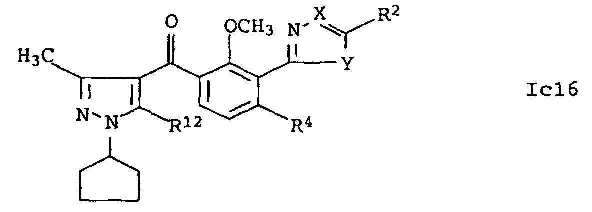

Ebenso außerordentlichst bevorzugt sind die Verbindungen der Formel Ic17, insbesondere die Verbindungen Ic17.1 bis Ic17.720, die sich von den Verbindungen Ic1.1 bis Ic1.720 dadurch unterscheiden, daß R¹ für Methoxy und R¹³ für Cylcohexyl stehen.

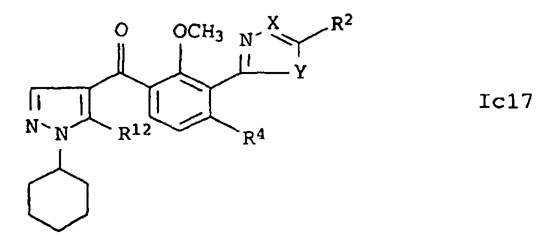

Ebenso außerordentlichst bevorzugt sind die Verbindungen der Formel Ic18, insbesondere die Verbindungen Ic18.1 bis Ic18.720, die sich von den Verbindungen Ic1.1 bis Ic1.720 dadurch unterscheiden, daß R¹ für Methoxy, R¹³ für Cyclohexyl und R¹⁴ für Methyl stehen.

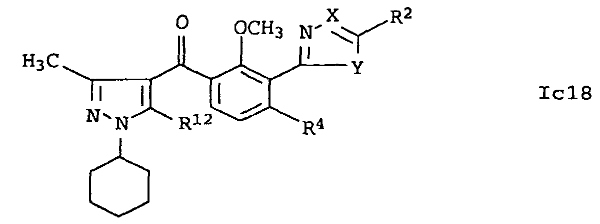

Die 1-Cycloalkylpyrazolyl-benzoyl-Derivate der Formel I, sind auf verschiedene Art und Weise erhältlich, beispielsweise nach folgenden Verfahren.

### Verfahren A:

Durch Umsetzung von Pyrazolen der Formel II mit einer aktivierten Benzoesäure IIIα oder einer Benzoesäure IIIβ, die vorzugsweise in situ aktiviert wird, zu dem entsprechenden Acylieruhgsprodukt IV und anschließende Umlagerung erhält man Verbindungen der Formel I mit R¹² = OH.

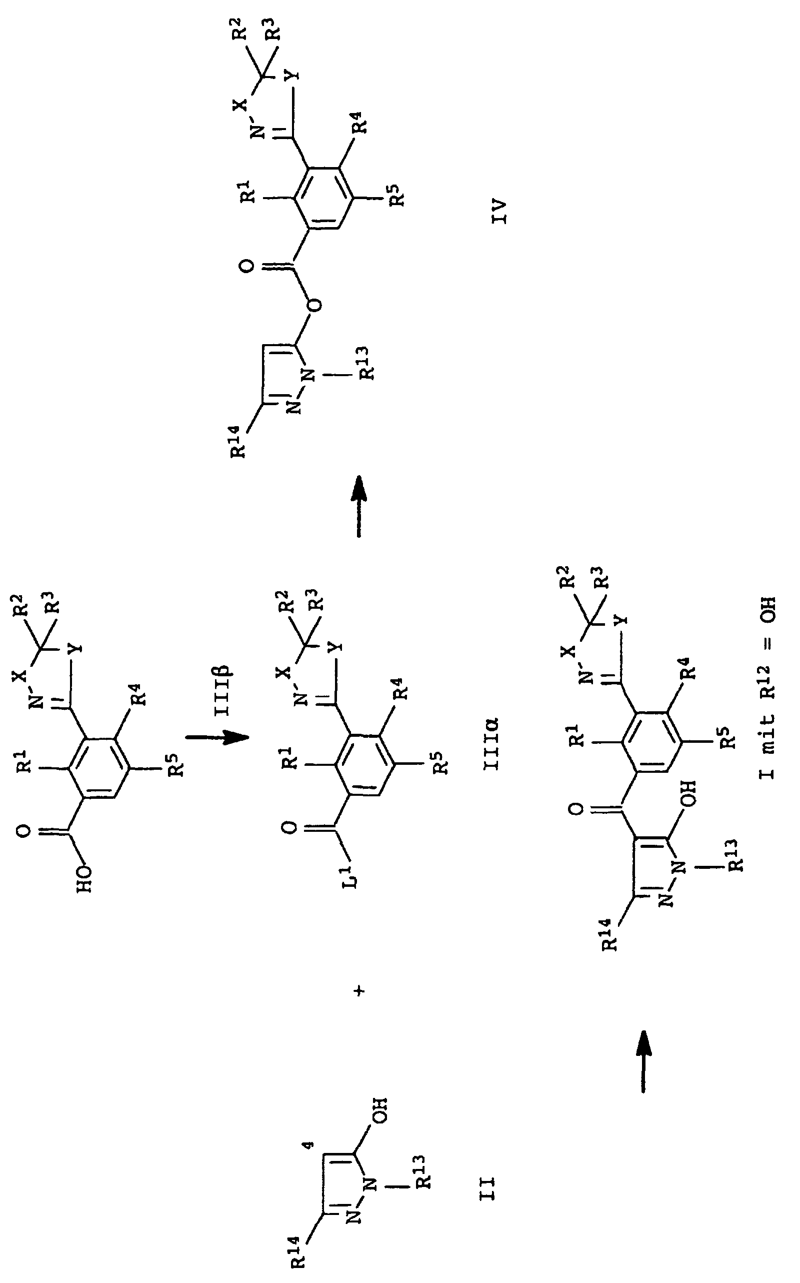

L¹ steht für eine nucleophil verdrängbare Abgangsgruppe, wie Halogen z.B. Brom, Chlor, Hetaryl, z.B. Imidazolyl, Pyridyl, Carboxylat, z.B. Acetat, Trifluoracetat etc.

Die aktivierte Benzoesäure kann direkt eingesetzt werden, wie im Fall der Benzoylhalogenide oder in situ erzeugt werden, z.B. mit Dicyclohexylcarbodiimid, Triphenylphosphin/Azodicarbonsäureester, 2-Pyridindisulfid/Triphenylphosphin, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuß der Hilfsbase z.B. 1,2 bis 1,5 Moläquivalente, bezogen auf II, kann unter Umständen vorteilhaft sein.

Als Hilfsbasen eignen sich tertiäre Alkylamine, Pyridin oder Alkalimetallcarbonate. Als Lösungsmittel können z.B. chlorierte Kohlenwasserstoffe, wie Methylenchlorid, 1,2-Dichlorethan, aromatische Kohlenwasserstoffe, wie Toluol, Xylol, Chlorbenzol, Ether, wie Diethylether, Methyl-tert.-butylether, Dimethoxyethan, Tetrahydrofuran, Dioxan, polare aprotische Lösungsmittel, wie Acetonitril, Dimethylformamid, Dimethylsulfoxid oder Ester wie Essigsäureethylester oder Gemische hiervon verwendet werden.

Werden Benzoylhalogenide als aktivierte Carbonsäurekomponente eingesetzt, so kann es zweckmäßig sein, bei Zugabe dieses Reaktionspartners die Reaktionsmischung auf 0 - 10°C abzukühlen. Anschließend rührt man bei 20 - 100°C, vorzugsweise bei 25 - 50°C, bis die Umsetzung vollständig ist. Die Aufarbeitung erfolgt in üblicher Weise, z.B. wird das Reaktionsgemisch auf Wasser gegossen, das Wertprodukt extrahiert. Als Lösungsmittel eignen sich hierfür besonders Methylenchlorid, Diethylether, Dimethoxyethan und Essigsäureethylester. Nach Trocknen der organischen Phase und Entfernen des Lösungsmittels kann der rohe Ester ohne weitere Reinigung zur Umlagerung eingesetzt werden.

Die Umlagerung der Ester zu den Verbindungen der Formel I erfolgt zweckmäßigerweise bei Temperaturen von 20 bis 40°C in einem Lösungsmittel und in Gegenwart einer Base sowie gegebenenfalls mit Hilfe einer Cyanoverbindung als Katalysator.

Als Lösungsmittel können z.B. Acetonitril, Methylenchlorid, 1,2-Dichlorethan, Dioxan, Essigsäureethylester, Dimethoxyethan, Toluol oder Gemische hiervon verwendet werden. Bevorzugte Lösungsmittel sind Acetonitril und Dioxan.

Geeignete Basen sind tertiäre Amine wie Triethylamin, Pyridin oder Alkalicarbonate, wie Natriumcarbonat, Kaliumcarbonat, die vorzugsweise in äquimolarer Menge oder bis zu einem vierfachen Überschuß, bezogen auf den Ester, eingesetzt werden. Bevorzugt werden Triethylamin oder Alkalicarbonate verwendet, vorzugsweise in doppelt äquimolaren Verhältnis in Bezug auf den Ester.

Als Cyanoverbindungen kommen anorganische Cyanide, wie Natriumcyanid, Kaliumcyanid und organische Cyanoverbindungen, wie Acetoncyanhydrin, Trimethylsilycyanid in Betracht. Sie werden in einer Menge von 1 bis 50 Molprozent, bezogen auf den Ester, eingesetzt. Vorzugsweise werden Acetoncyanhydrin oder Trimethylsilylcyanid, z.B. in einer Menge von 5 bis 15, vorzugsweise 10 Molprozent, bezogen auf den Ester, eingesetzt.

Die Aufarbeitung kann in an sich bekannter weise erfolgen. Das Reaktionsgemisch wird z.B. mit verdünnter Mineralsäure, wie 5 %ige Salzsäure oder Schwefelsäure, angesäuert, mit einem organischen Lösungsmittel, z.B. Methylenchlorid, Essigsäureethylester extrahiert. Der organische Extrakt kann mit 5-10%iger Alkalicarbonatlösung, z.B. Natriumcarbonat-, Kaliumcarbonatlösung extrahiert werden. Die wäßrige Phase wird angesäuert und der sich bildende Niederschlag abgesaugt und/oder mit Methylenchlorid oder Essigsäureethylester extrahiert, getrocknet und eingeengt. (Beispiele für die Darstellung von Estern von Hydroxypyrazolen und für die Umlagerung der Ester sind z.B. in EP-A 282 944 und US 4 643 757 genannt).

Es ist aber auch möglich, das "Acylierungsprodukt" IV in situ zu erzeugen, indem man ein Pyrazol der Formel II, oder ein Alkalisalz hiervon, mit einem 3-(Heterocyclyl)-benzol-Derivat der Formel V in Gegenwart von Kohlenmonoxid, eines Katalysators sowie einer Base, umsetzt.

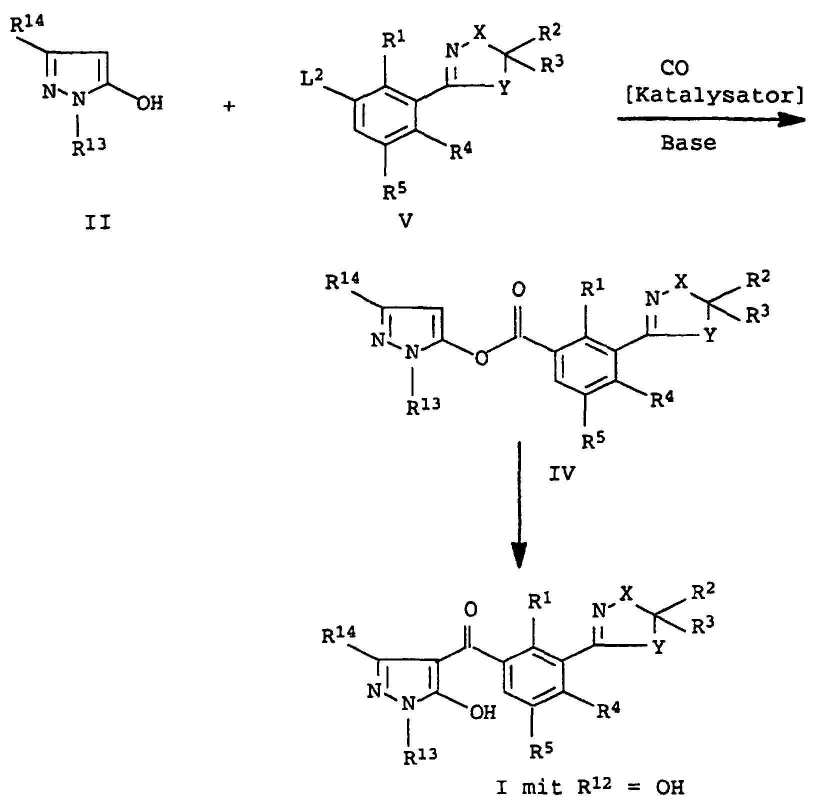

L² steht für eine Abgangsgruppe wie Halogen, z.B. Chlor, Brom oder Iod, oder Sulfonat wie Mesylat oder Triflat; bevorzugt sind Brom oder Triflat.

Das "Acylierungsprodukt" IV reagiert ggf. direkt zu dem 1-Cycloalkylpyrazolyl-benzoyl-Derivat der Formel I ab.

Als Katalysatoren eignen sich Palladiumligandkomplexe, in denen das Palladium in der Oxidationsstufe 0 vorliegt, metallisches Palladium, das gegebenenfalls auf einen Träger aufgezogen wurde, und vorzugsweise Palladium(II)salze. Die Umsetzung mit Palladium(II)salzen und metallischem Palladium wird vorzugsweise in Gegenwart von Komplexliganden durchgeführt.

Als Palladium(0)ligandkomplex kommt beispielsweise Tetrakis(triphenylphosphan)palladium in Frage.

Metallisches Palladium ist vorzugsweise auf einen inerten Träger wie beispielsweise Aktivkohle, Siliciumdioxid, Aluminiumoxid, Bariumsulfat oder Calciumcarbonat aufgezogen. Die Reaktion wird vorzugsweise in Gegenwart von Komplexliganden wie beispielsweise Triphenylphosphan durchgeführt.

Als Palladium(II)salze eigenen sich beispielsweise Palladiumacetat und Palladiumchlorid. Bevorzugt wird in Gegenwart von Komplexliganden wie beispielsweise Triphenylphosphan gearbeitet.

Geeignete Komplexliganden für die Palladiumligandkomplexe, bzw. in deren Gegenwart die Umsetzung mit metallischem Palladium oder Palladium(II)salzen vorzugsweise ausgeführt wird, sind tertiäre Phosphane, deren Struktur durch folgende Formeln wiedergegeben wird: 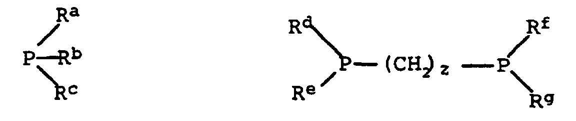 wobei z die Zahlen 1 bis 4 bedeutet und die Reste R^{a} bis R^{g} für C₁-C₆-Alkyl, C₃-C₆-Cycloalkyl, Aryl-C₁-C₂-alkyl oder vorzugsweise Aryl stehen. Aryl steht beispielsweise für Naphthyl und gegebenenfalls substituiertes Phenyl wie beispielsweise 2-Tolyl und insbesondere für unsubstituiertes Phenyl.

Die Herstellung der komplexen Palladiumsalze kann in an sich bekannter weise ausgehend von kommerziell erhältlichen Palladiumsalzen wie Palladiumchlorid oder Palladiumacetat und den entsprechenden Phosphanen wie z.B. Triphenylphosphan oder 1,2-Bis(diphenylphosphano)ethan erfolgen. Ein Großteil der komplexierten Palladiumsalze ist auch kommerziell erhältlich. Bevorzugte Palladiumsalze sind [(R)(+)2,2'-Bis(diphenylphosphano)-1,1'-binaphthyl]palladium(II)chlorid, Bis(triphenylphosphan)palladium(II)acetat und insbesondere Bis(triphenylphosphan)palladium(II)chlorid.

Der Palladiumkatalysator wird in der Regel in einer Konzentration von 0,05 bis 5 Mol%, bevorzugt 1-3 Mol%, eingesetzt.

Als Basen kommen tertiäre Amine wie beispielsweise N-Methylpiperidin, Ethyldiisopropylamin, 1,8-Bisdimethylaminonaphthalin oder insbesondere Triethylamin in Betracht. Ebenso eigenen sich Alkalicarbonat, wie Natriumcarbonat oder Kaliumcarbonat. Aber auch Gemische von Kaliumcarbonat und Triethylamin sind geeignet.

In der Regel werden 2 bis 4 Moläquivalente, insbesondere 2 Moläquivalente, des Alkalicarbonats, sowie 1 bis 4 Moläquivalente, insbesondere 2 Moläquivalente des tertiären Amins bezogen auf das 3-(Heterocyclyl)-benzol-Derivat der Formel V eingesetzt.

Als Lösungsmittel können Nitrile wie Benzonitril und Acetonitril, Amide wie Dimethylformamid, Dimethylacetamid, Tetra-C₁-C₄-alkylharnstoffe oder N-Methylpyrrolidon und vorzugsweise Ether wie Tetrahydrofuran, Methyl-tert.-butylether dienen. Insbesondere werden Ether wie 1,4-Dioxan und Dimethoxyethan als Lösungsmittel bevorzugt.

### Verfahren B:

Verbindungen der Formel I mit R¹² 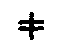 Hydroxy werden durch Umsetzung von Verbindungen der Formel I mit R¹² = Hydroxy mit Alkylierungsmitteln, Sulfonylierungsmittel bzw. Acylierungsmitteln L³-R^{12a} (VI) erhalten.

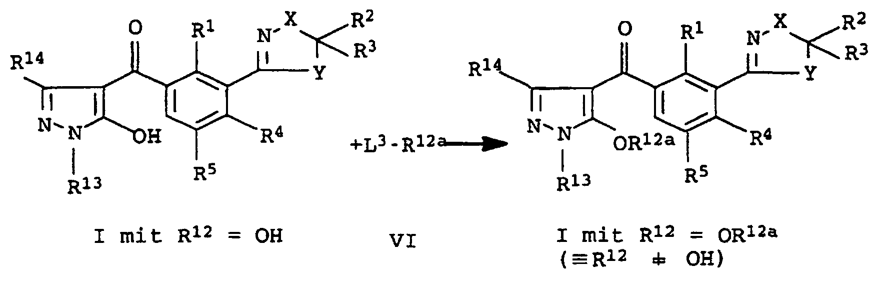

L³ steht für eine nucleophil verdrängbare Abgangsgruppe, wie Halogen, z.B. Brom oder Chlor, Acyloxy z.B. Acetyloxy, Ethylcarbonyloxy, oder Alkylsulfonyloxy, z.B. Methylsulfonyloxy oder Trifluormethylsulfonyloxy;

R^{12a} steht für C₁-C₆-Alkyl, C₃-C₆-Alkenyl, C₁-C₆-Alkylsulfonyl, C₁-C₆-Alkylcarbonyl, Phenylsulfonyl oder Phenylcarbonyl, wobei der Phenylrest der beiden letztgenannten Substituenten partiell oder vollständig halogeniert sein kann und/oder eine bis drei der folgenden Gruppen tragen kann:
Nitro, Cyano, C₁-C₄-Alkyl, C₁-C₄-Halogenalkyl, C₁-C₄-Alkoxy oder C₁-C₄-Halogenalkoxy;

Die Verbindungen der Formel VI können direkt eingesetzt werden, wie z.B. im Fall der Sulfonsäurehalogenide, Sulfonsäureanhydride oder in situ erzeugt werden, z.B. aktivierte Sulfonsäuren (mittels Sulfonsäure und Dicyclohexylcarbonyldiimid, Carbonyldiimidazol etc.).

Die Ausgangsverbindungen werden in der Regel im äquimolaren Verhältnis eingesetzt. Es kann aber auch von Vorteil sein, die eine oder andere Komponente im Überschuß einzusetzen.

Gegebenenfalls kann es von Vorteil sein, die Umsetzung in Gegenwart einer Base durchzuführen. Die Reaktanden und die Hilfsbase werden dabei zweckmäßigerweise in äquimolaren Mengen eingesetzt. Ein Überschuß der Hilfsbase z.B. 1,5 bis 3 Moläquivalente, bezogen auf I, kann unter Umständen vorteilhaft sein.

Als Hilfsbasen eignen sich tertiäre Alkylamine, wie Triethylamin, Pyridin, Alkalimetallcarbonate, z.B. Natriumcarbonat, Kaliumcarbonat und Alkalimetallhydride, z.B. Natriumhydrid. Bevorzugt verwendet werden Triethylamin und Pyridin.

Als Lösungsmittel kommen z.B. chlorierte Kohlenwasserstoffe, wie Methylenchlorid, 1,2-Dichlorethan, aromatische Kohlenwasserstoffe, z.B. Toluol, Xylol, Chlorbenzol, Ether, wie Diethylether, Methyl-tert.-butylether, Tetrahydrofuran, Dioxan, polare aprotische Lösungsmittel, wie Acetonitril, Dimethylformamid, Dimethylsulfoxid oder Ester, wie Essigsäureethylester, oder Gemische hiervon in Betracht.

In der Regel liegt die Reaktionstemperatur im Bereich von 0°C bis zur Höhe des Siedepunktes des Reaktionsgemisches.

Die Aufarbeitung kann in an sich bekannter Weise zum Produkt hin erfolgen.

Die als Ausgangsmaterialien verwendeten Pyrazole der Formel II sind bekannt oder können nach an sich bekannten Verfahren hergestellt werden (z.B. EP-A 240 001 und J. Prakt. Chem. 315, 383 (1973)). Ebenso sind die aktivierten Benzoesäuren IIIα bzw. die Benzoesäuren IIIβ bekannt oder können auf an sich bekannte Weise hergestellt werden (z.B. WO 96/26206). Weiterhin sind die 3-(Heterocyclyl)-benzol-Derivate der Formel V bekannt oder können auf an sich bekannte weise hergestellt werden (PCT/EP/99/03006).

### Herstellungsbeispiel:

### 4-[2-Methyl-3-(4,5-dihydroisoxazol-3-yl)-4-methylsulfonyl-benzoyl]-1-cyclopentyl-5-hydroxy-1H-pyrazol (Verbindung 4.6)

Zu einer Lösung von 2,5 g (8,8 mmol) 2-Methyl-3-(4,5-dihydro-isoxazol-3-yl)-4-methylsulfonylbenzoesäure in 100 ml Dioxan wurden 1,9 g (9,4 mmol) Dicyclohexylcarbodiimid und 1,3 g (8,8 mmol) 1-Cyclopentyl-5-hydroxy-1H-pyrazol gegeben und 12 Stunden bei Raumtemperatur gerührt. Anschließend wurden feste Bestandteile abfiltriert, das Filtrat mit 1,5 g (10 mmol) Kaliumcarbonat versetzt und 4 Stunden unter Rückfluß erhitzt. Nach Entfernen des Lösungsmittels wurde der Rückstand in Wasser aufgenommen, mit Essigsäureethylester gewaschen, anschließend mit 10 %iger Salzsäure ein pH-Wert von 3 eingestellt und mit Methylenchlorid extrahiert. Von der resultierenden organischen Phase wurde das Lösungsmittel abdestilliert, der Rückstand in 300 ml wäßrige Kaliumcarbonatlösung aufgenommen, unlösliche Bestandteile abfiltriert, mit 10 %iger Salzsäure ein pH-Wert von 3 eingestellt, der sich bildende Niederschlag abgetrennt und getrocknet.
Ausbeute: 1,5 g (42 % der Theorie) 4-[2-Methyl-3-(4,5-dihydroisoxazol-3-yl)-4-methylsulfonyl-benzoyl]-1-cyclopentyl-5-hydroxy-1Hpyrazol
Fp.: 135-140°C

In Tabelle 4 sind neben der voranstehenden Verbindung noch weitere 1-Cycloalkylpyrazolyl-benzoyl-Derivate der Formel I aufgeführt, die in analoger Weise hergestellt wurden oder herstellbar sind:

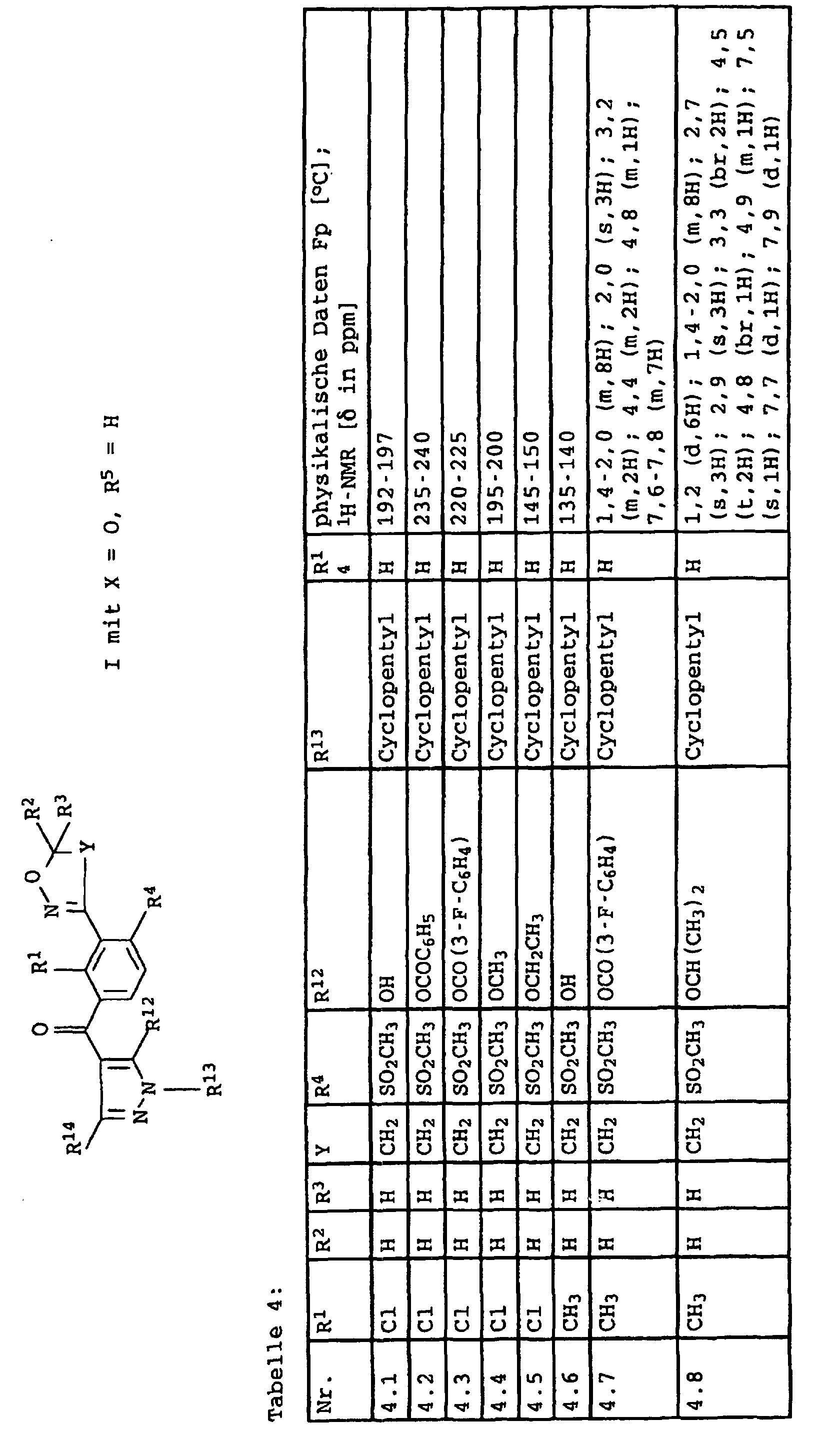

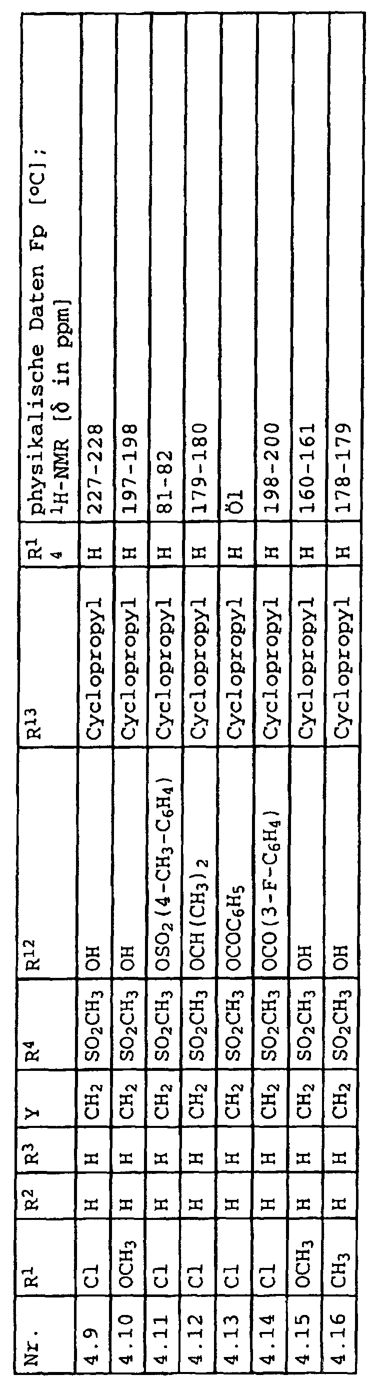

Die 1-Cycloalkylpyrazolyl-benzoyl-Derivate der Formel I und deren landwirtschaftlich brauchbaren Salze eignen sich - sowohl als Isomerengemische als auch in Form der reinen Isomeren - als Herbizide. Die Verbindungen der Formel I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa , Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Die Verbindungen der Formel I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlich brauchbaren Salzes von I und für die Formulierung von Pflanzenschutzmitteln übliche Hilfsmittel.

Als inerte Hilfsstoffe kommen im wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Verbindungen der Formel I in den anwendungsfertigen Zubereitungen können in weiten Bereichen variiert werden. Im allgemeinen enthalten die Formulierungen etwa von 0,001 bis 98 Gew.-%, vorzugsweise 0,01 bis 95 Gew.-%, mindestens eines Wirkstoffs. Die wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
I. 20 Gewichtsteile einer Verbindung Nr. 4.6 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile einer Verbindung Nr. 4.6 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile einer Verbindung Nr. 4.6 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile einer verbindung Nr. 4.6 werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gew.-% des Wirkstoffs enthält.
V. 3 Gewichtsteile einer Verbindung Nr. 4.6 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile einer Verbindung Nr. 4.6 werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil einer Verbindung Nr. 4.6 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil einer Verbindung Nr. 4.6 wird in einer Mischung gelöst, die aus 80 Gewichtsteilen Cyclohexanon und 20 Gewichtsteilen Wettol® EM 31 (= nichtionischer Emulgator auf der Basis von ethoxyliertem Rizinusöl) besteht. Man erhält ein stabiles Emulsionskonzentrat.

Die Applikation der Verbindungen der Formel I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Verbindung der Formel I betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

Zur Verbreiterung des Wirkungsspektrums und zur Erzielung synergistischer Effekte können die 1-Cycloalkylpyrazolyl-benzoyl-Derivate der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzo:furane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen der Formel I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Anwendungsbeispiele

Die herbizide Wirkung der 1-Cycloalkylpyrazolyl-benzoyl-Derivate der Formel I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,5 bzw. 0,25 kg/ha a.s.

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen

| | | |
|---|---|---|
| Lateinischer Name | Deutscher Name | Englischer Name |
| Brachiaria plantaginea | - | alexandergras |
| Chenopodium album | weißer Gänsefuß | lambsquaters |
| Echinochloa crusgalli | Hühnerhirse | barnyardgrass |
| Polygonum persicaria | Flohknöterich | ladysthumb |

Bei Aufwandmengen von 0,5 bzw. 0,25 kg/ha zeigte die Verbindung 4.6 (Tabelle 4) im Nachauflauf eine sehr gute Wirkung gegen die voranstehend genannten unerwünschten Pflanzen.

## Patentansprüche

1. 1-Cycloalkylpyrazol-benzoyl-Derivate der Formel I 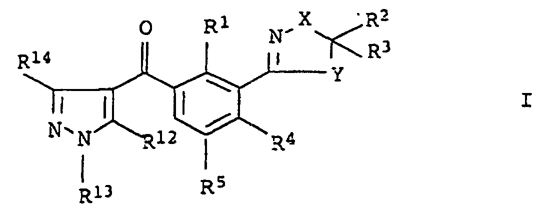 in der die Variablen folgende Bedeutung haben:
X O, NR⁶ oder CR⁷R⁸;
Y O, S, NR⁹ oder CR¹⁰R¹¹;
R¹ Nitro, Halogen, C₁-C₄-Alkyl, oder C₁-C₄-Alkoxy;
R²,R³,R⁷,R⁸,R¹⁰,R¹¹ Wasserstoff, C₁-C₄-Alkyl oder C₁-C₄-Halogenalkyl;
oder
R³ und R⁶ oder R³ und R⁸ oder R³ und R⁹ oder R³ und R¹¹ bilden gemeinsam eine Bindung aus;
R⁴ Halogen, Nitro, C₁-C₄-Halogenalkyl, C₁-C₄-Alkoxy, C₁-C₄-Halogenalkoxy, C₁-C₄-Alkylthio, oder C₁-C₄-Alkylsulfonyl;
R⁵ Wasserstoff;
R⁶,R⁹ Wasserstoff oder C₁-C₆-Alkyl;
R¹² Hydroxy, C₁-C₆-Alkoxy, C₃-C₆-Alkenyloxy, C₁-C₆-Alkylsulfonyloxy, C₁-C₆-Alkylcarbonyloxy, Phenylsulfonyloxy oder Phenylcarbonyloxy, wobei der Phenylrest der beiden letztgenannten Substituenten partiell oder vollständig halogeniert sein kann und/oder eine bis drei der folgenden Gruppen tragen kann:
Nitro, Cyano, C₁-C₄-Alkyl, C₁-C₄-Halogenalkyl, C₁-C₄-Alkoxy oder C₁-C₄-Halogenalkoxy;
R¹³ C₃-C₆-Cycloalkyl;
R¹⁴ Wasserstoff oder C₁-C₄-Alkyl;
sowie deren landwirtschaftlich brauchbaren Salze.

2. 1-Cycloalkylpyrazolyl-benzoyl-Derivate der Formel I nach Anspruch 1, wobei
X O;
R¹ Nitro, Halogen, C₁-C₄-Alkyl oder C₁-C₄-Alkoxy;
R⁴ Halogen, Nitro, C₁-C₄-Halogenalkyl, C₁-C₄-Alkoxy, C₁-C₄-Halogenalkoxy, C₁-C₄-Alkylthio oder C₁-C₄-Alkylsulfonyl;
bedeuten.

3. 1-Cycloalkylpyrazolyl-benzoyl-Derivate der Formel I nach den Ansprüchen 1 oder 2, wobei
X O;
Y CR¹⁰R¹¹;
R¹ Halogen, C₁-C₄-Alkyl oder C₁-C₄-Alkoxy;
R⁴ C₁-C₄-Alkylsulfonyl;
R¹³ C₃-C₆-Cycloalkyl;
bedeuten.

4. 1-Cycloalkylpyrazolyl-benzoyl-Derivate der Formel I nach Anspruch 1, wobei
X NR⁶;
R¹ Nitro, Halogen, C₁-C₄-Alkyl oder C₁-C₄-Alkoxy;
R⁴ Halogen, Nitro, C₁-C₄-Halogenalkyl, C₁-C₄-Alkoxy, C₁-C₄-Halogenalkoxy, C₁-C₄-Alkylthio oder C₁-C₄-Alkylsulfonyl;
bedeutet.

5. 1-Cycloalkylpyrazolyl-benzoyl-Derivate der Formel I nach Anspruch 1, wobei
X CR⁷R⁸;
R¹ Nitro, Halogen, C₁-C₄-Alkyl oder C₁-C₄-Alkoxy;
R⁴ Halogen, Nitro, C₁-C₄-Halogenalkyl, C₁-C₄-Alkoxy, C₁-C₄-Halogenalkoxy, C₁-C₄-Alkylthio oder C₁-C₄-Alkylsulfonyl;
bedeuten.

6. Verfahren zur Darstellung von 1-Cycloalkylpyrazolyl-benzoyl-Derivaten der Formel I mit R¹²=Hydroxy, nach Anspruch 1, **dadurch gekennzeichnet, daß** man ein Pyrazol der Formel II 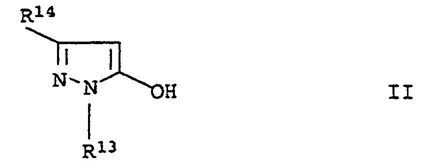 mit einer aktivierten Benzoesäure IIIα oder einer Benzoesäure IIIβ, 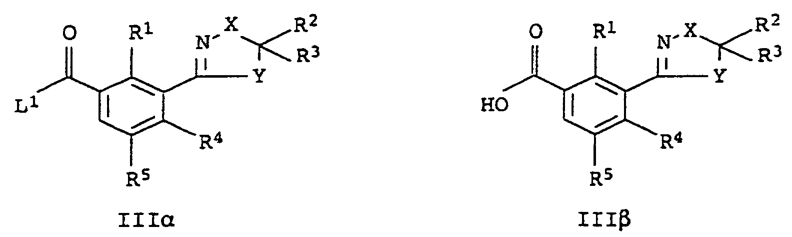 wobei die variablen X, Y, R¹ bis R⁵ und R¹³ bis R¹⁴ die in Anspruch 1 genannte Bedeutung haben und L¹ für eine nucleophil verdrängbare Abgangsgruppe steht, acyliert und Acylierungsprodukt ggf. in Gegenwart eines Katalysators zu den Verbindungen der Formel I mit R¹² = Hydroxy umlagert.

7. Verfahren zur Herstellung von 1-Cycloalkylpyrazolyl-benzoyl-Derivaten der Formel I mit R¹² = OH, nach Anspruch 1, **dadurch gekennzeichnet, daß** man ein Pyrazol der Formel II, 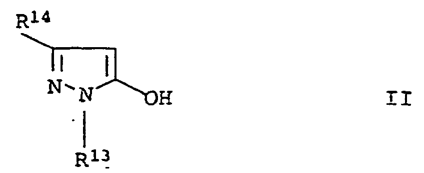 in der die Variablen R¹³ und R¹⁴ die in Anspruch 1 genannte Bedeutung haben, oder ein Alkalisalz hiervon, mit einem 3-(Heterocyclyl)-benzol-Derivat der Formel V, 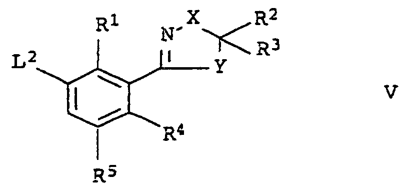 wobei die Variablen X, Y und R¹ bis R⁵ die in Anspruch 1 genannte Bedeutung haben und L² für eine Abgangsgruppe steht, in Gegenwart von Kohlenmonoxid, eines Katalysators sowie einer Base umsetzt.

8. Verfahren zur Herstellung von 1-Cycloalkylpyrazolyl-benzoyl-Derivaten der Formel I mit R¹² 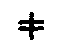 Hydroxy, nach Anspruch 1, **dadurch gekennzeichnet, daß** man ein 1-Cycloalkylpyrazolylbenzoyl-Derivat I mit R¹² = Hydroxy, 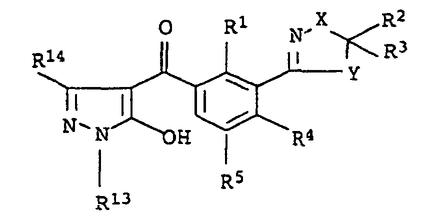 I mit R¹² = OH mit einer Verbindung der Formel VI
L³-R^{12a} VI
umsetzt, wobei
L³ eine nucleophil verdrängbare Abgangsgruppe;
R^{12a} C₁-C₆-Alkyl, C₃-C₆-Alkenyl, C₁-C₆-Alkylsulfonyl, C₁-C₆-Alkylcarbonyl, Phenylsulfonyl oder Phenylcarbonyl, wobei der Phenylrest der beiden letztgenannten Substituenten partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy;
bedeuten.

9. Mittel, enthaltend eine herbizid wirksame Menge mindestens eines 1-Cycloalkylpyrazolyl-benzoyl-Derivats der Formel I oder eines landwirtschaftlich brauchbaren Salzes von I gemäß den Ansprüchen 1 bis 5, und für die Formulierung von Pflanzenschutzmitteln übliche Hilfsmittel.

10. Verfahren zur Herstellung von Mitteln gemäß Anspruch 9, **dadurch gekennzeichnet, daß** man eine herbizid wirksame Menge mindestens eines 1-Cycloalkylpyrazolyl-benzoyl-Derivats der Formel I oder eines landwirtschaftlich brauchbaren Salzes von I gemäß den Ansprüchen 1 bis 6 und für die Formulierung von Pflanzenschutzmitteln übliche Hilfsmittel mischt.

11. Verfahren zur Bekämpfung von unerwünschtem Pflanzenwuchs, **dadurch gekennzeichnet, daß** man eine herbizid wirksame Menge mindestens eines 1-Cycloalkylpyrazolyl-benzoyl-Derivats der Formel I oder eines landwirtschaftlich brauchbaren Salzes von I gemäß den Ansprüchen 1 bis 5, auf Pflanzen, deren Lebensraum und/oder auf Samen einwirken läßt.

12. Verwendung der 1-Cycloalkylpyrazolyl-benzoyl-Derivate der Formel I und/oder deren landwirtschaftlich brauchbaren Salze gemäß den Ansprüchen 1 bis 5 als Herbizide.

## Claims

1. A 1-cycloalkylpyrazolylbenzoyl derivative of the formula I 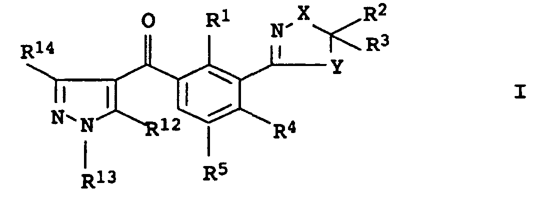 where:
X is O, NR⁶ or CR⁷R⁸;
Y is O, S, NR⁹ or CR¹⁰R¹¹;
R¹ is nitro, halogen, C₁-C₄-alkyl or C₁-C₄-alkoxy;
R²,R³,R⁷,R⁸,R¹⁰,R¹¹ are hydrogen, C₁-C₄-alkyl or C₁-C₄-haloalkyl;
or
R³ and R⁶ or R³ and R⁸ or R³ and R⁹ or R³ and R¹¹ together form a bond;
R⁴ is halogen, nitro, C₁-C₄-haloalkyl, C₁-C₄-alkoxy, C₁-C₄-haloalkoxy, C₁-C₄-alkylthio or C₁-C₄-alkylsulfonyl;
R⁵ is hydrogen;
R⁶,R⁹ are hydrogen or C₁-C₆-alkyl;
R¹² is hydroxyl, C₁-C₆-alkoxy, C₃-C₆-alkenyloxy, C₁-C₆-alkylsulfonyloxy, C₁-C₆-alkylcarbonyloxy, phenylsulfonyloxy or phenylcarbonyloxy, where the phenyl radical of the two last-mentioned substituents may be partially or fully halogenated and/or may carry one to three of the following groups:
nitro, cyano, C₁-C₄-alkyl, C₁-C₄-haloalkyl, C₁-C₄-alkoxy or C₁-C₄-haloalkoxy;
R¹³ is C₃-C₆ cycloalkyl;
R¹⁴ is hydrogen or C₁-C₄-alkyl;
and its agriculturally useful salts.

2. A 1-cycloalkylpyrazolylbenzoyl derivative of the formula I as claimed in claim 1 where
X is O;
R¹ is nitro, halogen, C₁-C₄-alkyl or C₁-C₄-alkoxy;
R⁴ is halogen, nitro, C₁-C₄-haloalkyl, C₁-C₄-alkoxy, C₁-C₄-haloalkoxy, C₁-C₄-alkylthio or C₁-C₄-alkylsulfonyl.

3. A 1-cycloalkylpyrazolylbenzoyl derivative of the formula I as claimed in claim 1 or 2, where
X is O;
Y is CR¹⁰R¹¹;
R¹ is halogen, C₁-C₄-alkyl or C₁-C₄-alkoxy;
R⁴ is C₁-C₄-alkylsulfonyl;
R¹³ is C₃-C₆-cycloalkyl.

4. A 1-cycloalkylpyrazolylbenzoyl derivative of the formula I as claimed in claim 1 where
X is NR⁶;
R¹ is nitro, halogen, C₁-C₄-alkyl or C₁-C₄-alkoxy;
R⁴ is halogen, nitro, C₁-C₄-haloalkyl, C₁-C₄-alkoxy, C₁-C₄-haloalkoxy, C₁-C₄-alkylthio or C₁-C₄-alkylsulfonyl.

5. A 1-cycloalkylpyrazolylbenzoyl derivative of the formula I as claimed in claim 1 where
X is CR⁷R⁸;
R¹ is nitro, halogen, C₁-C₄-alkyl or C₁-C₄-alkoxy;
R⁴ is halogen, nitro, C₁-C₄-haloalkyl, C₁-C₄-alkoxy, C₁-C₄-haloalkoxy, C₁-C₄-alkylthio or C₁-C₄-alkylsulfonyl.

6. A process for preparing 1-cycloalkylpyrazolylbenzoyl derivatives of the formula I where R¹²=hydroxyl as claimed in claim 1, which comprises acylating a pyrazole of the formula II 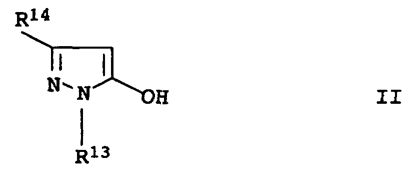 with an activated benzoic acid IIIα or a benzoic acid IIIβ, 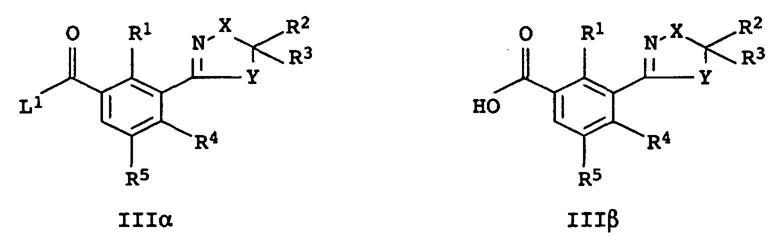 where the variables X, Y, R¹ to R⁵ and R¹³ to R¹⁴ are as defined in claim 1 and L¹ is a nucleophilically displaceable leaving group and rearranging the acylation product in the presence or absence of a catalyst to give the compounds of the formula I where R¹² = hydroxyl.

7. A process for preparing 1-cycloalkylpyrazolylbenzoyl derivatives of the formula I where R¹² = OH as claimed in claim 1, which comprises reacting a pyrazole of the formula II, 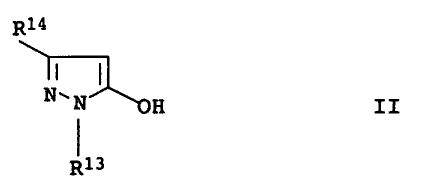 where the variables R¹³ and R¹⁴ are as defined in claim 1 or an alkali metal salt thereof with a 3-(heterocyclyl)benzene derivative of the formula V 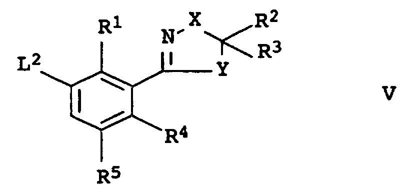 where the variables X, Y and R¹ to R⁵ are as defined in claim 1 and L² is a leaving group in the presence of carbon monoxide, a catalyst and a base.

8. A process for preparing 1-cycloalkylpyrazolylbenzoyl derivatives of the formula I where R¹² 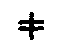 hydroxyl as claimed in claim 1, which comprises reacting a 1-cycloalkylpyrazolylbenzoyl derivative I where R¹² = hydroxyl, 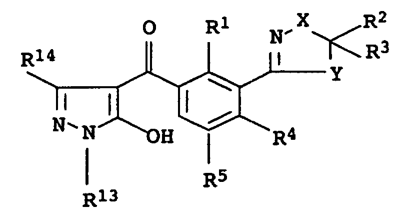 I where R¹² = OH with a compound of the formula VI
L³-R^{12a} VI
where
L³ is a nucleophilically displaceable leaving group;
R^{12a} is C₁-C₆-alkyl, C₃-C₆-alkenyl, C₁-C₆-alkylsulfonyl, C₁-C₆-alkylcarbonyl, phenylsulfonyl or phenylcarbonyl, where the phenyl radical of the two last-mentioned substituents may be partially or fully halogenated and/or may carry one to three of the following groups:
nitro, cyano, C₁-C₄-alkyl, C₁-C₄-haloalkyl, C₁-C₄-alkoxy or C₁-C₄-haloalkoxy.

9. A composition comprising a herbicidally effective amount of at least one 1-cycloalkylpyrazolylbenzoyl derivative of the formula I or an agriculturally useful salt of I as claimed in any of claims 1 to 5 and auxiliaries which are customarily used for formulating crop protection agents.

10. A process for preparing compositions as claimed in claim 9, which comprises mixing a herbicidally effective amount of at least one 1-cycloalkylpyrazolylbenzoyl derivative of the formula I or an agriculturally useful salt of I as claimed in any of claims 1 to 6 and auxiliaries which are customarily used for formulating crop protection agents.

11. A method for controlling undesirable vegetation, which comprises allowing a herbicidally effective amount of at least one 1-cycloalkylpyrazolylbenzoyl derivative of the formula I or an agriculturally useful salt of I as claimed in any of claims 1 to 5 to act on plants, their habitat and/or on seeds.

12. The use of the 1-cycloalkylpyrazolylbenzoyl derivatives of the formula I and/or their agriculturally useful salts as claimed in any of claims 1 to 5 as herbicides.

## Revendications

1. Dérivés de 1-cycloalkylpyrazol-benzoyle répondant à la formule I 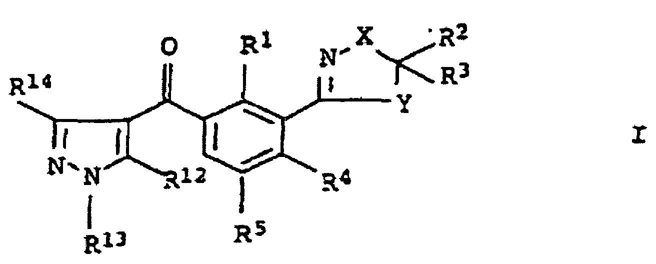 dans laquelle les variables possèdent la signification ci-après :
X représente un atome d'oxygène, un groupe NR⁶ ou un groupe CR⁷R⁸ ;
Y représente un atome d'oxygène, un atome de soufre, un groupe NR⁹ ou un groupe CR¹⁰R¹¹ ;
R¹ représente un groupe nitro, un atome d'halogène, un groupe alkyle en C₁-C₄ ou un groupe alcoxy en C₁-C₄ ;
R², R³, R⁷, R⁸, R¹⁰, R¹¹ représentent un atome d'hydrogène, un groupe alkyle en C₁-C₄ ou un groupe halogénalkyle en C₁-C₄ ;
ou bien
R³ et R⁶ ou R³ et R⁸ ou R³ et R⁹ ou R³ et R¹¹ forment ensemble une liaison ;
R⁴ représente un atome d'halogène, un groupe nitro, un groupe halogénoalkyle en C₁-C₄, un groupe alcoxy en C₁-C₄, un groupe halogénoalcoxy en C₁-C₄, un groupe alkyl(en C₁-C₄)thio ou un groupe alkyl(en C₁-C₄) sulfonyle ;
R⁵ représente un atome d'hydrogène ;
R⁶, R⁹ représentent un atome d'hydrogène ou un groupe alkyle en C₁-C₆ ;
R¹² représente un groupe hydroxyle, un groupe alcoxy en C₁-C₆, un groupe alcényl(en C₃-C₆)oxy, un groupe alkyl(en C₁-C₆) sulfonyloxy, un groupe alkyl(en C₁-C₆)carbonyloxy, un groupe phénylsulfonyloxy ou un groupe phénylcarbonyloxy, le radical phényle des deux substituants mentionnés en dernier lieu pouvant être halogéné en partie ou complètement et/ou pouvant porter de 1 à 3 groupes parmi ceux repris ci-après : un groupe nitro, un groupe cyano, un groupe alkyle en C₁-C₄, un groupe halogénoalkyle en C₁-C₄, un groupe alcoxy en C₁-C₄ ou un groupe halogénoalcoxy en C₁-C₄ ;
R¹³ représente un groupe cycloalkyle en C₃-C₆ ;
R¹⁴ représente un atome d'hydrogène ou un groupe alkyle en C₁-C₄ ;
ainsi que leurs sels utiles en agriculture.

2. Dérivés de 1-cycloalkylpyrazolyl-benzoyle répondant à la formule I selon la revendication 1, dans lesquels
X représente un atome d'oxygène ;
R¹ représente un groupe nitro, un atome d'halogène, un groupe alkyle en C₁-C₄ ou un groupe alcoxy en C₁-C₄ ;
R⁴ représente un atome d'halogène, un groupe nitro, un groupe halogénoalkyle en C₁-C₄, un groupe alcoxy en C₁-C₄, un groupe halogénoalcoxy en C₁-C₄, un groupe alkyl(en C₁-C₄)thio ou un groupe alkyl(en C₁-C₄) sulfonyle.

3. Dérivés de 1-cycloalkylpyrazolyl-benzoyle répondant à la formule 1 selon la revendication 1 ou 2, dans lesquels
X représente un atome d'oxygène ;
Y représente un groupe CR¹⁰R¹¹ ;
R¹ représente un atome d'halogène, un groupe alkyle en C₁-C₄ ou un groupe alcoxy en C₁-C₄ ;
R⁴ représente un groupe alkyl(en C₁-C₄) sulfonyle ;
R¹³ représente un groupe cycloalkyle en C₃-C₆.

4. Dérivés de 1-cycloalkylpyrazolyl-benzoyle répondant à la formule I selon la revendication 1, dans lesquels
X représente un groupe NR⁶ ;
R¹ représente un groupe nitro, un atome d'halogène, un groupe alkyle en C₁-C₄ ou un groupe alcoxy en C₁-C₄ ;
R⁴ représente un atome d'halogène, un groupe nitro, un groupe halogénoalkyle en C₁-C₄, un groupe alcoxy en C₁-C₄, un groupe halogénoalcoxy en C₁-C₄, un groupe alkyl(en C₁-C₄)thio ou un groupe alkyl(en C₁-C₄) sulfonyle.

5. Dérivés de 1-cycloalkylpyrazolyl-benzoyle répondant à la formule I selon la revendication 1, dans lesquels
X représente un groupe CR⁷R⁸ ;
R¹ représente un groupe nitro, un atome d'halogène, un groupe alkyle en C₁-C₄ ou un groupe alcoxy en C₁-C₄ ;
R⁴ représente un atome d'halogène, un groupe nitro, un groupe halogénoalkyle en C₁-C₄, un groupe alcoxy en C₁-C₄, un groupe halogénoalcoxy en C₁-C₄, un groupe alkyl(en C₁-C₄)thio ou un groupe alkyl(en C₁-C₄) sulfonyle.

6. Procédé pour la préparation de dérivés de 1-cycloalkylpyrazolyl-benzoyle répondant à la formule I dans laquelle R¹² représente un groupe hydroxyle, selon la revendication 1, **caractérisé en ce qu'**on soumet à une acylation un pyrazole répondant à la formule II 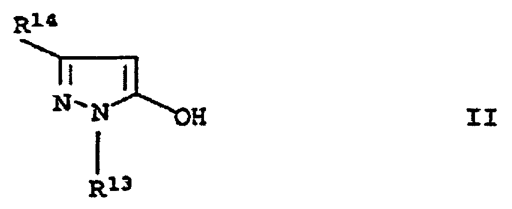 avec un acide benzoïque activé IIIα ou avec un acide benzoïque IIIβ, 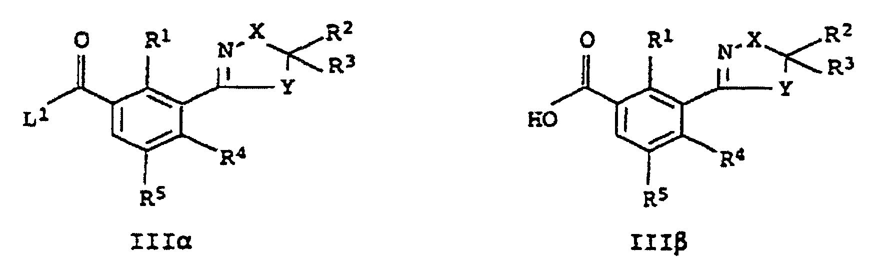 dans lequelles les variables X, Y, R¹ à R⁵ et R¹³ à R¹⁴ possèdent la signification indiquée à la revendication 1 et L¹ représente un groupe de départ apte à un déplacement nucléophile, et on convertit le produit de l'acylation, le cas échéant en présence d'un catalyseur, pour obtenir les composés répondant à la formule I dans laquelle R¹² représente un groupe hydroxyle.

7. Procédé pour la préparation de dérivés de 1-cycloalkylpyrazolyl-benzoyle répondant à la formule I dans laquelle R¹² représente un groupe OH, selon la revendication 1, **caractérisé en ce qu'**on fait réagir un pyrazole répondant à la formule II 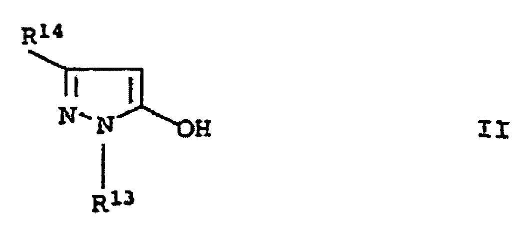 dans laquelle les variables R¹³ et R¹⁴ possèdent la signification indiquée à la revendication 1, ou un de ses sels de métal alcalin, avec un dérivé de 3-(hétérocyclyl)-benzène répondant à la formule V, 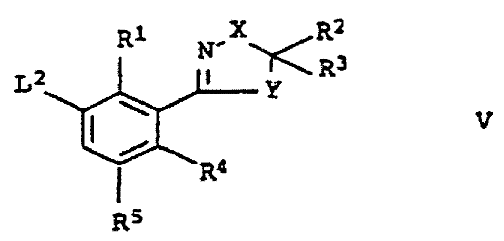 dans laquelle les variables X, Y, et R¹ à R⁵ possèdent la signification indiquée à la revendication 1 et L² représente un groupe de départ, en présence de monoxyde de carbone, d'un catalyseur et d'une base.

8. Procédé pour la préparation de dérivés de 1-cycloalkylpyrazolyl-benzoyle répondant à la formule I dans laquelle R¹² ne représente pas un groupe hydroxyle, selon la revendication 1, **caractérisé en ce qu'**on fait réagir un dérivé de 1-cycloalkylpyrazolyl-benzoyle I dans lequel R¹² représente un groupe hydroxyle 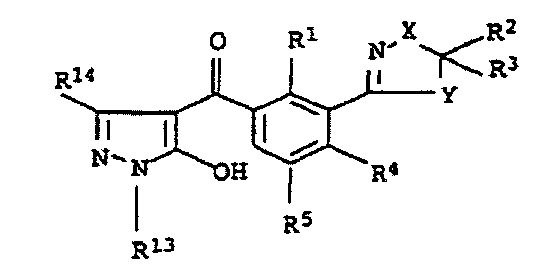 I avec R¹² = un groupe OH
avec un composé répondant à la formule VI
L³-R^{12a} VI
dans laquelle
L³ représente un groupe de départ apte à un déplacement nucléophile ;
R^{12a} représente un groupe alkyle en C₁-C₆, un groupe alcényle en C₃-C₆, un groupe alkyl(en C₁-C₆)sulfonyle, un groupe alkyl(en C₁-C₆)carbonyle, un groupe phénylsulfonyle ou un groupe phénylcarbonyle, le radical phényle des deux substituants mentionnés en dernier lieu pouvant être halogéné en partie ou complètement et/ou pouvant porter de 1 à 3 groupes parmi ceux repris ci-après : un groupe nitro, un groupe cyano, un groupe alkyle en C₁-C₄, un groupe halogénoalkyle en C₁-C₄, un groupe alcoxy en C₁-C₄ ou un groupe halogénoalcoxy en C₁-C₄.

9. Agents contenant une quantité efficace du point de vue herbicide d'au moins un dérivé de 1-cycloalkylpyrazolyl-benzoyle répondant à la formule I ou d'un sel de I utile en agriculture, selon les revendications 1 à 5, ainsi que des adjuvants habituels pour la formulation d'agents de protection des plantes.

10. Procédé pour la préparation d'agents selon la revendication 9, **caractérisé en ce qu'**on mélange une quantité efficace du point de vue herbicide d'au moins un dérivé de 1-cycloalkylpyrazolyl-benzoyle répondant à la formule I ou d'un sel de I utile en agriculture, selon les revendications 1 à 6 et des adjuvants habituels pour la formulation d'agents de protection des plantes.

11. Procédé pour lutter contre la croissance non désirée de plantes, **caractérisé en ce qu'**on laisse agir une quantité efficace du point de vue herbicide d'au moins un dérivé de 1-cycloalkylpyrazolyl-benzoyle répondant à la formule I ou d'un sel de I utile en agriculture, selon les revendications 1 à 5, sur des plantes, sur leur biotope et/ou sur des semences.

12. Utilisation des dérivés de 1-cycloalkylpyrazolyl-benzoyle répondant à la formule I et/ou de leurs sels utiles en agriculture, selon les revendications 1 à 5, à titre d'herbicides.
